# EUROPEAN PATENT APPLICATION

(11) **EP 1 964 922 A1**
(43) Date of publication of application: **03.09.2008**
(21) Application number: 07103406.0
(22) Date of filing: 02.03.2007
(51) Int. Cl.: C12N 15/67

(54) **Improvement of protein production**

(71) Applicant: Boehringer Ingelheim Pharma GmbH & Co. KG, 55216 Ingelheim am Rhein (DE)
(72) Inventor: The designation of the inventor has not yet been filed
(74) Representative: Hammann, Heinz

(57) **Abstract**

The invention concerns the field ofprotein production and cell culture technology. CERT is identified as a novel *in vivo* PKD substrate. Phosphorylation on serine 132 by PKD decreases the affinity of CERT towards its lipid target phosphatidylinositol 4-phosphate at Golgi membranes and reduces ceramide transfer activity, identifying PKD as a regulator of lipid homeostasis. The present invention shows that CERT in turn is critical for PKD activation and PKD dependent protein cargo transport to the plasma membrane. The interdependence of PKD and CERT is thus a key to the maintenance of Golgi membrane integrity and secretory transport.

## Description

### BACKGROUND OF THE INVENTION

### TECHNICAL FIELD

The invention concerns the field of cell culture technology. It concerns a method for producing proteins as well as a method to generate novel expression vectors and host cells for biopharmaceutical manufacturing. The invention further concerns pharmaceutical compositions and methods of treatment.

### BACKGROUND

The market for biopharmaceuticals for use in human therapy continues to grow at a high rate with 270 new biopharmaceuticals being evaluated in clinical studies and estimated sales of 30 billions in 2003 (Werner, 2004). Biopharmaceuticals can be produced from various host cell systems, including bacterial cells, yeast cells, insect cells, plant cells and mammalian cells including human-derived cell lines. Currently, an increasing number of biopharmaceuticals is produced from eukaryotic cells due to their ability to correctly process and modify human proteins. Successful and high yield production of biopharmaceuticals from these cells is thus crucial and depends highly on the characteristics of the recombinant monoclonal cell line used in the process. Therefore, there is an urgent need to generate new host cell systems with improved properties and to establish methods to culture producer cell lines with high specific productivities as a basis for high yield processes.

Early approaches focused on process design and reactor design. Now the main improvements are driven by media formulation development and genetically engineering of host cells. The most common industrial mammalian host cell systems for the production of biopharmaceuticals are immortalized Chinese hamster ovary (CHO) cell lines (Wurm, 2004).
Initial metabolic engineering strategies to improve mammalian production cell lines focused on their ability to grow in suspension in serum free media. Stable expression of transferrin and insulin-like growth factor 1 (IGF-1) in CHO-K1 cells resulted in a cell line able to proliferate under protein-free conditions (Pak et al., 1996). Further approaches to improve the production cell lines included the use of regulatory DNA elements on the transfection vectors aimed to target or create transcriptional hot spots. Regulatory elements such as S/MARs (Scaffold/matrix-associated regions) which effect chromatin structure and UCOEs (Ubiquitous chromatin opening elements) derived from house keeping genes were both shown to positively effect specific productivities of recombinant proteins produced from CHO cell lines (Barnes and Dickson, 2006).
As apoptosis has been shown to be the predominant cause of cell death in mammalian cell culture production processes (al-Rubeai and Singh, 1998) the effect of expression of anti-apoptotic genes in mammalian host cells on culture viability was thoroughly investigated. Most antiapoptosis engineering strategies are focused on the overexpression of anti-apoptotic genes of the bcl-2 family (e.g. bcl-1 or bcl-xL; (Kaufmann and Fussenegger, 2003). By increasing the cellular resistance to apoptotic stimuli during fermentation, such as nutrient depletion and waste byproduct accumulation, production processes with apoptosis engineered cell lines showed prolonged culture viability and in some cases an increase in product yield (Chiang and Sisk, 2005).

Since most biopharmaceutical products are proteins that are secreted from the cells during the production process, the secretory transport machinery of the production cell line is another interesting target for novel host cell engineering strategies.

Protein secretion is a complex multi-step mechanism: Proteins destined to be transported to the extracellular space or the outer plasma membrane are first co-translationally imported into the endoplasmic reticulum. From there, they are packed in lipid vesicles and transported to the Golgi apparatus and finally from the trans-Golgi network (TGN) to the plasma membrane where they are released into the culture medium (Seth et al., 2006).

The yield of any biopharmaceutical production process depends largely on the amount of protein product that the producing cells secrete per time when grown under process conditions. Many complex biochemical intracellular processes are necessary to synthesize and secrete a therapeutic protein from a eukaryotic cell. All these steps such as transcription, RNA transport, translation, post-translational modification and protein transport are tightly regulated in the wild-type host cell line and will impact on the specific productivity of any producer cell line derived from this host.

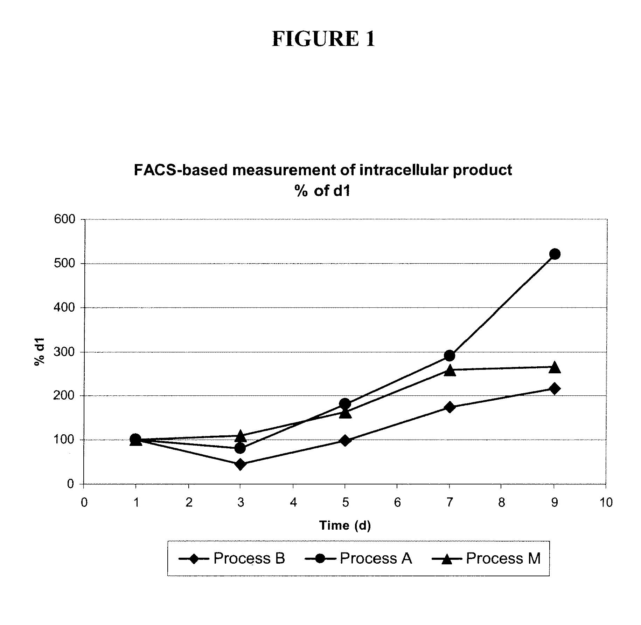

Many engineering approaches have employed the growing understanding of the molecular networks that drive processes such as transcription and translation to increase the yield of these steps in protein production. However, as for any multi-step production process, widening a bottle-neck during early steps of the process chain possibly creates bottle necks further downstream, especially post translation. Up to a certain threshold, the specific productivity of a production cell has been reported to correlate linearly with the level of product gene transcription (Barnes et al., 2007). Further enhancement of product expression at the mRNA level , however, may lead to an overload of the protein synthesis, folding or transport machinery, resulting in intracellular accumulation of the protein product. Indeed, this can be frequently observed in current manufacturing processes (FIGURE 1).

Specific targeted engineering approaches aimed to address this problem and to efficiently improve the secretion of protein products from eukaryotic cells are hampered by the current lack of understanding of the complex regulatory network that drives the transport of proteins to the plasma membrane.

The first studies on engineering the intracellular transport of secreted therapeutic proteins were centered around the overexpression of molecular chaperones like binding protein BiP/GRP78, protein disulfide isomerase (PDI). Chaperones are cellular proteins hosted within the endoplasmic reticulum (ER) and assist the folding and assembly of newly synthesised proteins. In contrast to what could be expected, BiP overexpression in mammalian cells has been shown to reduce rather than increase the secretion of proteins it associates with (Dorner and Kaufman, 1994). Likewise, PDI overexpression in CHO cells reduced the expression of a TNFR:FC fusion protein (Davis et al., 2000), whereas the specific production rate of an antibody was increased by 40% (Borth et al., 2005). A possible explanation for these surprising findings, that the increase of the cell's protein folding capacity creates a production bottle neck further downstream, is supported by a report describing ER to cis-Golgi transport problems for IFN-gamma production in a CHO cell line (Hooker et al., 1999).

Another recent approach to increase the secretion capacity of mammalian cells is the heterologous overexpression of the transcription factor X-box binding protein 1 (XBP-1). XBP-1 is one of the master-regulators in the differentiation of plasma cells, a specialized cell type optimized for high-level production and secretion of antibodies (Iwakoshi et al., 2003). XBP-1 regulates this process by binding to the so called ER stress responsive elements (ERSE) within the promoters of a wide spectrum of secretory pathway genes, resulting in (i) a physical expansion of the ER, (ii) increased mitochondrial mass and function, (iii) larger cell size and (iv) enhanced total protein synthesis (Shaffer et al., 2004).

Recently, attempts were described to increase protein secretion by overexpressing XBP-1 in non-plasma cells, especially production cell lines. In CHO-K1 cells, the production level of two reporter proteins (secreted alkaline phospatase (SEAP) and secreted alpha-amylase (SAMY)) was shown to increase after XBP-1 introduction in CHO-K1 cells. However, no effect could be demonstrated in transient studies with other cell lines such as HEK293, HeLa or HT-1080 cells (Tigges and Fussenegger, 2006). The patent application WO2004111194 by Ailor Eric claims the overexpression of XBP-1 or ATF6 for the generation of highly productive cell lines.

Notably, XBP-1 does not only regulate plasma cell differentiation but also plays an important role in the unfolded protein response (UPR) (Brewer and Hendershot, 2005). The UPR represents a complex signal transduction network activated by inhibition of protein folding in the endoplasmic reticulum (ER). The UPR coordinates adaptive responses to this stress situation, including induction of ER resident molecular chaperone and protein foldase expression to increase the protein folding capacity of the ER, induction of phospholipid synthesis, attenuation of general translation, and upregulation of ER-associated degradation to decrease the unfolded protein load of the ER. Upon severe or prolonged ER stress, the UPR ultimately induces apoptotic cell death (Schroder, 2006).

The process of terminal differentiation, such as the maturation from a lymphocyte to a plasma cell, is usually regarded an apoptosis-like program, during which the cell loses its proliferative capacity to give rise to a terminally differentiated secretory cell. In fact, nearly all cell types specifically designed for high-level protein secretion (e.g. glandular cells, pancreatic beta cells) are terminally differentiated, are not able to proliferate and have a limited life-span before ultimately undergoing programmed cell death (Chen-Kiang, 2003). Therefore, overexpressing XBP-1 as a regulator of both plasma cell differentiation and UPR, is potentially disadvantageous due to its inherent risk to inhibit proliferation and/or induce apoptosis .

Taken together, there is a need for improving the secretory capacity of host cells for recombinant protein production. This might even become more important in combination with novel transcription-enhancing technologies and in high-titer processes in order to prevent post-translational bottle necks and intracellular accumulation of the protein product (FIGURE 1). However, at present, there are two major hurdles on the way to targeted manipulation of the secretory transport machinery: The still limited knowledge about the underlying regulatory mechanisms and the requirement to prevent a concomitant growth-inhibitory or apoptotic response of the producer cell.

The present invention describes a novel and surprising role for the ceramide transfer protein CERT in the transport of secreted proteins to the plasma membrane and furthermore provides a method to efficiently improve the production of proteins that are transported via the secretory pathway from eukaryotic cells.

CERT (also known as Goodpasture antigen-binding protein) is a cytosolic protein essential for the non-vesicular delivery of ceramide from its site of production at the endoplasmic reticulum (ER) to Golgi membranes, where conversion to sphingomyelin (SM) takes place (Hanada et al., 2003).

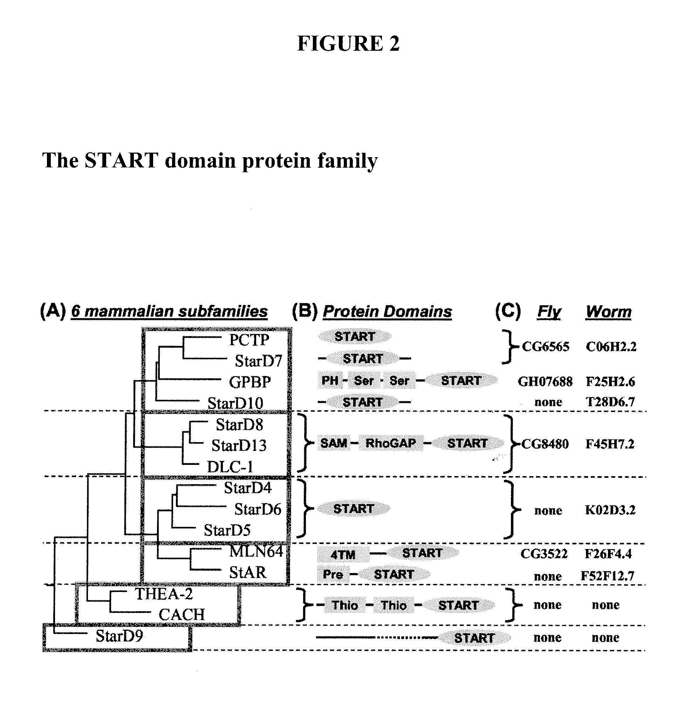

Two CERT isoforms exist: the more abundantly expressed, alternatively spliced form missing a 26-amino-acid, serine-rich region (SEQ ID NO. 10, 11) and the full-length 624 amino acid protein, designated CERT_{L} (SEQ ID NO.12,13)(Raya et al., 2000). Both CERT isoforms possess a carboxyterminal steroidogenic acute regulatory (StAR)-related lipid transfer (START) domain that is necessary and sufficient for ceramide binding and transport (Hanada et al., 2003). START domains are highly conserved from fly and worm to humans (FIGURE 2). They are -210 amino acids in length and form a hydrophobic tunnel that accommodates a monomeric lipid (Alpy and Tomasetto, 2005; Soccio and Breslow, 2003). START domains are found in 15 mammalian proteins, with CERT being most closely related to the phosphatidylcholine transfer protein Pctp, which binds and shuttles phosphatidylcholine (PC) between membranes, and StarD10, a lipid transfer protein specific for PC and PE (Olayioye et al., 2005; Soccio and Breslow, 2003; Wirtz, 2006). In addition to the START domain, the CERT proteins further contain an aminoterminal PH domain with specificity for PI(4)P that is responsible for Golgi localization (Hanada et al., 2003; Levine and Munro, 2002) and a FFAT motif (two phenylalanines in an acidic tract) that targets the protein to the ER via interaction with the ER resident transmembrane proteins VAP-A and VAP-B (Kawano et al., 2006; Loewen et al., 2003).

The fundamental role of CERT in lipid trafficking was demonstrated in the Chinese hamster ovary cell line LY-A, in which the expression of a mutant non-functional CERT protein impaired ceramide transport, thus resulting in reduced cellular levels of sphingomyelin (Hanada et al., 2003). Non-vesicular lipid transfer is thought to occur at so-called membrane contact sites (MCS), at which the ER comes into close apposition with other organelles (Levine and Loewen, 2006). CERT may thus shuttle a very short distance between ER and Golgi membranes, or perhaps contact both compartments simultaneously.

When overexpressed, the START domain of CERT is sufficient for ceramide transfer to the Golgi apparatus (Kawano et al., 2006). However, under physiological conditions, both Golgi and ER targeting motifs are essential for CERT function. In LY-A cells, CERT was identified to contain a mutation within its PH domain (G67E), rendering the protein defective in PI(4)P binding (Hanada et al., 2003). The requirement for PI(4)P for CERT function is further supported by a recent report that PI4KIII-beta activity is necessary for efficient ceramide trafficking to the Golgi (Toth et al., 2006), the enzymatic activity of which is stimulated by protein kinase D (PKD).

PKD belongs to a subfamily of serine-/threonine-specific protein kinases (comprising PKD1/PKCµ, PKD2 and PKD3/PKCυ) and was recently identified to be of crucial importance for the regulation of protein transport from the Golgi membrane to the plasma membrane (reviewed in (Rykx et al., 2003; Wang, 2006)). Recruitment and activation of PKD at the TGN is mediated by the lipid diacylglycerol (DAG; (Baron and Malhotra, 2002)), a pool of which is generated by sphingomyelin synthase from ceramide and phosphatidylcho line.

The present invention shows that PKD phosphorylates CERT on serine 132 adjacent to the PH domain, whereby PI(4)P binding, Golgi targeting and ceramide transfer activity are negatively regulated. Furthermore, by transferring ceramide that is required for DAG production to Golgi membranes, CERT stimulates PKD activity, thus establishing a regulatory feedback-loop that ensures the maintenance of constitutive secretory transport.

Importantly, the data provided furthermore show that in different eukaryotic cell lines (COS7 and HEK293), introduction of the gene encoding CERT significantly enhances the secretion of a heterologous protein into the culture medium. This effect is even more pronounced when using a CERT mutant which cannot be phosphorylated by PKD. Deletion of the phosphorylation acceptor site within CERT interrupts the negative control of PKD on CERT, but leaving the positive feedback of CERT on PKD intact through the support of ceramide conversion to sphingomyelin and DAG. It can therefore be speculated that the secretion enhancing mechanism of the present invention can be exerted not only by wild type CERT but also by all mutants of CERT which uncouple CERT from the negative influence of PKD, including point mutations of the acceptor serine, deletions including this residue as well as mutation or deletion of the PKD docking site within CERT or even the START domain alone.

CERT belongs to the family of StAR-related Lipid Transfer proteins (Soccio and Breslow, 2003), which are characterized by their START domains for lipid binding. As the START domain of CERT has been demonstrated to be both required and sufficient for CERT action (Hanada et al., 2003), it is possible that the secretion-promoting effect of CERT could equally be observed when overexpressing another member of this protein family. This is especially likely for the closely related members of the PCTP-subfamily, comprising PCTP (SEQ ID NO.26, 27), CERT/GPBP itself, StarD7 and StarD10. These proteins have distinct lipid-binding specificities and could equally impact on the function of organelles involved in the secretion of heterologous proteins.
Furthermore, expression of the related proteins STARD4 (SEQ ID NO.20, 21) and STARD5 (SEQ ID NO.22, 23), that are induced upon ER stress, may function to fulfill the increased demand of lipid transfer of cells during a production process.

The existence of START domain proteins in eukaryotic organisms from fly, worm and mouse to humans indicates that the basic mechanisms of lipid trafficking are conserved among the eukaryotic kingdom. It furthermore suggests, that the principle described in the present invention - that is increasing secretion by enforced expression of CERT - may well be applicable to all eukaryotic cells, including yeast.

In summary, the present invention provides a method for enhancing the secretory transport of proteins in eukaryotic cells by heterologous expression of CERT, CERT mutants or another member of the START protein family. This method is particularly useful for the generation of optimized host cell systems with enhanced production capacity for the expression and manufacture of recombinant protein products.

### The method described in the present invention is advantageous in several respects:

First, we demonstrate heterologous expression of CERT to be a strategy to enhance recombinant protein production by increasing the secretory capacity of the host cell. Enhancing the specific productivity of producer cells translates into higher product yields in industrial protein production processes. With the current trend towards high-titer processes and more sophisticated expression enhancing technologies, post-translational bottle necks will become the evident rate-limiting steps in protein production and hence will draw increasing attention to secretion engineering approaches.

Second, the START domain of CERT is highly conserved in eukaryotes from *C. elegans* to humans. This strongly suggests that the method of the present invention can not only be used in mammalian host cell systems, but is equally applicable for protein production in all eukaryotic cells, including insect cells and yeast cells.

As a third important feature, CERT as a cytosolic factor is not part of the unfolded protein response and thus is not involved in a cellular stress response program which induces the shut-down of protein translation and - if not resolved - leads to cell cycle arrest or even apoptosis. In contrast, by playing an independent role in lipid trafficking, targeting CERT might confer enhanced protein secretion without concomitant induction of apoptosis. Thus, overexpressing CERT in producer host cells might be advantageous over XBP-1 based genetically engineering approaches.

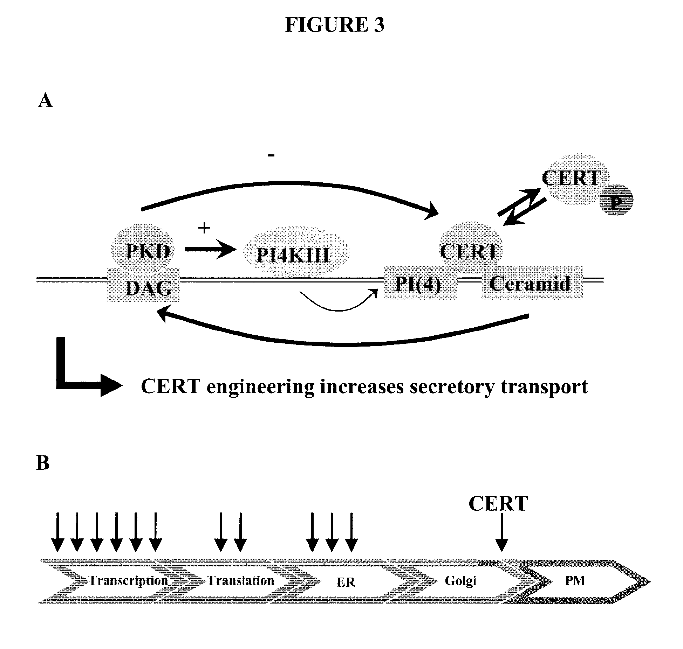

Fourth, it is shown in the present invention that mutation of Ser132 of CERT impairs the phosphorylation of CERT by PKD which frees CERT from a negative regulatory influence. Meanwhile, the positive stimulation of PKD by CERT via DAG is left intact (FIGURE 3A). This finding places CERT in the signalling pathway "upstream" of PKD, which has been published to be critically involved in the regulation of the late stages of secretory transport, namely the transport from the trans-Golgi network to the plasma membrane (Liljedahl et al., 2001). With regard to protein transport, this means, that CERT acts "downstream" of the ER which makes CERT the preferable target for manipulation compared to XBP-1 or specific ER-residing proteins (FIGURE 3B).
Since CERT can impact even on the latest steps of the secretory pathway, it can be speculated that heterologous expression of CERT has the potential to enhance secretion without creating bottle necks further downstream. To our knowledge, CERT is currently the most downstream acting target for genetical engineering of the secretory pathway to enhance heterologous protein production.

Taken together, the impact of the lipid-transfer protein CERT on the secretory transport from ER to Golgi and from the Golgi apparatus to the plasma membrane, without the disadvantageous connection to a growth-inhibiting or apoptosis-inducing stress response make CERT, CERT mutants and other START family proteins very attractive and promising targets for genetic engineering approaches aiming to enhance the secretory capacity of eukaryotic cells.

### APPLICABILITY

The targeted manipulation of CERT which is described in the present invention can be used for a broad range of applications. In particular, two basic approaches can be distinguished:
(i) Overexpression and/or enhancing the activity of CERT or a CERT derivative to increase the secretory transport capacity of a cell, or
(ii) reducing CERT activity and/or expression as a means of gene therapy in order to reduce cancer cell proliferation and/or invasion.

### Applicability of CERT overexpression

The described invention describes a method to generate improved eukaryotic host cells for the production of heterologous proteins by introducing the gene encoding CERT, CERT mutants or other proteins of the START protein family. This will enable to increase the protein yield in production processes based on eukaryotic cells. It will thereby reduce the cost of goods of such processes and at the same time reduce the number of batches that need to be produced to generate the material needed for research studies, diagnostics, clinical studies or market supply of a therapeutic protein. The invention will furthermore speed up drug development as often the generation of sufficient amounts of material for pre-clinical studies is a critical work package with regard to the timeline.

The invention can be used to increase the property of all eukaryotic cells used for the generation of one or several specific proteins for either diagnostic purposes, research purposes (target identification, lead identification, lead optimization) or manufacturing of therapeutic proteins either on the market or in clinical development.

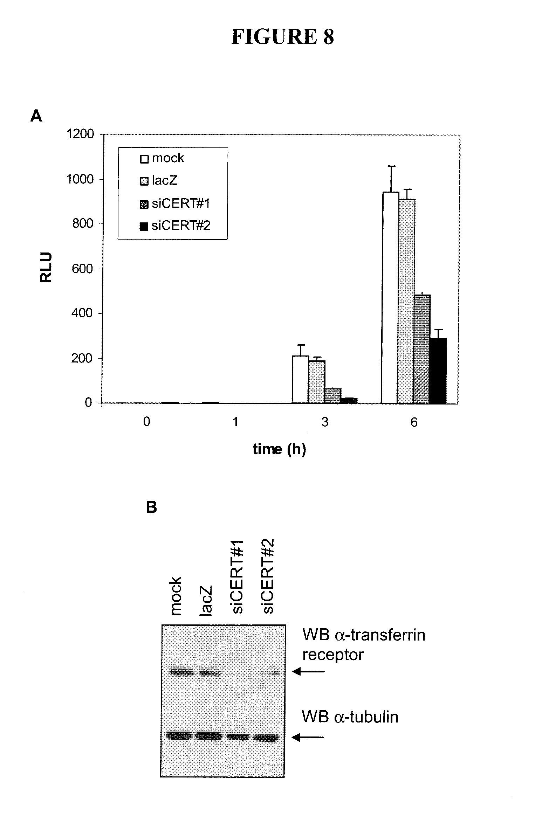

As shown in the present invention, heterologous expression of CERT does not only enhance protein secretion, but also has an influence on the abundance of transmembrane proteins on the cell surface. Inhibition or reduced expression of CERT leads to a dramatic reduction of the amount of cell surface receptors such as the transferrin receptor (FIGURE 8). As secreted and transmembrane proteins share the same secretory pathways and are equally transported in lipid-vesicles, these data underscore the importance of CERT in the modulation of secretion as well as the transport of membrane-bound cell-surface receptors.

Therefore, the method described herein can also be used for academic and industrial research purposes which aim to characterize the function of cell-surface receptors. E.g. it can be used for the production and subsequent purification, crystallization and/or analysis of surface proteins. This is of crucial importance for the development of new human drug therapies as cell-surface receptors are a predominant class of drug targets. Moreover, it might be advantageous for the study of intracellular signalling complexes associated with cell-surface receptors or the analysis of cell-cell-communication which is mediated in part by the interaction of soluble growth factors with their corresponding receptors on the same or another cell.

### Applicability of decreasing / inhibiting CERT

In the present invention, we provide evidence that the reduction of CERT expression leads to reduced secretion of soluble extracellular proteins as well as a lower abundance of cell surface receptors. This makes CERT an attractive target for therapeutic manipulation.

One of the hallmarks in the conversion from a normal healthy cell to a cancer cell is the acquisition of independency from the presence of exogenous growth factors (Hanahan and Weinberg, 2000). In contrast to the normal cell, tumor cells are able to produce all growth factors necessary for their survival and proliferation by themselves. In addition to this autocrine mechanism, cancer cells often show an upregulated expression of growth factor receptors on their surface, which leads to an increased responsiveness towards paracrine-acting growth and survival factors secreted from cells in the surrounding tissue. By targeting CERT in tumor cells, e.g. by using siRNA approaches, it might be possible to disrupt autocrine as well as paracrine growth-stimulatory and/or survival mechanisms in two ways: (i) By reducing growth factor transport and secretion and (ii) by decreasing the amount of the corresponding growth factor-receptor on tumor cells. Thereby both, the amount of growth stimulating signal and the ability of the cancer cell to perceive and respond to these signals will be reduced. Inhibition of CERT expression in cancer cells might therefore represent a powerful tool to prevent cancer cell proliferation and survival.

CERT might furthermore be a potent therapeutic target to suppress tumor invasion and metastasis. During the later stages of most types of human cancer, primary tumors spawn pioneer cells that move out, invade adjacent tissues, and travel to distant sites where they may succeed in founding new colonies, known as metastasis.

As a prerequisite for tissue invasion, cancer cells express a whole set of proteases which enable them to migrate through the surrounding healthy tissue, to cross the basal membrane, to get into the blood stream and to finally invade the tissue of destination. Some of these proteases are expressed as membrane-bound proteins, e.g. MT-MMPs (Egeblad and Werb, 2002) and ADAMs (Blobel, 2005). Due to their crucial role in matrix remodelling, shedding of growth factors and tumor invasion, proteases themselves are discussed as drug targets for cancer therapy (Overall and Kleifeld, 2006). We hypothesize that inhibition of CERT expression and/or activity in tumor cells will reduce the amount of membrane-bound proteases on the surface of the targeted cell. This might decrease or even impair the invasive capacity of the tumor cell as well as its ability for growth factor shedding, resulting in reduced invasiveness and metastatic potential of the tumor. Thus, targeting CERT might offer a novel way of preventing late-stage tumorgenesis, especially the conversion from a benign / solid nodule to an aggressive, metastasizing tumor.

For therapeutic applications it is, thus, the goal to reduce and/or inhibit the activity and/or expression of CERT. This can be achieved either by a nucelotide composition which is used as human therapeutic to treat a disease by inhibiting CERT function whereby the drug is composed of an RNAi, and siRNA or an antisense RNA specificly inhibiting CERT through binding a sequence motive of CERT RNA. Reduction / inhibition of CERT activity/expression can also be achieved by a drug substance containing nucleotides binding and silencing the promoter of the CERT gene.
Furthermore, a drug substance or product can be composed of a new chemical entity or peptide or protein inhibiting CERT expression or activity. In case of a protein being the active pharmaceutical compound it may be a (i) protein binding to CERT promoter thereby inhibiting CERT expression, (ii) protein binding to CERT or PKD thus preventing binding of PKD and CERT and hindering CERT phosphorylation by PKD, (iii) a protein similar to CERT which however does not fulfill CERT functions, that means a "dominant-negative" CERT variant, or (iv) a protein acting as scaffold for both CERT and PKD, resulting in irreversible binding of CERT to PKD (= a stable PKD/CERT complex) which is not functional due to the inhibitory phosphorylation of CERT by PKD and the hindering of dissociation of CERT from said complex.

### SUMMARY OF THE INVENTION

The present invention is not obvious from the prior art. Up to this point the only experimental data available on the protein CERT pointed to a role in transport of ceramide from the endoplasmic reticulum to the Golgi apparatus as a precursor of sphingomyelin. Only the data described in this invention lead to a novel working model for a role of CERT in protein transport form the Golgi to the plasma membrane in eukaryotic cells. The prior art does not give any hint on the possibility of enhancing the rate of secretory transport of proteins in eukaryotic cell lines by introducing the gene encoding CERT or another member of the START domain protein family.

The surprising and unexpected working model of the present invention identifies CERT as a novel *in vivo* PKD substrate and crucial regulator of Golgi function.

PKD is known from the prior art. It is a family of serine/threonine-specific protein kinases comprising three structurally related members: PKD1/PKCµ, PKD2 and PKD3/PKCυ. PKD contains two aminoterminal zinc finger-like cysteine-rich motifs that bind DAG, a pleckstrin homology (PH) domain that negatively regulates PKD enzymatic function and a carboxyterminal kinase domain.

The three PKD isoforms localize to the cytosol, nucleus, Golgi complex and plasma membrane, where they regulate diverse cellular processes, ranging from proliferation, differentiation, apoptosis, cytoskeletal reorganization and metastasis to vesicle trafficking (reviewed in (Rykx et al., 2003; Wang, 2006)). Thus far, only a few physiological PKD substrates are known, which include the neuronal protein Kidins220, the Ras effector RIN1, histone deacetylase 5, E-cadherin and PI4KIIIβ (Iglesias et al., 2000; Jaggi et al., 2005; Vega et al., 2004; Wang et al., 2002). At the TGN, PKD is critically involved in the fission of transport carriers en route to the cell surface (Liljedahl et al., 2001; Yeaman et al., 2004). PKD is recruited to the TGN by its cysteine-rich regions (Baron and Malhotra, 2002; Hausser et al., 2002; Maeda et al., 2001), where it is activated by PKCç-mediated phosphorylation (az Anel and Malhotra, 2005).
Recently PI4KIIIâ was identified, a key player in structure and function of the Golgi apparatus, as a PKD substrate at this organelle (Hausser et al., 2005). PKD-mediated phosphorylation of PI4KIIIâ at serine 294 stimulates its lipid kinase activity, resulting in enhanced phosphatidylinositol 4-phosphate (PI(4)P) production and vesicular stomatitis virus G-protein transport to the plasma membrane (Hausser et al., 2005).

Protein kinase D (PKD) has been identified as a crucial regulator of secretory transport at the trans-Golgi-network (TGN). Recruitment and activation of PKD at the TGN is mediated by the lipid diacylglycerol (DAG), a pool of which is generated by sphingomyelin synthase from ceramide and phosphatidylcholine. The non-vesicular transfer of ceramide from the endoplasmic reticulum to the Golgi complex is mediated by the lipid transfer protein CERT. This is described for example in Hanada et al, 2003, Nature Vol 426, 803-809 and Hanada 2006, Molecular and Cellular Biochemistry 286, 23-31 as well as in the corresponding patent applications WO2005004898 and EP1652530. In neither one of these documents, however, Hanada shows or points towards an implication of modulating CERT expression or activity (let alone other START domain proteins) in a method of producing proteins for diagnostic, research or therapeutic purposes. Furthermore, these documents / patent applications do not describe in any way the use of a blocking agent which reduces or completely blocks CERT expression or activity in a pharmaceutical composition. Hanada rather concludes to use CERT itself as a drug to promote ceramide transport.

The present invention, however, identifies CERT as a novel *in vivo* PKD substrate. Phosphorylation on serine 132 by PKD decreases the affinity of CERT towards its lipid target phosphatidylinositol 4-phosphate at Golgi membranes and reduces ceramide transfer activity, identifying PKD as a regulator of lipid homeostasis. The present invention also shows that CERT in turn is critical for PKD activation and PKD dependent protein cargo transport to the plasma membrane. The interdependence of PKD and CERT is thus a key to the maintenance of Golgi membrane integrity and secretory transport.

### DESCRIPTION OF THE FIGURES

### FIGURE 1:

Intracellular product accumulation.
Increase of intracellular product during Fed-batch fermentations shown for three processes. Fed-batch fermentation was performed using three different CHO producer cell clones expressing human IgG antibodies: Process A (circles), B (diamonds) and M (triangles), respectively). Every other day, cell samples were taken, fixed and subjected to direct immunofluorescence to detect the antibody light-chain. The amount of product was measured by FACS and plotted relative to the amount at day 1.

### FIGURE 2:

### The START domain protein family

Phylogenetic assembly of (A) human START domain proteins, (B) their domain organization (4 TM, four transmembrane; Pre, mitochondrial presequence; Thio, acyl-CoA thioesterase), and (C) their homologs in fly and worm. (taken from (Soccio and Breslow, 2003))

### FIGURE 3:

CERT is a crucial regulator of Golgi function and acts downstream of XBP-1 in the secretory pathway.
(A) CERT and PKD are connected in a regulatory feedback-loop. The scheme summarizes the current working hypothesis where PKD is activated by DAG and phosphorylates CERT. Phosphorylated CERT dissembles from PI(4)P and releases ceramide at the site of its destination. Ceramide at the Golgi is converted to sphingomyelin and DAG which in turn is necessary for PKD activation. This circuit can be interrupted by mutation of the CERT phosphorylation site (S132A).
(B) The schematic drawing shows the way of a secreted protein from transcription and translation through the ER and Golgi compartments to the plasma membrane where the protein is finally released from the cell into the medium. The arrows represent recent genetic engineering approaches aiming to enhance protein production. Most efforts focused on transcription enhancing technologies, few on translation engineering, and at present, only three examples have been reported which target proteins involved in post-translational processing within the ER (BiP, PDI and XBP-1). CERT acts downstream of the ER in the secretory pathway and thus to our knowledge represents the first target for engineering at later stages of the secretion process

### FIGURE 4:

CERT is detected by a PKD substrate antibody.
(A) HEK293T cells were transfected with expression plasmids encoding Flag-tagged CERTL and CERT. Cells were lysed 24 h post transfection and CERT isoforms were immunoprecipitated with anti-Flag antibody. Immunoprecipitated proteins were subjected to SDS-PAGE, followed by immunoblotting with PKD substrate antibody (pMOTIF; top panel) and, after stripping, with anti-Flag antibody (bottom panel).
(B) HEK293T cells were transfected with Flag-CERT expression plasmid, along with GFP-PKD1 K612W (PKD-KD) or empty vector. CERT was analyzed by Western blotting as described in (A). Expression of PKD-KD was verified by immunoblotting with a PKD-specific antibody (C20; bottom panel).
(C) COS7 cells were cotransfected with Flag-CERT and PKD1-GFP expression plasmids, fixed and stained with Flag-specific antibody (red). The images shown are stacks of several confocal sections. Scale bar, 20 µm.

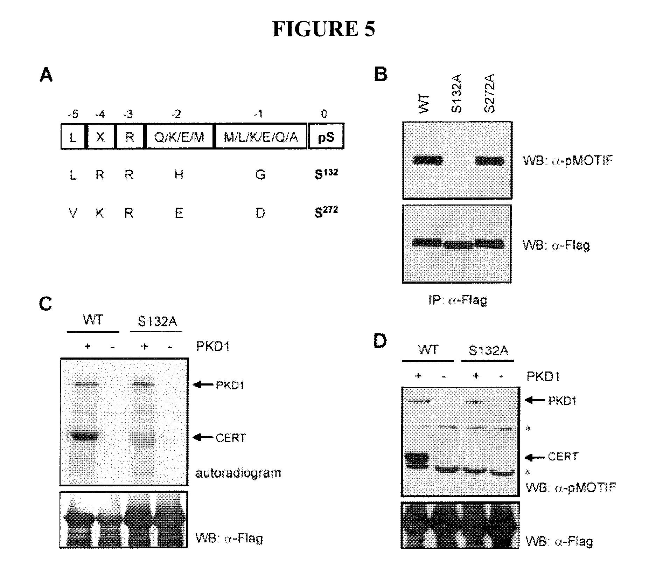

### FIGURE 5: PKD PHOSPHORYLATES CERT ON SERINE 132.

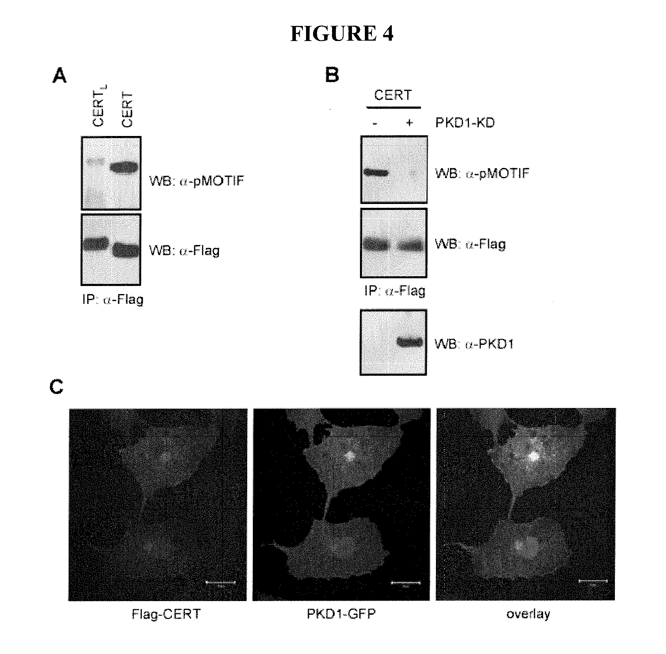

(A) Alignment of the peptide sequences used to raise the PKD substrate antibody and two potential PKD motifs in CERT.
(B) HEK293T cells were transfected with expression plasmids encoding Flag-tagged CERT wild type (WT), CERT-S132A, and CERT-S272A. The cells were lysed and CERT proteins were immunoprecipitated and analyzed by Western blotting as described in FIGURE 4.
(C) Recombinant GST-Flag-CERT wild type (WT) and S132A fusion proteins were incubated in kinase buffer containing [32P]-ã-ATP in the absence (-) and presence (+) of purified PKD1 for 30 min. Proteins were separated by SDS-PAGE and transferred to membrane. Incorporation of radioactive phosphate was analyzed using a PhosphoImager (top), followed by immunoblotting with Flag-specific antibody to verify equal loading of the CERT proteins.
(D) Recombinant CERT proteins were subjected to an *in vitro* kinase with purified PKD1 as described in (C) in the presence of cold ATP. Immunoblotting was performed with the pMOTIF antibody and, after stripping, with Flag-specific antibody to verify equal loading of the CERT proteins. PKD1 and CERT proteins are marked with arrows; the bands with asterisks are due to non-specific binding.

### FIGURE 6:

CERT phosphorylation on serine 132 modulates PI(4)P binding and ceramide transfer activity.
HEK293T cells were transfected with expression plasmids encoding GFPtagged CERT wild type (WT, SEQ ID NO.10, 12) and CERT-S132A (SEQ ID NO.14). Cells were harvested by hypotonic lysis 24 h post transfection and the cytosol fraction was recovered after centrifugation at 100.000xg.
Samples containing equal amounts of GFP fluorescence were used for (A) Protein-lipid overlay assays. Cytosol from HEK293T cells transiently expressing the CERT variants was incubated with membranes spotted with a concentration gradient of the different phosphoinositides and bound CERT proteins were detected via their GFP tag.
(B) Donor liposomes containing TNPPE and pyrene-ceramide were mixed with a 10-fold excess of unlabeled acceptor liposomes. After 60 sec, cytosol from cells transiently expressing GFP-tagged CERT wild type (WT), S 132A, or GFP alone (con) was added and pyrene fluorescence at 395 nm was recorded (excitation: 340 nm). Spectra were normalized to maximum fluorescence in Triton X-100 and to maximum GFP fluorescence.

### FIGURE 7

CERT regulates PKD activation and secretory transport.
(A) Western Blot of whole cell lysates from HEK293T cells transfected with either Flag-tagged CERT wild type (SEQ ID NO.10, 12) or the CERT mutant S132A (SEQ ID NO. 14). The blot was probed with phosphospecific pS916 PKD antibody (top panel), a PKD-specific antibody (middle panel) and a Flag-specific antibody (bottom panel), respectively, to verify expression of the Flag-tagged CERT constructs.
(B) Measurement of HRP-activity in the supernatants of HEK293T cells cotransfected with Flag-ss-HRP and empty vector (black bars), PKD1-GFP kinase dead (KD, white bars), Flag-CERT wild type (WT, shaded bars) or Flag-CERT-S132A (dark grey). Relative light units (RLU) were plotted at the indicated time points after medium change. The values correspond to the mean of triplicate samples, error bars = SEM.
(C) Confocal immunofluorescence of GFP-CERT (green) and the cis/medial-Golgi marker GS28 (red) in COS7 cells. The images shown are stacks of several confocal sections. Scale bar, 20 µm.
(D) Stacks of confocal images showing the co-localization of GFP-CERT (green) and HRP-Flag (red) in COS7 cells. Scale bar, 20 µm and 5 µm (enlargement).

### FIGURE 8:

CERT downregulation by RNA interference inhibits secretory transport.
(A) Quantitative detection of HRP activity in the supernatants of COS7 cells treated with either mock- (white), lacZ- (light grey = lacZ-specific siRNA SEQ ID No 9) or CERT-specific siRNA oligonucleotides (dark grey = siCERT#1 SEQ ID No 7 and black = siCERT#2 SEQ ID No. 8). The relative light units (RLU) of triplicate experiments are shown, error bars = SEM.
(B) Western Blot of the cell lysates of (A) probed with an anti-transferrin receptor antibody. Equal loading was confirmed by using an anti-Tubulin-specific antibody.

### DETAILED DESCRIPTION OF THE INVENTION

Post-translational modification of proteins by phosphorylation is a common mechanism to induce conformational changes that modulate enzymatic activity, mediate protein-protein interactions or regulate subcellular localization. PKD is a key regulator at the Golgi complex with PI4KIIIβ being the only local substrate identified thus far. To test whether the Golgi complex-localized CERT protein may serve as a substrate for PKD, we made use of a phosphospecific substrate antibody, termed pMOTIF, raised against consensus motifs phosphorylated by PKD (Doppler et al., 2005). HEK293T cells were transfected with expression vectors encoding Flag-tagged CERT and CERT_{L}. The CERT isoforms were immunoprecipitated with Flag-specific antibodies and analyzed by Western blotting with the pMOTIF antibody (FIGURE 4A). A pMOTIF signal corresponding to the molecular weight of CERT and, more weakly, to that of CERT_{L} was detected (FIGURE 4A). The weaker detection of the phosphorylated CERT_{L} isoform may be related to its known behaviour to form aggregates, which may impact phosphosite accessibility to kinases (Raya et al., 2000). To investigate whether recognition of CERT by the pMOTIF antibody was dependent upon PKD, we expressed CERT together with a kinase dead variant of PKD1 (K621W) in HEK293T cells. This mutant has been shown to localize to the Golgi complex and suppressed PI4KIIIβ phosphorylation in a dominant negative fashion (Hausser et al., 2005). Coexpression of inactive PKD abolished detection of CERT with the pMOTIF antibody, suggesting that the pMOTIF signal was indeed due to PKD-mediated CERT phosphorylation (FIGURE 4B). Lipid transfer proteins are thought to act at MCS, which are formed between the ER and TGN (Levine and Loewen, 2006), where PKD is localized. Immunofluorescence staining of Flag-tagged CERT in COS7 cells coexpressed with GFP-tagged PKD1 verified that the two proteins colocalize at the Golgi complex (FIGURE 4C). RNA interference experiments suggest that simultaneous knock-down of PKD1 and PKD2 was required to reduce CERT phosphorylation, indicating that these two isoforms were primarily responsible for phosphorylating CERT, whereas PKD3 appeared to play a minor role (data not shown). This is in accordance with previously reported overlapping substrate specificities of PKD1 and PKD2. For example, PKD1 and PKD2 were both shown to phosphorylate PI4KIIIβ, whereas PKD3 failed to do so (Hausser et al., 2005).

To identify pMOTIF recognition sites in CERT, we searched for potential PKD consensus motifs characterized by a leucine, isoleucine or valine residue in the -5 and arginine in the - 3 position relative to a serine or threonine. Two serines at positions 132 and 272, matching the PKD consensus motif and conserved across species (FIGURE 5A), were exchanged for alanines by site-directed mutagenesis. These mutants were expressed in HEK293T cells and tested for recognition by the pMOTIF antibody. Interestingly, mutation of serine 132 to alanine abrogated detection of CERT with the pMOTIF antibody and caused an increase in electrophoretic mobility, indicative of loss of phosphorylation, while the S272A mutation did not affect the pMOTIF signal (FIGURE 5B). This suggested that serine 132 is a PKD phosphorylation site specifically recognized by the PKD substrate antibody. To confirm that PKD was capable of directly phosphorylating this serine residue in CERT, we performed *in vitro* kinase assays with purified PKD1 and recombinant CERT GST-fusion proteins produced in *E. coli* comprising the first 138 amino acids of the protein. When the truncated wild type CERT fusion protein was incubated with PKD1 in the presence of [γ-³²P]-ATP, incorporation of radioactivity was detected (FIGURE 5C). This was significantly impaired in the case of the CERT-S132A fusion protein. *In vitro* PKD phosphorylation of wild type but not CERT-S132A is further shown to generate a recognition site for the pMOTIF antibody (FIGURE 5D). Taken together, these results prove that CERT is a genuine PKD substrate *in vitro* and *in vivo* and identify serine 132 as a specific PKD phosphorylation site in CERT.

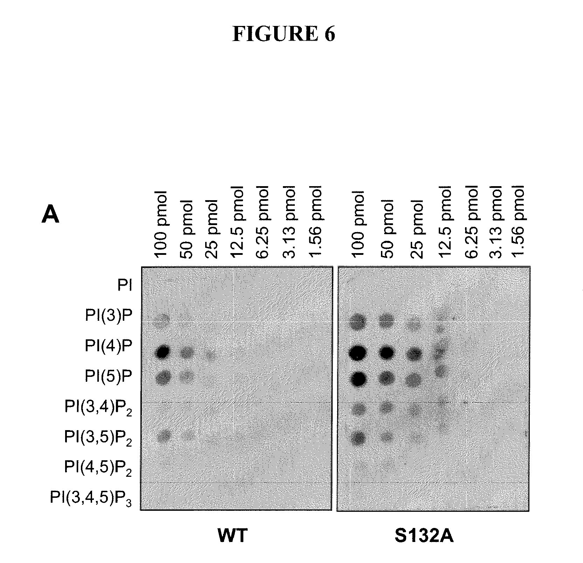

Serine 132 is in very close proximity to the CERT PH domain (amino acids 23 - 117), making it possible that phosphorylation on this site affects PI(4)P binding by increasing the local negative charge. We therefore quantified PI(4)P binding of wild type CERT and the CERT-S132A mutant by performing protein-lipid overlay assays. Here, cytosol from HEK293T cells transiently expressing the CERT variants was incubated with membranes spotted with a concentration gradient of the different phosphoinositides and bound CERT proteins were detected via their GFP tag. As reported previously, the full-length wild type protein demonstrated weak binding to several phospholipid species, but displayed strong interaction with PI(4)P (Hanada et al., 2003; Levine and Munro, 2002). CERT-S 132A binding to PI(4)P was detectable at two- to fourfold lower concentrations as compared to that of the wild type protein, suggesting increased affinity of the CERT-S132A mutant to this phospholipid (FIGURE 6A). Together, these data imply that CERT, once bound to the Golgi complex, is phosphorylated by PKD. This then decreases the affinity of CERT to PI(4)P and thereby regulates the interaction of CERT with Golgi membranes.

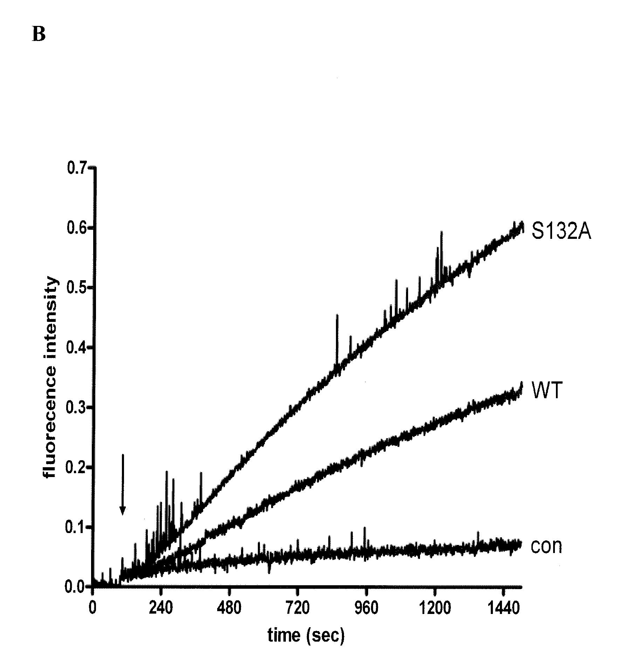

The CERT protein has been shown to function as a lipid transfer protein (Hanada et al., 2003). We thus investigated whether CERT phosphorylation on serine 132 influenced its ability to bind and transfer ceramide between membranes. To this end, GFP-tagged versions of wild type CERT and CERT-S132A were transiently expressed in HEK239T cells and the cytosol fraction was analyzed for ceramide-specific lipid transfer activity using a FRET-based assay (FIGURE 6B). In this assay, small unilamellar vesicles containing pyrene-labeled ceramide as a fluorescent donor and quenching amounts of head group-labeled TNP-PE were employed (Olayioye et al., 2005; Somerharju, 2002). When these donor liposomes were mixed with an excess of unlabeled acceptor liposomes, the increase in pyrene fluorescence was negligible, indicating minimal spontaneous ceramide transfer to acceptor membranes (data not shown). Upon addition of wild type CERT-containing cytosol, a steady increase in fluorescence was noted, which was not observed when control cytosol of vector-transfected cells was used (FIGURE 6B). Compared to the wild type protein, CERT-S132A displayed a higher rate of lipid transfer, evident from a more rapid increase in pyrene fluorescence (FIGURE 6B). This suggests that CERT phosphorylation on serine 132 downregulates ceramide transfer activity by decreasing association of the protein with membranes. Previous data have already shown that PKD regulates the level of PI(4)P at the Golgi complex by phosphorylation-mediated activation of PI4KIIIβ (Hausser et al., 2005). Interestingly, PI4KIIIβ is critical for the transport of ceramide between the ER and the Golgi complex (Toth et al., 2006). Accordingly, together with the data presented here, a dual role for PKD in maintaining lipid homeostasis of Golgi membranes becomes apparent by controlling the on-rate (via PI(4)P levels) and the off-rate (via direct phosphorylation) of CERT.

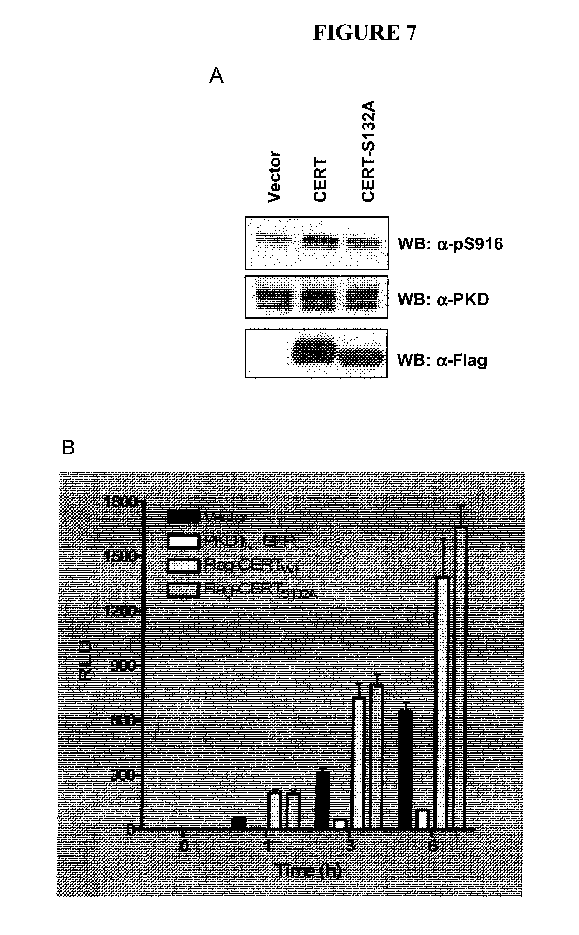

The transfer of ceramide from the ER to the TGN is essential for SM synthesis at this compartment (Hanada et al., 2003). Golgi-localized SM synthase 1 (SMS1) utilizes ceramide and PC to generate SM and DAG (Perry and Ridgway, 2005), the latter being a prerequisite for PKD recruitment and activation (Baron and Malhotra, 2002). Compounds that block DAG production at the TGN inhibit the binding of PKD to TGN membranes and interfere with secretory transport (Baron and Malhotra, 2002). Therefore, increased ceramide transfer from the ER to the TGN by overexpression of CERT should result in an elevated local DAG pool and may consequently stimulate PKD activity and secretory transport. To test this hypothesis, we transiently expressed CERT wild type and CERT-S132A in HEK293T cells and analyzed autophosphorylation of endogenous PKD. Compared to the control, expression of both CERT wild type and CERT-S132A increased PKD activity, as revealed by analyses with a phosphospecific PKD antibody (FIGURE 7A). This shows that PKD activation is regulated by CERT proteins, likely due to increased ceramide delivery and enforced SM/DAG synthesis. A similar function has recently been described for the lipid transfer protein Nir2 in the maintenance of DAG levels at the Golgi apparatus via regulation of the CDP-choline pathway (Litvak et al., 2005). RNAi-mediated knock-down of Nir2 decreased the levels of DAG and PKD at the Golgi complex and blocked secretory transport. Interestingly, this effect could be rescued by the addition of exogenous C₆-ceramide (Litvak et al., 2005), indicating a critical role for ceramide in DAG synthesis and PKD recruitment to the Golgi complex.

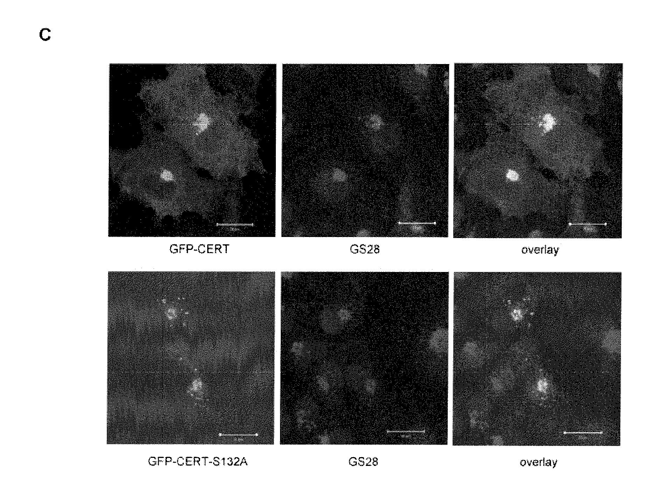

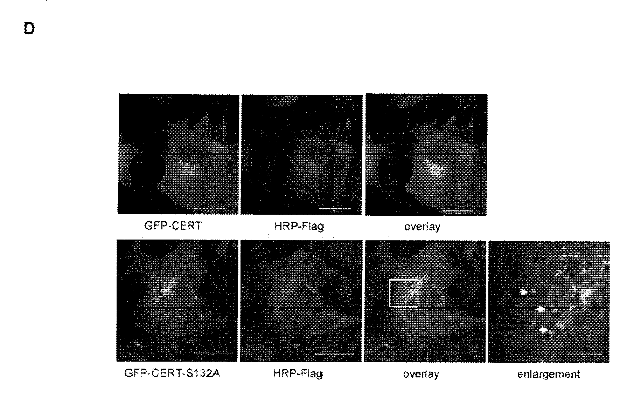

To address the question of whether CERT-mediated PKD activation indeed translated into enhanced secretory transport, we made use of a plasmid encoding horseradish peroxidase fused to a signal sequence (ss). The fusion protein ss-HRP can be used as a reporter for constitutive protein secretion (Bard et al., 2006). In control cells, secretion of ss-HRP could be detected within 1 hour and increased over time (FIGURE 7B). Coexpression of kinase dead PKD1, which inhibits secretory transport of cargo protein (Hausser et al., 2005; Liljedahl et al., 2001), almost entirely abrogated the secretion of ss-HRP into the supernatant. This confirmed that HRP was secreted in a PKD-dependent manner in our assay. Coexpression of CERT wild type and CERT-S132A strongly augmented the amount of secreted HRP (FIGURE 7B). Interestingly, we could only detect a slight increase in secretion with the CERT-S132A mutant compared to the one observed with the CERT wild type protein. This is in accordance with the comparable activation of PKD by CERT and CERT-S132A (FIGURE 7A), but was unexpected in the light of the significantly enhanced *in vitro* lipid transfer activity of the CERT mutant (FIGURE 6B). However, increased levels of ceramide may not necessarily translate into equivalent increases in DAG, because DAG synthesis might be limited by the availability of PC and the activity of SM synthase. Accumulation of ceramide is known to affect Golgi membrane stability and induces vesicle fission (Fukunaga et al., 2000; Weigert et al., 1999). We therefore investigated whether overexpression of the CERT-S132A mutant affected its localization and/or caused morphological changes of the Golgi apparatus. CERT has been demonstrated to colocalize with the cis/medial-Golgi marker GS28 (Hanada et al., 2003). Immunofluorescence analysis of GFP-tagged CERT expressed in COS7 cells showed that the protein localized to GS28-positive Golgi regions (FIGURE 7C). By contrast, in addition to the partial colocalization with GS28 at the Golgi complex, the CERT-S132A mutant protein displayed a dispersed, punctate staining. Of note, some of these vesicular structures were found to contain the cargo protein ss-HRP, providing evidence that these structures indeed represent Golgi-derived transport carriers (FIGURE 7D). This finding is in accordance with the observed changes in Golgi membrane structure due to local increases in ceramide levels (Fukunaga et al., 2000; Weigert et al., 1999).

In conclusion, we have identified CERT as a PKD substrate and provide evidence for a novel relationship between membrane lipid biogenesis and protein secretion. We show that CERT plays an important role in vesicular transport processes by providing ceramide as a substrate for the synthesis of the PKD activator DAG at Golgi membranes. We further demonstrate that the system is tightly regulated by a negative feedback loop: Active PKD phosphorylates CERT at serine 132, thus decreasing the affinity of CERT towards its lipid target PI(4)P to ensure continuous rounds of lipid transfer from the ER to the Golgi compartment.

The data of the present invention clearly demonstrate that overexpression of CERT enhances protein secretion. To investigate whether also the opposite is true, meaning that reduced CERT expression would result in diminished secretion, siRNA experiments were performed. The activity of HRP was detected after 3 hours and showed equal comparable levels in both control cells. In contrast, a dramatic reduction of HRP activity was measured in cells that had been treated with any of the CERT-specific siRNA oligonucleotides (FIGURE 8). This indicates that reduced CERT levels lead to reduced HRP secretion from the cells and further underscores the important role of CERT in the secretory transport.

Interestingly, not only protein secretion, but also the abundance of the transmembrane protein transferrin receptor was affected by the reduction of CERT (FIGURE 8B). When the cells from FIGURE 8A were pooled and the lysates probed with transferrin receptor-specific antibodies in Western blot experiments, a strong decrease in the amount of transferrin receptor became apparent, whereas similar transferrin receptor levels were detected in both control cells.
This finding suggests, that the lipid transfer protein CERT is not only implicated in the transport of secreted but also of membran-standing cell-surface proteins. This might not be surprising as both types of proteins are equally transported in lipid vesicles from the ER via the Golgi to the plasma membrane and thus use the same cellular export routes which - as we demonstrate in the present invention for the first time - are influenced by CERT.

The findings and the resulting new model for regulation of secretory protein transport from the Golgi complex to the plasma membrane described in the present invention can be applied to biopharmaceutical protein manufacturing. Overexpression of CERT increases biopharmaceutical protein production of diverse proteins such as antibodies, cytokines, growth factors such as erythropoietin or insulin, surface receptors such as epithelial growth factor, and membrane-bound proteases.
Although the method described in this invention can be generally applied, to all protein production processes, the degree of success of this strategy as measured by the increase in the amount of protein produced can certainly depend on the particular nature of the protein of interest. CHO or other producer cells are transfected with an expression construct encoding a START domain protein such as CERT, StarD4 or StarD5 or a mutant or derivative thereof.
Notably, the highest titers are detected in cells expressing unphosphorylatable CERT mutant S132A. Heterologous expression of CERT, and especially mutant CERT, in CHO cells can enhance protein secretion, for example of a monoclonal antibody, on the transient transfection level. This can be particularly useful for fast production of smaller quantities of drug candidates or drug targets necessary in pharmaceutical research and development.
In a further embodiment of this invention, a producer cell line is transfected with the same DNA constructs as above and subsequently subjected to selection to obtain stable cell pools. For six cell culture passages subsequent to the selection procedure, culture supernatant is collected to be analysed for the content of protein of interest. In case of a monoclonal antibody, the concentration of the protein product is determined by ELISA and divided by the mean number of cells to calculate the specific productivity. Again, the highest values are seen in the cell pools harbouring the CERT mutant. In cells containing a START domain construct expression of the protein of interest is significantly enhanced compared to MOCK or untransfected cells. Very similar results can be obtained if the stable transfectants are subjected to batch or fed-batch fermentations. In each of these settings, overexpression of START domain proteins leads to enhanced expression of antibodies, single cell proteins and surface receptors in transiently as well as stably transfected CHO cell lines, indicating that START domain proteins such as CERT or StarD4 and StarD5 are able to enhance the specific production capacity of the cells under fermentation conditions.

### DEFINITIONS

The general embodiments "comprising" or "comprised" encompass the more specific embodiment "consisting of". Furthermore, singular and plural forms are not used in a limiting way.

Terms used in the course of this present invention have the following meaning.

The term "START domain" stands for steroidogenic acute regulatory protein (StAR) related lipid transfer (START) domain. This domain of about 200-210 amino acids was identified initially as lipid binding domain (Soccio and Breslow, 2003; Tsujishita and Hurley, 2000). The length of the START domain may vary between 116 to 250 amino acids, or between 180 to 223 amino acids, or more specifically between 219 to 223 amino acids depending on the START domain family member. The most striking feature of the START domain structure is a predominantly hydrophobic tunnel extending nearly the entire length of the protein which is used to binding a single molecule of large lipophilic compounds, like cholesterol. The structural resolution of the START domain family member MLN64-START revealed an α/β type structure consisting of nine-stranded twisted antiparallel β-sheets and four α-helices (Tsujishita and Hurley, 2000). The domain found in various eukaryotic proteins is referred to as 'classical START domain' (CSD) while a similar domain specific to plants is known as Birch allergen START domain (BA-START).

The term "CERT" encompasses both splice forms of CERT: CERT (SEQ ID NO.11) and CERT_{L} (SEQ ID No.13). The term "CERT" furthermore encompasses any other possible splice form of CERT derived from the nucleotide sequence SEQ ID No. 12.

The term "CERT" further encompasses hCERT protein and its recombinants, hCERT, hCERTA, PH protein, hCERT A MR protein, and hCERTA STprotein, and further, PHhCERT protein, MRhCERT protein and SThCERT protein (see also EP1652530, (Hanada, 2006), (Hanada et al., 2003)).

The term "derivative" in general includes sequences suitable for realizing the intended use of the present invention, which means that the sequences mediate the increase in secretory transport in a cell.
The term "derivative" as used in the present invention means a polypeptide molecule or a nucleic acid molecule which is at least 70% identical in sequence with the original sequence or its complementary sequence. Preferably, the polypeptide molecule or nucleic acid molecule is at least 80% identical in sequence with the original sequence or its complementary sequence. More preferably, the polypeptide molecule or nucleic acid molecule is at least 90% identical in sequence with the original sequence or its complementary sequence. Most preferred is a polypeptide molecule or a nucleic acid molecule which is at least 95% identical in sequence with the original sequence or its complementary sequence and displays the same or a similar effect on secretion as the original sequence.
Sequence differences may be based on differences in homologous sequences from different organisms. They might also be based on targeted modification of sequences by substitution, insertion or deletion of one or more nucleotides or amino acids, preferably 1, 2, 3, 4, 5, 7, 8, 9 or 10. Deletion, insertion or substitution mutants may be generated using site specific mutagenesis and /or PCR-based mutagenesis techniques. Corresponding methods are described by (Lottspeich and Zorbas, 1998) in Chapter 36.1 with additional references.

The sequence identity of a reference sequence (in the present invention being for example START domain SEQ ID No.16, 17 or 18, 19) can be determined by using for example standard "alignment" algorithmnes, e.g. "BLAST" ((Altschul et al., 1990); (Madden et al., 1996); (Zhang and Madden, 1997)). Sequences are aligned when they fit together in their sequence and are identifiable with the help of standard "alignment" algorithms.
Furthermore, in the present invention the term "derivative" means a nucleic acid molecule (single or double strand) which hybridizes to SEQ ID No.10,12, 14, 16, 18, 20, 22, 24, 26) or with fragments or derivates thereof or with sequences which are complementary to SEQ ID No. 10,12, 14, 16, 18, 20, 22, 24, 26. Preferably the hybridization is performed under stringent hybridization- and washing conditions (e.g. hybridisation at 65°C in a buffer containing 5x SSC; washing at 42°C using 0,2x SSC/0,1% SDS). Corresponding techniques are described exemplary in (Ausubel et al., 2002).
The term "derivatives" further means protein deletion mutants, phosphorylation mutants especially at a serine, threonine or tyrosine position, the deletion of a PKD binding site or the CERT Ser132A mutation.

The term "activity" describes and quantifies the biological functions of the protein within the cell or in in vitro assays
An example of how to measure "activity" is described in the patent application EP1652530 (Hanada et al.), which detects ceramide release promotion activity from membranes. The lipid membrane containing ceramide has to be prepared so that it contains 12.5 nCi (225 pmol) per sample of [palmitoyl-1 -I4C] N-palmitoyl-D-ethyro-sphigosine (hereinafter, may be referred to as I4C-ceramide) on the basis of a mixed lipid consisting of phosphatidylcholine and phosphati- dylethanolamine at the ratio of 4:1 derived from egg yolk. Its concentration of ceramide thus is 2.5 mg/mL. For one sample of the activity measurement this lipid membrane is required at an amount of 20 pL. After the amount of lipid required for activity measurement has been dispensed in an Eppendorf tube, it has to be dried by spraying nitrogen gas. After this, the buffer 1 [20 mM Hepes- NaOH buffer (pH7.4) to which 50 mM NaCl and 1 mM EDTA have been added] has to be added to the dried lipid membrane, so that the concentration becomes 2.5 mg/mL. A gently supersonic treatment has to be performed using bath type supersonic generator [Model 221 0 manufactured by Branson, Co., Ltd.]. The supersonic treatment has to be performed at 25°C for 3 minutes. The sample then has to be mixed (vortex) for 30 seconds and then the supersonic treatment is repeated for 3 minutes. The lipid membrane prepared in this way is used in a ceramide release assay. The ceramide release reaction for the lipid membrane and its detection is performed as follows: CERT protein or a recombinant protein thereof (under the standard conditions, the amount of protein corresponding to 450 picomoles, which is 2-fold molar equivalent amount of ceramide contained in the donating membrane was used) is mixed up to 30 pL using buffer 2 [50 mM Hepes- NaOH buffer (pH7.4) to which 100 mM NaCl and 0.5 mM EDTA have been added] . Here, the reaction is initiated by adding 20 pL of lipid membrane containing ceramide. The final concentration of phospholipids is 1 mg/mL. Ceramide is contained at a ratio of about 0.3% comparing to the total phospholipid amount. After the mixture of these has been incubated at 37°C for 30 minutes, it is centrifuged at 50,000 xg for 30 minutes and the lipid membrane is precipitated. In the case where CERT protein from E.coli is used, most of the protein remains in the supernatant under these centrifugation conditions. Therefore, when I4C-ceramide binds to CERT protein, it is releases from the lipid membrane and transfered to the supernatant fraction. The activity for promoting ceramide release with CERT is calculated by measuring the radioactive activity of 1% in the supernatant fraction using a liquid scintillation counter.

A further possibility to measure "activity" is an *in vitro* ceramide transfer assay using recombinant material or cell lysate containing CERT. Hereby, the protein-mediated transfer of ceramide between SUVs is measured as described previously (Olayioye et al., 2005). The transfer assay mixture contained donor vesicles (2 nmol lipid/ml) composed of porcine brain lipids (Avanti Polar Lipids), pyrene-labeled C₁₆-ceramide, and 2,4,6-trinitrophenyl-phosphatidylethanolamine (TNP-PE) (88.6:0.4:11 mol %), provided by P. Somerharju, and a 10-fold excess of acceptor vesicles composed of porcine brain lipids. Fluorescence intensity is recorded at 395 nm (excitation, 345 nm; slit widths, 4 nm) before and after the addition of 75 µg cytosol from HEK293T cells transiently expressing the GFP-tagged CERT wild type and S132A proteins (see above). Fluorescence intensities are normalized to (i) the maximum intensity obtained after the addition of Triton X-100 (0.5 % final concentration) and (ii) the maximum GFP fluorescence, to account for different protein expression levels.

Another possibility to measure "activity" is a phosphorylation state analysis of CERT S132A e.g. by using an anti-phospho specific antibody in a Western blot. Whole cell extracts are obtained by solubilizing cells in NP40 extraction buffer (NEB) [50 mM Tris (pH 7.5), 150 mM Nad, 1% NP40, 1 mM sodium orthovanadate, 10 mM sodium fluoride, and 20 mM β-glycerophosphate plus Complete protease inhibitors]. Lysates are clarified by centrifugation at 16,000 x g for 10 min. Whole cell extracts or immunoprecipitated proteins are boiled in sample buffer and subjected to SDS-PAGE. The proteins are blotted onto polyvinylidine difluoride membranes (Roth). After blocking with 0.5% blocking reagent (Roche) in PBS containing 0.1% Tween 20, filters are probed with a phosphospecific antibody such as phosphospecific substrate antibody, termed pMOTIF, raised against consensus motifs phosphorylated by PKD (Doppler et al., 2005). Proteins are visualized with peroxidase-coupled secondary antibody using the enhanced chemiluminescence detection system (Pierce).

Still another assay for measuring the "activity" is a secretion assay e.g. for a model protein, an antibody or a protein of interest. Cells are cotransfected with ss-HRP-Flag plasmid and empty vector, pEGFP-N1-PKD1KD and a plasmid encoding CERT, a variant of CERT of any START family protein at a ratio of 1: 6.5, respectively. 24h post-transfection cells are washed with serum-free media and HRP secretion is quantified after 0, 1, 3 and 6 h by incubation of clarified cell supernatant with ECL reagent. Measurements are done with a luminometer (Lucy2, Anthos) at 450 nm.

Another way to measure the "activity" is by using a fluorescent ceramide analog e.g. Bodipy-labeled C5-ceramide, perform chase experiments in intact cells and measure the accumulation of protein in the Golgi complex.
Quantification of the distribution of BODIPY® FL C5-ceramide between the Golgi and the ER: The transport of the fluorescent ceramide was quantified post-aquisition using the linescan function of the Metamorph software. A line was drawn through the cells in the confocal pictures taken in different time points and the fluorescent intensity was measured in the cytoplasm and over the Golgi complex of the cells. The "uptake ratio" was calculated from the fluorescent light intensity in the Golgi divided by the intensity measured in the cytoplasm. The maximum uptake ratio was measured in control cells after 25 min incubation on 37°C and this value was taken as 100 percent. The quantification was made from the data of three independent experiments in which confocal pictures were taken in twelve different time points and in each time points 7 cells were analyzed.

The terms "inhibitor" or "suppressor" as used in the present invention means any molecule that acts to inhibit or suppress the expression or activity of a START domain protein like CERT. The term includes small chemical compounds, nucleic acids such as antisense DNA, antisense RNA or siRNA, single chain antibodies and proteins that block CERT transcription and translation as well as peptides or proteins that interfere with lipid binding of START domain proteins such as CERT.

"Host cells" in the meaning of the present invention are cells such as hamster cells, preferably BHK21, BHK TK⁻, CHO, CHO-K1, CHO-DUKX, CHO-DUKX B1, and CHO-DG44 cells or the derivatives/progenies of any of such cell line. Particularly preferred are CHO-DG44, CHO-DUKX, CHO-K1 and BHK21, and even more preferred CHO-DG44 and CHO-DUKX cells. In a further embodiment of the present invention host cells also mean murine myeloma cells, preferably NS0 and Sp2/0 cells or the derivatives/progenies of any of such cell line. Examples of murine and hamster cells which can be used in the meaning of this invention are also summarized in Table 1. However, derivatives/progenies of those cells, other mammalian cells, including but not limited to human, mice, rat, monkey, and rodent cell lines, or eukaryotic cells, including but not limited to yeast, insect and plant cells, can also be used in the meaning of this invention, particularly for the production of biopharmaceutical proteins.

**TABLE 1: Eukaryotic production cell lines**

| CELL LINE | ORDER NUMBER |
|---|---|
| NS0 | ECACC No. 85110503 |
| Sp2/0-Ag14 | ATCC CRL-1581 |
| BHK21 | ATCC CCL-10 |
| BHK TK⁻ | ECACC No. 85011423 |
| HaK | ATCC CCL-15 |
| 2254-62.2 (BHK-21 derivative) | ATCC CRL-8544 |
| CHO | ECACC No. 8505302 |
| CHO wild type | ECACC 00102307 |
| CHO-K1 | ATCC CCL-61 |
| CHO-DUKX (= CHO duk⁻, CHO/dhfr⁻) | ATCC CRL-9096 |
| CHO-DUKX B11 | ATCC CRL-9010 |
| CHO-DG44 | (Urlaub et al., 1983) |
| CHO Pro-5 | ATCC CRL-1781 |
| V79 | ATCC CCC-93 |
| B14AF28-G3 | ATCC CCL-14 |
| HEK 293 | ATCC CRL-1573 |
| COS-7 | ATCC CRL-1651 |
| U266 | ATCC TIB-196 |
| HuNS1 | ATCC CRL-8644 |
| CHL | ECACC No. 87111906 |

Host cells are most preferred, when being established, adapted, and completely cultivated under serum free conditions, and optionally in media which are free of any protein/peptide of animal origin. Commercially available media such as Ham's F12 (Sigma, Deisenhofen, Germany), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA), CHO-S-Invtirogen), serum-free CHO Medium (Sigma), and protein-free CHO Medium (Sigma) are exemplary appropriate nutrient solutions. Any of the media may be supplemented as necessary with a variety of compounds examples of which are hormones and/or other growth factors (such as insulin, transferrin, epidermal growth factor, insulin like growth factor), salts (such as sodium chloride, calcium, magnesium, phosphate), buffers (such as HEPES), nucleosides (such as adenosine, thymidine), glutamine, glucose or other equivalent energy sources, antibiotics, trace elements. Any other necessary supplements may also be included at appropriate concentrations that would be known to those skilled in the art. In the present invention the use of serum-free medium is preferred, but media supplemented with a suitable amount of serum can also be used for the cultivation of host cells. For the growth and selection of genetically modified cells expressing the selectable gene a suitable selection agent is added to the culture medium.

The term "protein" is used interchangeably with amino acid residue sequences or polypeptide and refers to polymers of amino acids of any length. These terms also include proteins that are post-translationally modified through reactions that include, but are not limited to, glycosylation, acetylation, phosphorylation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with like properties.

The term "polypeptide" means a sequence with more than 10 amino acids and the term "peptide" means sequences up to 10 amino acids length.

The present invention is suitable to generate host cells for the production of biopharmaceutical polypeptides/proteins. The invention is particularly suitable for the high-yield expression of a large number of different genes of interest by cells showing an enhanced cell productivity.

"Gene of interest" (GOI), "selected sequence", or "product gene" have the same meaning herein and refer to a polynucleotide sequence of any length that encodes a product of interest or "protein of interest", also mentioned by the term "desired product". The selected sequence can be full length or a truncated gene, a fusion or tagged gene, and can be a cDNA, a genomic DNA, or a DNA fragment, preferably, a cDNA. It can be the native sequence, i.e. naturally occurring form(s), or can be mutated or otherwise modified as desired. These modifications include codon optimizations to optimize codon usage in the selected host cell, humanization or tagging. The selected sequence can encode a secreted, cytoplasmic, nuclear, membrane bound or cell surface polypeptide.
The "protein of interest" includes proteins, polypeptides, fragments thereof, peptides, all of which can be expressed in the selected host cell. Desired proteins can be for example antibodies, enzymes, cytokines, lymphokines, adhesion molecules, receptors and derivatives or fragments thereof, and any other polypeptides that can serve as agonists or antagonists and/or have therapeutic or diagnostic use. Examples for a desired protein/polypeptide are also given below.
In the case of more complex molecules such as monoclonal antibodies the GOI encodes one or both of the two antibody chains.

The "product of interest" may also be an antisense RNA.

"Proteins of interest" or "desired proteins" are those mentioned above. Especially, desired proteins/polypeptides or proteins of interest are for example, but not limited to insulin, insulin-like growth factor, hGH, tPA, cytokines, such as interleukines (IL), e.g. IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosisfactor (TNF), such as TNF alpha and TNF beta, TNF gamma, TRAIL; G-CSF, GM-CSF, M-CSF, MCP-1 and VEGF. Also included is the production of erythropoietin or any other hormone growth factors. The method according to the invention can also be advantageously used for production of antibodies or fragments thereof. Such fragments include e.g. Fab fragments (Fragment antigen-binding = Fab). Fab fragments consist of the variable regions of both chains which are held together by the adjacent constant region. These may be formed by protease digestion, e.g. with papain, from conventional antibodies, but similar Fab fragments may also be produced in the mean time by genetic engineering. Further antibody fragments include F(ab')2 fragments, which may be prepared by proteolytic cleaving with pepsin.

The protein of interest is preferably recovered from the culture medium as a secreted polypeptide, or it can be recovered from host cell lysates if expressed without a secretory signal. It is necessary to purify the protein of interest from other recombinant proteins and host cell proteins in a way that substantially homogenous preparations of the protein of interest are obtained. As a first step, cells and/or particulate cell debris are removed from the culture medium or lysate. The product of interest thereafter is purified from contaminant soluble proteins, polypeptides and nucleic acids, for example, by fractionation on immunoaffinity or ion-exchange columns, ethanol precipitation, reverse phase HPLC, Sephadex chromatography, chromatography on silica or on a cation exchange resin such as DEAE. In general, methods teaching a skilled person how to purify a protein heterologous expressed by host cells, are well known in the art. Such methods are for example described by (Harris and Angal, 1995) or (Robert Scopes, 1988).

Using genetic engineering methods it is possible to produce shortened antibody fragments which consist only of the variable regions of the heavy (VH) and of the light chain (VL). These are referred to as Fv fragments (Fragment variable = fragment of the variable part). Since these Fv-fragments lack the covalent bonding of the two chains by the cysteines of the constant chains, the Fv fragments are often stabilised. It is advantageous to link the variable regions of the heavy and of the light chain by a short peptide fragment, e.g. of 10 to 30 amino acids, preferably 15 amino acids. In this way a single peptide strand is obtained consisting of VH and VL, linked by a peptide linker. An antibody protein of this kind is known as a single-chain-Fv (scFv). Examples of scFv-antibody proteins of this kind known from the prior art are described in (Huston et al., 1988).

In recent years, various strategies have been developed for preparing scFv as a multimeric derivative. This is intended to lead, in particular, to recombinant antibodies with improved pharmacokinetic and biodistribution properties as well as with increased binding avidity. In order to achieve multimerisation of the scFv, scFv were prepared as fusion proteins with multimerisation domains. The multimerisation domains may be, e.g. the CH3 region of an IgG or *coiled coil* structure (helix structures) such as *Leucin-zipper* domains. However, there are also strategies in which the interaction between the VH/VL regions of the scFv are used for the multimerisation (e.g. dia-, tri- and pentabodies). By diabody the skilled person means a bivalent homodimeric scFv derivative. The shortening of the *Linker* in an scFv molecule to 5- 10 amino acids leads to the formation of homodimers in which an inter-chain VH/VL-superimposition takes place. Diabodies may additionally be stabilised by the incorporation of disulphide bridges. Examples of diabody-antibody proteins from the prior art can be found in (Perisic et al., 1994).

By minibody the skilled person means a bivalent, homodimeric scFv derivative. It consists of a fusion protein which contains the CH3 region of an immunoglobulin, preferably IgG, most preferably IgG1 as the dimerisation region which is connected to the scFv via a *Hinge region* (e.g. also from IgG1) and a *Linker* region. Examples of minibody-antibody proteins from the prior art can be found in (Hu et al., 1996).

By triabody the skilled person means a: trivalent homotrimeric scFv derivative (Kortt et al., 1997). ScFv derivatives wherein VH-VL are fused directly without a linker sequence lead to the formation of trimers.

By "scaffold proteins" a skilled person means any functional domain of a protein that is coupled by genetic cloning or by co-translational processes with another protein or part of a protein that has another function.

The skilled person will also be familiar with so-called miniantibodies which have a bi-, tri- or tetravalent structure and are derived from scFv. The multimerisation is carried out by di-, tri- or tetrameric coiled coil structures (Lovejoy et al., 1993; Pack et al., 1993; Pack et al., 1995).

By definition any sequences or genes introduced into a host cell are called "heterologous sequences" or "heterologous genes" or "transgenes" with respect to the host cell, even if the introduced sequence or gene is identical to an endogenous sequence or gene in the host cell.

A "heterologous" protein is thus a protein expressed from a heterologous sequence.

Heterologous gene sequences can be introduced into a target cell by using an "expression vector", preferably an eukaryotic, and even more preferably a mammalian expression vector. Methods used to construct vectors are well known to a person skilled in the art and described in various publications. In particular techniques for constructing suitable vectors, including a description of the functional components such as promoters, enhancers, termination and polyadenylation signals, selection markers, origins of replication, and splicing signals, are reviewed in considerable details in (Sambrook et al., 1989) and references cited therein. Vectors may include but are not limited to plasmid vectors, phagemids, cosmids, articificial/mini-chromosomes (e.g. ACE), or viral vectors such as baculovirus, retrovirus, adenovirus, adeno-associated virus, herpes simplex virus, retroviruses, bacteriophages. The eukaryotic expression vectors will typically contain also prokaryotic sequences that facilitate the propagation of the vector in bacteria such as an origin of replication and antibiotic resistance genes for selection in bacteria. A variety of eukaryotic expression vectors, containing a cloning site into which a polynucleotide can be operatively linked, are well known in the art and some are commercially available from companies such as Stratagene, La Jolla, CA; Invitrogen, Carlsbad, CA; Promega, Madison, WI or BD Biosciences Clontech, Palo Alto, CA.

In a preferred embodiment the expression vector comprises at least one nucleic acid sequence which is a regulatory sequence necessary for transcription and translation of nucleotide sequences that encode for a peptide/polypeptide/protein of interest.

The term "expression" as used herein refers to transcription and/or translation of a heterologous nucleic acid sequence within a host cell. The level of expression of a desired product/ protein of interest in a host cell may be determined on the basis of either the amount of corresponding mRNA that is present in the cell, or the amount of the desired polypeptide/ protein of interest encoded by the selected sequence as in the present examples. For example, mRNA transcribed from a selected sequence can be quantitated by Northern blot hybridization, ribonuclease RNA protection, in situ hybridization to cellular RNA or by PCR (see (Sambrook et al., 1989); (Ausubel et al., 2002) updated). Proteins encoded by a selected sequence can be quantitated by various methods, e.g. by ELISA, by Western blotting, by radioimmunoassays, by immunoprecipitation, by assaying for the biological activity of the protein, by immunostaining of the protein followed by FACS analysis (see (Sambrook et al., 1989); (Ausubel et al., 2002) updated) or by homogeneous time-resolved fluorescence (HTRF) assays.

"Transfection" of eukaryotic host cells with a polynucleotide or expression vector, resulting in genetically modified cells or transgenic cells, can be performed by any method well known in the art and described, e.g., in (Sambrook et al., 1989)or (Ausubel et al., 2002) updated. Transfection methods include but are not limited to liposome-mediated transfection, calcium phosphate co-precipitation, electroporation, polycation (such as DEAE-dextran)-mediated transfection, protoplast fusion, viral infections and microinjection. Preferably, the transfection is a stable transfection. The transfection method that provides optimal transfection frequency and expression of the heterologous genes in the particular host cell line and type is favoured. Suitable methods can be determined by routine procedures. For stable transfectants the constructs are either integrated into the host cell's genome or an artificial chromosome/mini-chromosome or located episomally so as to be stably maintained within the host cell.

The practice of the present invention will employ, unless otherwise indicated, conventional techniques of cell biology, molecular biology, cell culture, immunology and the like which are in the skill of one in the art. These techniques are fully disclosed in the current literature. See e.g. Sambrook et al., Molecular Cloning: A Laboratory Manual, 2nd Ed., Cold Spring Harbor Laboratory Press, Cold Spring Harbor, N.Y. (1989); Ausubel et al., Current Protocols in Molecular Biology (1987, updated); Brown ed., Essential Molecular Biology, IRL Press (1991); Goeddel ed., Gene Expression Technology, Academic Press (1991); Bothwell et al. eds., Methods for Cloning and Analysis of Eukaryotic Genes, Bartlett Publ. (1990); Wu et al., eds., Recombinant DNA Methodology, Academic Press (1989); Kriegler, Gene Transfer and Expression, Stockton Press (1990); McPherson et al., PCR: A Practical Approach, IRL Press at Oxford University Press (1991); Gait ed., Oligonucleotide Synthesis (1984); Miller & Calos eds., Gene Transfer Vectors for Mammalian Cells (1987); Butler ed., Mammalian Cell Biotechnology (1991); Pollard et al., eds., Animal Cell Culture, Humana Press (1990); Freshney et al., eds., Culture of Animal Cells, Alan R. Liss (1987); Studzinski, ed., Cell Growth and Apoptosis, A Practical Approach, IRL Press at Oxford University Presss (1995); Melamed et al., eds., Flow Cytometry and Sorting, Wiley-Liss (1990); Current Protocols in Cytometry, John Wiley & Sons, Inc. (updated); Wirth & Hauser, Genetic Engineering of Animals Cells, in: Biotechnology Vol. 2, Pühler ed., VCH, Weinheim 663-744; the series Methods of Enzymology (Academic Press, Inc.), and Harlow et al., eds., Antibodies: A Laboratory Manual (1987).

### EMBODIMENTS

The invention relates to a method of producing a heterologous protein of interest in a cell comprising increasing the expression or activity of a protein having an amino acid sequence comprising a steroidogenic acute regulatory related lipid transfer (START) domain or aderivative or mutant thereof, and effecting the expression of said protein of interest. In a preferred embodiment of the present invention the method is characterized in that the heterologous protein is a membrane or secreted protein.

In a specific embodiment of the present invention the method is characterized in that the START domain protein is a mammalian START domain family member such as PCTP (SEQ ID NO. 27), StarD7, GPBP, StarD10, StarD8, StarD13, DLC-1, StarD4 (SEQ ID NO. 21), StarD6 (SEQ ID NO. 25), StarD5 (SEQ ID NO. 23), MLN64, StAR, THEA-2, CACH or StarD9 or a derivative or mutant thereof.

In a further specific embodiment of the present invention the method is characterized in that the START domain protein is characterized by being induced upon ER stress and/or is structurally characterized by consisting solely of a START domain such as StarD4 (SEQ ID NO. 21), StarD5 (SEQ ID NO. 23), StarD6 (SEQ ID NO. 25) or phosphatidylcholin transfer protein (PCTP) (SEQ ID NO. 27).

In another specific embodiment of the present invention the method is characterized in that the START domain protein is selected from the group consisting of CERT (SEQ ID NO. 11 or 13), StarD4 (SEQ ID NO. 21) and StarD5 (SEQ ID NO. 23).
In a further embodiment of the present invention the method is characterized in that the START domain protein is StarD6 (SEQ ID NO. 25). In a preferred embodiment StarD6 is encoded by a nucleotide with the SEQ ID NO. 24.

In a preferred embodiment of the present invention the method is characterized in that the START domain comprises at least the 219 amino acid START domain of CERT_{L} (SEQ ID NO. 19), or at least the 223 amino acid START domain of CERT and CERT S132A (SEQ ID NO. 17), or at least the START domain of StarD4 (SEQ ID NO. 21) or at least the START domain of StarD5 (SEQ ID NO. 23) or a derivative or mutant thereof.

In a particularly preferred embodiment of the present invention the method is characterized in that the START domain protein is ceramide transfer protein CERT (CERT=SEQ ID NO. 11 or CERT_{L}=SEQ ID NO. 13) or a derivative or mutant thereof.

In another specific embodiment of the present invention the method is characterized in that the START domain protein is mutated ceramide transfer protein CERT and said mutation disables and /or deletes a phosphorylation site at any serine, threonine or tyrosine position of CERT.

In a further specific embodiment of the present invention the method is characterized in that the START domain protein is mutated ceramide transfer protein CERT and said mutation disables and /or deletes the protein kinase D (PKD) phosphorylation site of CERT at position 132.

In a particularly preferred embodiment of the present invention the method is characterized in that the mutated CERT is CERT _{S132A} (SEQ ID NO. 15).

In another embodiment of the present invention the method is characterized in that said method results in increased specific cellular productivity of said protein of interest in said cell in comparison to a control cell expressing said protein of interest, but whereby said control cell does not have increased expression or activity of a protein having an amino acid sequence comprising a steroidogenic acute regulatory related lipid transfer (START) domain or a derivative or mutant thereof.

In another specific embodiment of the present invention the method is characterized in that the increase in productivity is about 5% to about 10%, about 11% to about 20%, about 21 % to about 30%, about 31 % to about 40%, about 41 % to about 50%, about 51 % to about 60%, about 61 % to about 70%, about 71 % to about 80%, about 81 % to about 90%, about 91% to about 100%, about 101% to about 149%, about 150% to about 199%, about 200% to about 299%, about 300% to about 499%, or about 500% to about 1000%.

In a preferred embodiment of the present invention the method is characterized in that said cell is a eukaryotic cell such as a yeast, plant, worm, insect, avian, fish, reptile or mammalian cell. In a specific embodiment of the present invention the method is characterized in that said cell is an animal cell. In a further specific embodiment of the present invention the method is characterized in that said cell is a metazoan cell such as *C. elegans.* In another specific embodiment of the present invention the method is characterized in that said cell is a bilateria cell such as *Drosophila melanogaster.* In a further embodiment of the present invention the method is characterized in that said cell is a vertebrate cell such as an avian, fish, reptile or mammalian cell. In a specific embodiment of the present invention the method is characterized in that said cell is a human cell such as the human myeloma cellline U266, HEK293, HeLa, HepG2 or HT1080. In a preferred embodiment of the present invention the method is characterized in that said cell is a rodent cell such as murine NSO, Sp2/0 or Ag8653 cell, YO or YB2.0.

In a further embodiment of the present invention the method is characterized in that said eukaryotic cell is a mammalian cell.

In a specific embodiment of the present invention the method is characterized in that said mammalian cell is a Chinese Hamster Ovary (CHO), monkey kidney CV1, monkey kidney COS, human lens epitheliaim PER.C6TM, human embryonic kidney, HEK293, baby hamster kidney, African green monkey kidney, human cervical carcinoma, canine kidney, buffalo rat liver, human lung, human liver, mouse mammary tumor or myeloma cell, a dog, pig or macaque cell, rat, rabbit, cat, goat, preferably a CHO cell.

In a preferred embodiment of the present invention the method is characterized in that said CHO cell is CHO wild type, CHO K1, CHO DG44, CHO DUKX-B11, CHO Pro-5, preferably CHO DG44.

In a specific embodiment of the present invention the method is characterized in that the protein of interest is a membrane or secreted protein. In a preferred embodiment of the present invention the method is characterized in that the protein of interest is an antibody or antibody fragment.

In a further preferred embodiment of the present invention the method is characterized in that the antibody is monoclonal, polyclonal, mammalian, murine, chimeric, humanized, primatized, primate, human or an antibody fragment or derivative thereof such as antibody, immunoglobulin light chain, immunoglobulin heavy chain, immunoglobulin light and heavy chains, Fab, F(ab')2, Fc, Fc-Fc fusion proteins, Fv, single chain Fv, single domain Fv, tetravalent single chain Fv, disulfide-linked Fv, domain deleted, minibody, diabody, or a fusion polypeptide of one of the above fragments with another peptide or polypeptide, Fc-peptide fusion, Fc-toxine fusion, scaffold proteins.

The invention further relates to a method for increasing secretion of a membrane or secreted protein of interest in a cell comprising expressing said protein of interest and expressing a protein having an amino acid sequence comprising a steroidogenic acute regulatory related lipid transfer (START) domain or a derivative or mutant thereof.

The invention further relates to a method of producing a membrane or secreted protein of interest in a cell comprising increasing the expression of a protein having an amino acid sequence comprising a steroidogenic acute regulatory related lipid transfer (START) domain or a derivative or mutant thereof, and effecting the expression of said protein of interest, whereby the order or steps a and b may be reversed.

In a specific embodiment of the present invention the method is further characterized in that step a) is carried out before step b). In a further specific embodiment of the present invention the method is further characterized in that step a) and b) are carried out simultaneously. In another embodiment of the present invention the method is further characterized in that step b) is carried out before step a).
In a preferred embodiment of the present invention the method further comprises an additional step of recovering the protein of interest.
In an especially preferred embodiment of the present invention the method further comprises an additional step of isolating and purifying the protein of interest.

In a specific embodiment of the present invention the method comprises increasing the expression of a protein having an amino acid sequence comprising a steroidogenic acute regulatory related lipid transfer (START) domain or a derivative or mutant thereof by transfecting a cell with a polynucleotide encoding for a protein having an amino acid sequence comprising a steroidogenic acute regulatory related lipid transfer (START) domain or a derivative or mutant thereof.

In a specific embodiment of the present invention the method comprises transfecting said cell with a first polynucleotide encoding for a protein having an amino acid sequence comprising a steroidogenic acute regulatory related lipid transfer (START) domain or a derivative or mutant thereof and transfecting said cell with a second polynucleotide encoding for a protein of interest.

In a specific embodiment of the present invention the START domain protein of the method is characterized by being induced upon ER stress and/or is structurally characterized by having no further structural motifs besides the START domain such as StarD4 (SEQ ID NO. 21), StarD5 (SEQ ID NO: 23), StarD6 (SEQ ID NO. 25) or PCTP (SEQ ID NO: 27).

In a preferred embodiment of the present invention the method comprises increasing the expression of a protein having an amino acid sequence comprising a steroidogenic acute regulatory related lipid transfer (START) domain or a derivative or mutant thereof, preferably by transfecting said cell with a first polynucleotide encoding for a protein having an amino acid sequence comprising a steroidogenic acute regulatory related lipid transfer (START) domain or a derivative or mutant thereof, whereby the increase is measured in comparison to an untransfected cell, transfecting said cell with a second polynucleotide encoding for a protein of interest

In a preferred embodiment of the present invention the method is characterized by that the proteins expressed in step a) and b) are not identical.

The invention further relates to a method of producing a membrane or secreted protein of interest in a cell comprising
Increasing the expression of a protein having an amino acid sequence comprising a steroidogenic acute regulatory related lipid transfer (START) domain or a derivative or mutant thereof in said cell and effecting the expression of said protein of interest in said cell.

The invention furthermore relates to a method of producing a membrane or secreted protein of interest in a cell comprising increasing the expression of a protein having an amino acid sequence comprising a steroidogenic acute regulatory related lipid transfer (START) domain or a derivative or mutant thereof in said cell and expressing said protein of interest in said cell.

In a specific embodiment of the present invention the method is characterized in that said method results in increased specific cellular productivity of said protein of interest in said cell in comparison to a control cell previously transfected with a polynucleotide encoding for the protein of interest, but whereby said control cell does not have increased expression of a protein having an amino acid sequence comprising a steroidogenic acute regulatory related lipid transfer (START) domain or a derivative or mutant thereof.

In a specific embodiment of the present invention the method is characterized in that the protein of interest is a protein which is passing through the Golgi complex.

The invention further relates to a method of increasing specific cellular productivity of a membrane or secreted protein of interest in a cell comprising introducing into a cell one or more vector systems comprising nucleic acid sequences encoding for at least two polypeptides whereby a first polynucleotide encodes a protein having an amino acid sequence comprising a steroidogenic acute regulatory related lipid transfer (START) domain or a derivative or mutant thereof and a second polynucleotide encodes a protein of interest and whereby the protein of interest and the protein having an amino acid sequence comprising a steroidogenic acute regulatory related lipid transfer (START) domain or a derivative or mutant thereof are expressed by said cell.

The invention furthermore relates to a method of increasing the transfection efficiency of a cell expressing a membrane or secreted protein of interest in a cell comprising transfecting said cell with a first polynucleotide encoding a protein having an amino acid sequence comprising a steroidogenic acute regulatory related lipid transfer (START) domain or a derivative or mutant thereof, subsequently transfecting said cell with a second polynucleotide encoding a protein of interest,whereby said first and second polynucleotides are located on different vector systems.

In a further embodiment the invention relates to a method of increasing the transfection efficiency of a cell comprising the additional step of transfecting a reporter gene such as GFP, YFP, HRP, SEAP or LacZ, which might be fused to the protein of interest, located on the same expression construct or on a separate plasmid.

In a preferred embodiment the invention relates to a method of increasing the transfection efficiency of a cell comprising the additional step of detecting and/or measuring the transfection efficiency by either detection of the protein of interest or the expression of the reporter gene.

The invention further relates to an expression vector comprising two polynucleotides, a first polynucleotide encoding for a protein having an amino acid sequence comprising a steroidogenic acute regulatory related lipid transfer (START) domain or a derivative or mutant thereof and a second polynucleotide encoding for a protein of interest.

In a specific embodiment of the present invention the expression vector is characterized in that the START domain protein is a mammalian START domain family member such as PCTP (SEQ ID NO. 27), StarD7, GPBP, StarD10, StarD8, StarD13, DLC-1, StarD4 (SEQ ID NO. 21), StarD6 (SEQ ID NO. 25), StarD5 (SEQ ID NO. 23), MLN64, StAR, THEA-2, CACH or StarD9 or a derivative or mutant thereof.

In another embodiment of the present invention the expression vector is characterized in that the START domain protein is ceramide transfer protein CERT (CERT=SEQ ID NO. 11 or CERT_{L}=SEQ ID NO. 13) or a derivative or mutant thereof.

In a specific embodiment of the present invention the expression vector is characterized in that the mutated CERT is CERT _{S132A} (SEQ ID NO. 15).

In a specific embodiment of the present invention the expression vector is characterized in that said first polynucleotide increases the protein transport in a cell via the secretory pathway.

In a specific embodiment of the present invention the expression vector is characterized in that the START domain protein is mutated ceramide transfer protein CERT and said mutation disables and /or deletes a phosphorylation site at any serine, threonine or tyrosine position within the CERT protein.

In another embodiment of the present invention the expression vector is characterized in that the START domain protein is mutated ceramide transfer protein CERT and said mutation disables and /or deletes the protein kinase D (PKD) phosphorylation site of CERT at position 132.

The present invention further relates to a cell comprising the expression vector of the invention. In a specific embodiment of the present invention the cell is characterized in that said cell is a eukaryotic cell such as a yeast, plant, worm, insect, avian, fish, reptile or mammalian cell. In a specific embodiment of the present invention the cell is characterized in that said eukaryotic cell is a mammalian cell.

In a specific embodiment of the present invention the cell is characterized in that said mammalian cell is a Chinese Hamster Ovary (CHO), monkey kidney CV1, monkey kidney COS, human lens epitheliaim PER.C6TM, human embryonic kidney, HEK 293, baby hamster kidney, African green monkey kidney, human cervical carcinoma, canine kidney, buffalo rat liver, human lung, human liver, mouse mammary tumor or myeloma cell, a dog, pig or macaque cell, rat, rabbit, cat, goat, preferably a CHO cell. In a specific embodiment of the present invention the cell is characterized in that said CHO cell is CHO wild type, CHO K1, CHO DG44, CHO DUKX-B11, CHO Pro-5, preferably CHO DG44.

In a specific embodiment of the present invention the cell is characterized in that said cell is an animal cell, preferably a metazoan cell such as *C. elegans.* In a further embodiment of the present invention the cell is characterized in that said cell is a bilateria cell such as *Drosophila melanogaster,* preferably a vertebrate cell such as an avian, fish, reptile or mammalian cell. In a specific embodiment of the present invention the cell is characterized in that said eukaryotic cell is a mammalian cell, preferably a human cell such as a the human myeloma cellline U266, HEK293, HeLa, HepG2 or HT1080, more preferably a rodent cell such as murine NSO, Sp2/0 or Ag8653 cell, YO or YB2.0.

The invention further relates to a protein of interest, preferably an antibody produced by any of the methods described.

The invention further relates to a pharmaceutical composition comprising a polynucleotide sequence useful for blocking or reducing the expression of a protein having an amino acid sequence comprising a steroidogenic acute regulatory related lipid transfer (START) domain or a derivative or mutant thereof. The invention furthermore relates to a pharmaceutical composition comprising a polynucleotide sequence which blocks or reduces the expression of a protein having an amino acid sequence comprising a START domain or a derivative or mutant thereof.

In a specific embodiment of the present invention the pharmaceutical composition is characterized in that the START domain sequence is ceramide transfer protein CERT (CERT=SEQ ID NO. 11 or CERT_{L}=SEQ ID NO. 13) or a derivative or mutant thereof.
In another specific embodiment of the present invention the pharmaceutical composition is characterized in that the START domain is (SEQ ID NO. 17 or 19) or a derivative or mutant thereof.

In a specific embodiment of the present invention the pharmaceutical composition is characterized in that the polynucleotide sequence is RNAi, siRNA or antisense-RNA.

In a preferred embodiment of the present invention the pharmaceutical composition is characterized in that the START domain protein is a mammalian START domain family member such as PCTP (SEQ ID NO. 27), StarD7, GPBP, StarD10, StarD8, StarD13, DLC-1, StarD4 (SEQ ID NO. 21), StarD6 (SEQ ID NO. 25), StarD5 (SEQ ID NO. 23), MLN64, StAR, THEA-2, CACH or StarD9 or a derivative or mutant thereof.
In a particularly preferred embodiment of the present invention the pharmaceutical composition is characterized in that said polynucleotide is complementary to the CERT nucleotide sequence or parts thereof, especially to the START domain.
In a most preferred embodiment of the present invention the pharmaceutical composition is characterized in that said polynucleotide binds to either the CERT gene or the CERT promoter.
In a further embodiment of the present invention the pharmaceutical composition is characterized in that said polynucleotide is anti-sense oligonucleotide to the CERT gene or parts thereof.

The invention further relates to a pharmaceutical composition comprising an inhibitor or suppressor of a protein having an amino acid sequence comprising a steroidogenic acute regulatory related lipid transfer (START) domain, preferably CERT (SEQ ID NO. 11 or SEQ ID NO. 13) or a derivative or mutant thereof.

In a specific embodiment of the present invention the pharmaceutical composition is characterized in that said inhibitor or suppressor is a chemical substance or a peptid-inhibitor or an inhibiting protein such as. (i) protein binding to CERT promoter thereby inhibiting CERT expression, (ii) protein binding to CERT or PKD thus preventing binding of PKD and CERT and hindering CERT phosphorylation by PKD, (iii) a protein similar to CERT which however does not fulfill CERT functions, that means a "dominant-negative" CERT variant, or (iv) a protein acting as scaffold for both CERT and PKD, resulting in irreversible binding of CERT to PKD (= a stable PKD/CERT complex) which is not functional due to the inhibitory phosphorylation of CERT by PKD and the hindering of dissociation of CERT from said complex.

In a specific embodiment of the present invention the pharmaceutical composition is characterized in that said inhibitor or suppressor is a inhibitor or suppressor of CERT activity.

The invention further relates to a method for identifying a modulator of START domain protein function, preferably CERT function, comprising providing a protein having an amino acid sequence comprising a steroidogenic acute regulatory related lipid transfer (START) domain or a derivative or mutant thereof, preferably CERT, contacting said protein of step a) with a test agent, determining an effect related to increased or decreased protein secretion or expression of cell-surface proteins.

The invention further relates to a method comprising application of a pharmaceutical composition as described for the treatment of cancer.

The invention furthermore relates to a use of a START domain protein or a polynucleotide encoding for a START domain protein to increase secretion and /or production of a protein of interest.

The invention further relates to a diagnostic use of any of the methods, expression vectors, cells or pharmaceutical compositions as described.

The invention generally described above will be more readily understood by reference to the following examples, which are hereby included merely for the purpose of illustration of certain embodiments of the present invention. The following examples are not limiting. They merely show possible embodiments of the invention. A person skilled in the art could easily adjust the conditions to apply it to other embodiments.

### EXPERIMENTAL

### MATERIALS AND METHODS

### Antibodies and reagents

Antibodies are: rabbit anti-PKD substrate polyclonal antibody (Cell Signaling), mouse anti-Flag monoclonal antibody (Sigma-Aldrich), mouse anti-GFP monoclonal antibody (Roche), rabbit anti-PKD polyclonal antibody (C-20, Santa Cruz Biotechnology), mouse anti-GS28 (BD Biosciences) and mouse anti-tubulin (Neomarkers). The phosphospecific anti-pS916 PKD antibody monitoring PKD autophosphorylation is described elsewhere (Hausser et al., 2002). Peroxidase-labeled secondary anti-mouse and anti-rabbit IgG antibodies are from Amersham; alkaline phosphatase-labeled secondary anti-mouse IgG antibody is from Sigma; Alexa Fluor 488- and 546-labeled secondary anti-mouse and anti-rat IgG antibodies are from Molecular Probes.

### DNA constructs

Full-length CERT cDNA is amplified by PCR using pcDNA3-Flag-CERT as a template with primers containing EcoRI restriction sites and cloned into the pEGFPC1 vector. The point mutants of CERT are generated by Quikchange site-directed PCR mutagenesis following the manufacturer's instructions (Stratagene). Truncated CERT variants are generated by insertion of STOP codons. The following oligonucleotides are used: CERT-S132A (SEQ ID NO.1: 5'-cgtcgacatggcgcaatggtgtccctgg-3'), CERT-S132A rev (SEQ ID NO.2: 5'-ccagggacaccattgcgccatgtcgacg-3'), CERT-S272A (SEQ ID NO.3: 5'-ggttaaacgtgaggacgcctggcagaagagactgg-3'); CERT-S272Arev (SEQ ID NO.4: 5'-ccagtctcttctgccaggcgtcctcacgtttaacc-3'), CERT truncations at amino acid 138 (SEQ ID NO.5: 5'-ggtgtccctggtgtcttgagcaagtggctactc-3'); CERT-138 truncation rev (SEQ ID NO.6: 5'-gagtagccacttgctcaagacaccagggacacc-3'). The Flag-CERT cDNA is subcloned into pGEX6P1 using EcoRI restriction sites. pEGFP-N1-PKD and pEGFP-N1-PKD_{K612W} are described previously (Hausser et al., 2005). The plasmid encoding ss-HRP-Flag is kindly provided by Vivek Malhotra (UCSD).

### Cell culture and transfection

HEK293T and COS7 cells grow in RPMI supplemented with 10% fetal calf serum (FCS) in a humified atmosphere containing 5% CO₂. HEK293T cells are transfected using TransIT293 reagent (Mirus) according to the manufacturer's instructions. For immunofluorescence, COS7 cells are grown on glass coverslips for 24 hours and transfected with Lipofectamine 2000 reagent (Invitrogen).

### Immunofluorescence microscopy

Cells are washed with PBS containing magnesium and calcium, fixed in 4% paraformaldehyde at room temperature for 10 min, washed and incubated with PBS containing 0.1 M glycine for 15 min. Cells are then permeabilized with PBS containing 0.1% Triton for 5 min and then blocked with 5% goat serum in PBS containing 0.1% Tween-20 for 30 min. Cells are incubated with primary antibody diluted in blocking buffer for 2 hours, followed by incubation with secondary antibodies diluted in blocking buffer for 1 hour. Coverslips are mounted in Fluoromount G (Southern Biotechnology) and cells are analyzed on a confocal laser scanning microscope (TCS SL, Leica) using 488 and 543 nm excitation and a 40.0/1.25 HCX PL APO objective lens. Images are processed with Adobe Photoshop.

### Protein extraction, immunoprecipitation and Western blotting

Whole cell extracts are obtained by solubilizing cells in NP40 extraction buffer (NEB) [50 mM Tris (pH 7.5), 150 mM NaCl, 1% NP40, 1 mM sodium orthovanadate, 10 mM sodium fluoride, and 20 mM β-glycerophosphate plus Complete protease inhibitors]. Lysates are clarified by centrifugation at 16,000 x g for 10 min. For immunoprecipitations, equal amounts of protein are incubated with specific antibodies for 2 h on ice. Immune complexes are collected with protein G-Sepharose (GE Healthcare) and washed three times with NEB (see above). Whole cell extracts or immunoprecipitated proteins are boiled in sample buffer and subjected to SDS-PAGE. The proteins are blotted onto polyvinylidine difluoride membranes (Roth). After blocking with 0.5% blocking reagent (Roche) in PBS containing 0.1% Tween 20, filters are probed with specific antibodies. Proteins are visualized with peroxidase-coupled secondary antibody using the enhanced chemiluminescence detection system (Pierce). Stripping of membranes is performed in SDS buffer [62.5 mM Tris (pH 6.8), 2% SDS, and 100 mM β-mercaptoethanol] for 30 min at 60°C. Membranes are then reprobed with the indicated antibodies.

### Recombinant protein purification and in vitro kinase assays

BL21 bacteria are transformed with pGEX6P-Flag-CERT(1-138) and CERT-S132A(1-138) vectors. Expression is induced with 0.5 mM isopropyl-β-D-1-thiogalactopyranoside for 4 hrs at 30°C. Bacteria are harvested and resuspended in PBS containing 50 µg/ml lysozyme, Complete protease inhibitors (Roche), 10 mM sodium fluoride and 20 mM - glycerophosphate. Triton X-100 is added to a final concentration of 1% prior to sonication. GST-CERT fusions are purified from clarified lysate with glutathione resin (GE Healthcare). The purity of protein preparations is verified by SDS-PAGE and Coomassie staining. Recombinant proteins are incubated with purified PKD1 in kinase buffer [50 mM Tris, pH 7.5, 10 mM MgCl2 and 1 mM DTT] in the presence of either 2 µCi [γ-³²P]-ATP or 75 µM cold ATP for 30 min. Samples are resolved by SDS-PAGE, blotted onto membrane and analyzed on a Phospholmager (Molecular Dynamics) or by Western blotting with anti-PKD substrate antibody.

### PIP arrays

HEK293T cells transiently expressing GFP-tagged CERT variants are harvested in hypotonic buffer [50 mM Tris, pH 7.4, containing Complete protease inhibitors (Roche), 1 mM PMSF, 5 mM β-glycerophosphate and 5 mM sodium fluoride] and sheared by passage through a 25G/16 mm gauge needle. The cytosol fraction is obtained after 100.000xg centrifugation for 1 h and the amount of expressed protein is quantified by measuring GFP peak emission at 480 - 550 nm (excitation 466 nm). PIP arrays (Echelon) are blocked in TBS-T [10 mM Tris, pH 8, 150 mM NaCl, 0.1 % Tween-20] containing 3 % fatty acid-free BSA (Roth), followed by incubation with 500 µg cytosol containing equal amounts of GFP proteins (adjusted with cytosol from untransfected cells) in 5 ml blocking buffer for 1 h at room temperature. Bound proteins are detected by incubation with anti-GFP antibody, followed by HRP-conjugated secondary antibody.

### In vitro ceramide transfer assay

Protein-mediated transfer of ceramide between SUVs is measured as described previously (Olayioye et al., 2005). The transfer assay mixture contained donor vesicles (2 nmol lipid/ml) composed of porcine brain lipids (Avanti Polar Lipids), pyrene-labeled C₁₆-ceramide, and 2,4,6-trinitrophenyl-phosphatidylethanolamine (TNP-PE) (88.6:0.4:11 mol %), provided by P. Somerharju, and a 10-fold excess of acceptor vesicles composed of porcine brain lipids. Fluorescence intensity is recorded at 395 nm (excitation, 345 nm; slit widths, 4 nm) before and after the addition of 75 µg cytosol from HEK293T cells transiently expressing the GFP-tagged CERT wild type and S132A proteins (see above). Fluorescence intensities are normalized to (i) the maximum intensity obtained after the addition of Triton X-100 (0.5 % final concentration) and (ii) the maximum GFP fluorescence, to account for different protein expression levels.

### HRP transport assay

HEK293T cells are cotransfected with ss-HRP-Flag plasmid and empty vector, pEGFP-N1-PKD1KD, pcDNA3-Flag-CERT wt and pcDNA3-Flag-CERT-S132A at a ratio of 1: 6.5, respectively. 24h post-transfection cells are washed with serum-free media and HRP secretion is quantified after 0, 1, 3 and 6 h by incubation of clarified cell supernatant with ECL reagent. Measurements are done with a luminometer (Lucy2, Anthos) at 450 nm.

### siRNA assay

COS7 cells are transfected with a vector encoding ssHRP-Flag, harvested after 8 hrs, replated into triplicate wells and then transfected with CERT-specific siRNA oligonucleotides (siCERT#1, SEQ ID NO.7: 5'-ccacaugacuuacucauuatt-3'; siCERT#2, SEQ ID NO.8: 5'-gaacagaggaagcauauaatt-3') using Oligofectamine™ reagent (Invitrogen) according to the manufacturers instructions. Control cells are either mock transfected or transfected with a lacZ-specific siRNA (SEQ ID NO.9: 5'-gcggcugccggaauuuacctt-3'). 48 h later, cells are washed and fresh medium is added. The amount of HRP secreted into the supernatant is measured by a chemiluminescent assay as described above. Finally, cells are lysed, triplicate lysates are pooled and analyzed by immunoblotting using tubulin- und transferrin receptor-specific antibodies.

### EXAMPLES

### EXAMPLE 1: INTRACELLULAR PRODUCT ACCUMULATION INDICATES SECRETORY BOTTLE NECK.

A fed-batch process is performed using three different CHO producer cell clones expressing human IgG antibody (Process A, B and M, respectively, see FIGURE 1). Cell samples are taken every other day and the amount of intracellular antibody is determined by FACS analysis. In short, cells are fixed using PBS / 4% PFA, permeabilized and stained with FITC-conjugated anti-human kappa light chain antibody. Within the first four days, the intracellular IgG content remains at a constant level. However from day 5 to day 9, the level of intracellular product rises constantly, indicating an accumulation of either misfolded light chain or even the complete antibody product within the cell. These data represent the results of three independent production processes with different producer cell clones and products and they strongly suggest that the cell transcribes more antibody RNA than proteins secreted into the medium and thus points to a post-translational bottle neck which hinders the complete secretion of the produced antibody (FIGURE 1).

### EXAMPLE 2: CERT IS DETECTED BY A PKD SUBSTRATE ANTIBODY.

PKD is a key regulator at the Golgi complex with PI4KIIIβ being the only local substrate identified thus far. To test whether the Golgi complex-localized CERT protein (SEQ ID NO.11 and 13) may serve as a substrate for PKD, we make use of a phosphospecific substrate antibody, termed pMOTIF, raised against consensus motifs phosphorylated by PKD (Doppler et al., 2005). HEK293T cells are transfected with expression vectors encoding Flag-tagged CERT (SEQ ID NO.10) and CERT_{L}(SEQ ID No.12). The CERT isoforms are immunoprecipitated with Flag-specific antibodies and analyzed by Western blotting with the pMOTIF antibody (FIGURE 4A). A pMOTIF signal corresponding to the molecular weight of CERT (SEQ ID NO.11) and, more weakly, to that of CERT_{L}.(SEQ ID No.13) is detected. The weaker detection of the phosphorylated CERT_{L} isoform may be related to its known behaviour to form aggregates, which may impact phosphosite accessibility to kinases (Raya et al., 2000).
To investigate whether recognition of CERT by the pMOTIF antibody is dependent upon PKD, we express CERT together with a kinase dead variant of PKD1 (K621W) in HEK293T cells. This mutant has been shown to localize to the Golgi complex and suppressed PI4KIIIβ phosphorylation in a dominant negative fashion (Hausser et al., 2005). Coexpression of inactive PKD abolishes detection of CERT with the pMOTIF antibody, suggesting that the pMOTIF signal is indeed due to PKD-mediated CERT phosphorylation (FIGURE 4B).
Lipid transfer proteins are thought to act at membrane contact sited, which are formed between the ER and TGN (Levine and Loewen, 2006), where PKD is localized. Immunofluorescence staining of Flag-tagged CERT in COS7 cells coexpressed with GFP-tagged PKD1 verify that the two proteins colocalize at the Golgi complex (FIGURE 4C). Together, these data confirm that CERT is a PKD substrate at the Golgi apparatus.

### EXAMPLE 3: PKD PHOSPHORYLATES CERT ON SERINE 132.

To identify pMOTIF recognition sites in CERT, we search for potential PKD consensus motifs characterized by a leucine, isoleucine or valine residue in the -5 and arginine in the - 3 position relative to a serine or threonine. Two serines at positions 132 and 272, matching the PKD consensus motif and conserved across species (FIGURE 5A), are exchanged for alanines by site-directed mutagenesis. These mutants are expressed in HEK293T cells and tested for recognition by the pMOTIF antibody. Interestingly, mutation of serine 132 to alanine abrogate detection of CERT with the pMOTIF antibody and cause an increase in electrophoretic mobility, indicative of loss of phosphorylation, while the S272A mutation does not affect the pMOTIF signal (FIGURE 5B). This suggests that serine 132 is a PKD phosphorylation site specifically recognized by the PKD substrate antibody. To confirm that PKD is capable of directly phosphorylating this serine residue in CERT, we perform *in vitro* kinase assays with purified PKD1 and recombinant CERT GST-fusion proteins produced in *E. coli* comprising the first 138 amino acids of the protein. When the truncated wild type CERT fusion protein is incubated with PKD1 in the presence of [γ-³²P]-ATP, incorporation of radioactivity is detected (FIGURE 5C). This is significantly impaired in the case of the CERT-S 132A fusion protein. *In vitro* PKD phosphorylation of wild type but not CERT-S 132A (SEQ ID NO.15) is further shown to generate a recognition site for the pMOTIF antibody (FIGURE 5D). Taken together, these results prove that CERT is a genuine PKD substrate *in vitro* and *in vivo* and identify serine 132 as a specific PKD phosphorylation site in CERT.

### EXAPMLE 4: CERT PHOSPHORYLATION ON SERINE 132 MODULATES PI(4)P BINDING AND CERAMIDE TRANSFER ACTIVITY.

Serine 132 is in very close proximity to the CERT PH domain (amino acids 23 - 117), making it possible that phosphorylation on this site affects PI(4)P binding by increasing the local negative charge. We therefore quantify PI(4)P binding of wild type CERT and the CERT-S 132A mutant (SEQ ID NO.15) by performing protein-lipid overlay assays. Here, cytosol from HEK293T cells transiently expressing the CERT variants is incubated with membranes spotted with a concentration gradient of the different phosphoinositides and bound CERT proteins are detected via their GFP tag. As reported previously, the full-length wild type protein demonstrate weak binding to several phospholipid species, but displays strong interaction with PI(4)P (Hanada et al., 2003; Levine and Munro, 2002). CERT-S132A binding to PI(4)P is detectable at two- to fourfold lower concentrations as compared to that of the wild type protein, suggesting increased affinity of the CERT-S132A mutant to this phospholipid (FIGURE 6A).
Together, these data imply that CERT, once bound to the Golgi complex, is phosphorylated by PKD. This then decreases the affinity of CERT to PI(4)P and thereby regulates the interaction of CERT with Golgi membranes.

As CERT has been shown to function as a lipid transfer protein (Hanada et al., 2003). We investigate whether CERT phosphorylation on serine 132 influenced its ability to bind and transfer ceramide between membranes. To this end, GFP-tagged versions of wild type CERT (SEQ ID NO.10) and CERT-S132A (SEQ ID NO.14) are transiently expressed in HEK239T cells and the cytosol fraction is analyzed for ceramide-specific lipid transfer activity using a FRET-based assay (FIGURE 6B). In this assay, small unilamellar vesicles containing pyrene-labeled ceramide as a fluorescent donor and quenching amounts of head group-labeled TNP-PE are employed (Olayioye et al., 2005; Somerharju, 2002). When these donor liposomes are mixed with an excess of unlabeled acceptor liposomes, the increase in pyrene fluorescence is negligible, indicating minimal spontaneous ceramide transfer to acceptor membranes (data not shown).
Upon addition of wild type CERT-containing cytosol, a steady increase in fluorescence is noted, which is not observed when control cytosol of vector-transfected cells is used (FIGURE 6B). Compared to the wild type protein, CERT-S132A (SEQ ID No.15) displays a higher rate of lipid transfer, evident from a more rapid increase in pyrene fluorescence. This suggests that CERT phosphorylation on serine 132 downregulates ceramide transfer activity by decreasing association of the protein with membranes.

Previous data have already shown that PKD regulates the level of PI(4)P at the Golgi complex by phosphorylation-mediated activation of PI4KIIIβ (Hausser et al., 2005). Interestingly, PI4KIIIβ is critical for the transport of ceramide between the ER and the Golgi complex (Toth et al., 2006). Accordingly, together with the data presented here, a dual role for PKD in maintaining lipid homeostasis of Golgi membranes becomes apparent by controlling the on-rate (via PI(4)P levels) and the off-rate (via direct phosphorylation) of CERT.

### EXAMPLE 5: CERT REGULATES PKD ACTIVATION AND SECRETORY TRANSPORT.

We hypothesize that overexpression of CERT by transferring ceramide should result in elevated DAG levels and might consequently stimulate PKD activity. To test this, Flag-tagged CERT wild type (SEQ ID NO.10) and CERT-S132A (SEQ ID NO.14) are transiently expressed in HEK293T cells. Whole cells lysates are prepared 24 h post transfection and subjected to SDSPAGE. PKD activation is analyzed by immunoblotting with phosphospecific pS916 PKD antibody (FIGURE 7A, top panel). Equal loading is verified by reprobing with PKD-specific antibody (FIGURE 7A middle panel). Expression of CERT proteins is verified by immunoblotting with Flag-specific antibody (FIGURE 7A bottom panel). Compared to the control, expression of both CERT wild type and CERT-S132A increased PKD activity, as revealed by analyses with a phosphospecific PKD antibody. This shows that PKD activation is regulated by CERT proteins, likely due to increased ceramide delivery and enforced SM/DAG synthesis.
To address the question of whether CERT-mediated PKD activation indeed translates into enhanced secretory transport, we make use of a plasmid encoding secreted horseradish peroxidase (HRP-ss) which can be used as reporter for constitutive protein secretion. HEK293T cells are cotransfected with an expression plasmid encoding Flag-ss-HRP or empty vector, and PKD1-GFP kinase dead (KD), Flag-CERT wild type (WT), and Flag-CERT-S132A, respectively. 24 h post-transfection, cells are washed and fresh medium is added. The supernatant is analyzed for peroxidase activity after 0, 1, 3, and 6 h by chemiluminescence. In control cells, secretion of ss-HRP could be detected within 1 hour and increased over time (FIGURE 7B). Coexpression of kinase dead PKD1, which inhibits secretory transport of cargo protein almost entirely abrogates the secretion of ss-HRP into the supernatant. This confirms that HRP is secreted in a PKD-dependent manner in this assay. In Contrast, coexpression of CERT wild type and CERT-S132A strongly augmented the amount of secreted HRP (FIGURE 7B), the mutant showing even slightly higher values than wild type CERT. This experiment demonstrates that CERT overexpression stimulates PKD phosphorylation and in a functional assay enhances secretion of an extracellular protein into the culture medium by around 2-fold.

We furthermore investigates whether overexpression of the CERT-S132A mutant affected its localization and/or caused morphological changes of the Golgi apparatus. CERT has been demonstrated to colocalize with the cis/medial-Golgi marker GS28 (Hanada et al., 2003).
Immunofluorescence analysis of GFP-tagged CERT expressed in COS7 cells shows that the protein localized to GS28-positive Golgi regions (FIGURE 7C). By contrast, in addition to the partial colocalization with GS28 at the Golgi complex, the CERT-S132A mutant protein displays a dispersed, punctate staining. Of note, some of these vesicular structures are found to contain the cargo protein ss-HRP, providing evidence that these structures indeed represent Golgi-derived transport carriers (FIGURE 7D). This finding is in accordance with the observed changes in Golgi membrane structure due to local increases in ceramide levels (Fukunaga et al., 2000; Weigert et al., 1999).

### EXAMPLE 6: CERT DOWNREGULATION BY RNA INTERFERENCE INHIBITS SECRETORY TRANSPORT.

The data presented so far in the present invention clearly demonstrated that overexpression of CERT enhances protein secretion. To investigate whether also the opposite is true, meaning that reduced CERT expression would result in diminished secretion, siRNA experiments are performed. COS7 cells are transfected with a vector encoding ssHRP-Flag, harvested after 8 hrs, replated into triplicate wells and then transfected with CERT-specific siRNA oligonucleotides (SEQ ID NO.7 and 8) or either mock or lacZ-specific siRNA (SEQ ID NO.9) as controls. 48 h later, cells are washed, covered with fresh medium and the amount of HRP secreted into the supernatant is measured after the indicated times.

As shown in FIGURE 8A, activity of HRP is detected after 3 hours and showed equal comparable levels in both control cells. In contrast, a dramatic reduction of HRP activity is measured in cells that had been treated with any of the CERT-specific siRNA oligonucleotides. This indicates that reduced CERT levels lead to reduced HRP secretion from the cells and further underscores the important role of CERT in the secretory transport.

Interestingly, not only protein secretion, but also the abundance of the transmembrane protein transferrin receptor is affected by the reduction of CERT (FIGURE 8B). When the cells from FIGURE 8A are pooled and the lysates probed with transferrin receptor-specific antibodies in Western blot experiments, a strong decrease in the amount of transferrin receptor became apparent, whereas similar transferrin receptor levels are detected in both control cells.

This finding suggests, that the lipid transfer protein CERT is not only implicated in the transport of secreted but also of membrane-standing cell-surface proteins. This might not be surprising as both types of proteins are equally transported in lipid vesicles from the ER via the Golgi to the plasma membrane and thus use the same cellular export routes which- as we demonstrate in the present invention for the first time - are influenced by CERT.

### EXAMPLE 7: OVEREXPRESSION OF CERT INCREASES BIOPHARMACEUTICAL PROTEIN PRODUCTION OF AN ANTIBODY.

(a) An antibody producing CHO cell line (CHO DG44) secreting humanised anti-CD44v6 IgG antibody BIWA 4 is transfected with an empty vector (MOCK control) or expression constructs encoding wild type CERT (SEQ ID NO.10 and 12) or a mutant of CERT bearing the point-mutation Ser132A (SEQ ID NO.14) and subsequently subjected to selection to obtain stable cell pools. During six subsequent passages, supernatant is taken from seed-stock cultures of all stable cell pools, the IgG titer is determined by ELISA and divided by the mean number of cells to calculate the specific productivity. The highest values are seen in the cell pools harbouring the CERT mutant (SEQ ID No.14), followed by wild type CERT (SEQ ID No.10 or 12). In both, IgG expression is markedly enhanced compared to MOCK or untransfected cells. Very similar results can be obtained if the stable transfectants are subjected to batch or fed-batch fermentations. In each of these settings, overexpression of both wild type and mutant CERT leads to increased antibody secretion, indicating that CERT is able to enhance the specific production capacity of the cells grown in serial cultures or in bioreactor batch or fed batch cultures.
b) CHO host cells (CHO DG44) are first transfected with vectors encoding wild type CERT (SEQ ID NO.10 or 12) or a mutant of CERT bearing the point-mutation Ser132A (SEQ ID NO.14). Cells are subjected to selection pressure and cell lines are picked that demonstrate heterologous expression of CERT or the CERT mutant. Subsequently these cell lines and in parallel CHO DG 44 wild type cells are transfected with vectors encoding humanized anti-CD44v6 IgG antibody BIWA 4 as the gene of interest. After a second round of selection, supernatant is taken from seed-stock cultures of all stable cell pools over a period of six subsequent passages, the IgG titer is determined by ELISA and divided by the mean number of cells to calculate the specific productivity. The highest values are seen in the cell pools harbouring the CERT mutant (SEQ ID No.14), followed by wild type CERT (SEQ ID NO.10 or 12). In both, IgG expression is markedly enhanced compared to cells that don't have heterologous expression of CERT or CERT mutant. Very similar results can be obtained if the stable transfectants are subjected to batch or fed-batch fermentations. In each of these settings, overexpression of both wild type and mutant CERT leads to increased antibody secretion, indicating that CERT is able to enhance the specific production capacity of the cells grown in serial cultures or in bioreactor batch or fed batch cultures.
This indicates, that heterologous expression of CERT, and especially mutant CERT, can enhance antibody secretion in transiently as well as stably transfected CHO cell lines .

### EXAMPLE 8: OVEREXPRESSION OF CERT INCREASES BIOPHARMACEUTICAL PROTEIN PRODUCTION OF MONOCYTE CHEMOATTRACTANT PROTEIN 1 (MCP-1).

(a) A CHO cell line (CHO DG44) secreting monocyte chemoattractant protein 1 (MCP-1) is transfected with an empty vector (MOCK control) or expression constructs encoding wild type CERT (SEQ ID NO.10 and 12) or a mutant of CERT bearing the point-mutation Ser132A (SEQ ID NO.14) and subsequently subjected to selection to obtain stable cell pools. During six subsequent passages, supernatant is taken from seed-stock cultures of all stable cell pools, the MCP-1 titer is determined by ELISA and divided by the mean number of cells to calculate the specific productivity. The highest values are seen in the cell pools harbouring the CERT mutant, followed by wild type CERT. In both, IgG expression is markedly enhanced compared to MOCK or untransfected cells. Very similar results can be obtained if the stable transfectants are subjected to batch or fed-batch fermentations. In each of these settings, overexpression of both wild type and mutant CERT leads to increased MCP-1 secretion, indicating that CERT is able to enhance the specific production capacity of the cells grown in serial cultures or in bioreactor batch or fed batch cultures.
b) CHO host cells (CHO DG44) are first transfected with vectors encoding wild type CERT (SEQ ID NO.10 or 12) or a mutant of CERT bearing the point-mutation Ser132A (SEQ ID NO.14). Cells are subjected to selection pressure and cell lines are picked that demonstrate heterologous expression of CERT or the CERT mutant. Subsequently these cell lines and in parallel CHO DG 44 wild type cells are transfected with a vector encoding monocyte chemoattractant protein 1 (MCP-1) as the gene of interest. After a second round of selection, supernatant is taken from seed-stock cultures of all stable cell pools over a period of six subsequent passages, the MCP-1 titer is determined by ELISA and divided by the mean number of cells to calculate the specific productivity. The highest values are seen in the cell pools harbouring the CERT mutant, followed by wild type CERT. In both, MCP-1 expression is markedly enhanced compared to cells that don't have heterologous expression fo CERT or CERT mutant. Very similar results can be obtained if the stable transfectants are subjected to batch or fed-batch fermentations. In each of these settings, overexpression of both wild type and mutant CERT leads to increased antibody secretion, indicating that CERT is able to enhance the specific production capacity of the cells grown in serial cultures or in bioreactor batch or fed batch cultures.
This indicates, that heterologous expression of CERT, and especially mutant CERT, can enhance the secretion of single cell proteins in transiently as well as stably transfected CHO cell lines.

### EXAMPLE 9: OVEREXPRESSION OF CERT INCREASES BIOPHARMACEUTICAL PROTEIN PRODUCTION OF TRANSMEMBRANE PROTEIN EPITHELIAL GROWTH FACTOR RECEPTOR (EGFR).

(a) A CHO cell line (CHO DG44 expressing transmembrane protein epithelial growth factor receptor (EGFR) is transfected with an empty vector (MOCK control) or expression constructs encoding wild type CERT (SEQ ID NO.10 and 12) or a mutant of CERT bearing the point-mutation Ser132A (SEQ ID NO.14) and subsequently subjected to selection to obtain stable cell pools. During six subsequent passages, cells are taken from seed-stock cultures of all stable cell pools and the expression level of EGFR is determined by FACS or Western blotting. The highest values are seen in the cell pools harbouring the CERT mutant, followed by wild type CERT. In both, EGFR expression is markedly enhanced compared to MOCK or untransfected cells. Very similar results can be obtained if the stable transfectants are subjected to batch or fed-batch fermentations. In each of these settings, overexpression of both wild type and mutant CERT leads to increased EGFR expression, indicating that CERT is able to enhance the specific production capacity of the cells grown in serial cultures or in bioreactor batch or fed batch cultures.
b) CHO host cells (CHO DG44) are first transfected with vectors encoding wild type CERT (SEQ ID NO.10 or 12) or a mutant of CERT bearing the point-mutation Ser132A (SEQ ID NO.14). Cells are subjected to selection pressure and cell lines are picked that demonstrate heterologous expression of CERT or the CERT mutant. Subsequently these cell lines and in parallel CHO DG 44 wild type cells are transfected with a vector encoding EGFR as the gene of interest. After a second round of selection, cells are taken from seed-stock cultures of all stable cell pools for six consecutive passages and the expression level of EGFR is determined by FACS or Western blotting. The highest values are seen in the cell pools harbouring the CERT mutant, followed by wild type CERT. In both, EGFR expression is markedly enhanced compared to cells that don't have heterologous expression fo CERT or CERT mutant. Very similar results can be obtained if the stable transfectants are subjected to batch or fed-batch fermentations. In each of these settings, overexpression of both wild type and mutant CERT leads to increased EGFR ecpression, indicating that CERT is able to enhance the specific production capacity of the cells grown in serial cultures or in bioreactor batch or fed batch cultures.
This indicates, that heterologous expression of CERT, and especially mutant CERT, can enhance expression of surface receptors in transiently as well as stably transfected CHO cell lines .

### EXAMPLE 10: OVEREXPRESSION OF STARD4 INCREASES BIOPHARMACEUTICAL PROTEIN PRODUCTION OF AN ANTIBODY.

(a) An antibody producing CHO cell line (CHO DG44) secreting humanised anti-CD44v6 IgG antibody BIWA 4 is transfected with an empty vector (MOCK control) or expression constructs encoding StarD4 (SEQ ID NO.20) and subsequently subjected to selection to obtain stable cell pools. During six subsequent passages, supernatant is taken from seed-stock cultures of all stable cell pools, the IgG titer is determined by ELISA and divided by the mean number of cells to calculate the specific productivity. The highest values are seen in the cell pools harbouring StarD4. IgG expression is markedly enhanced compared to MOCK or untransfected cells. Very similar results can be obtained if the stable transfectants are subjected to batch or fed-batch fermentations. In each of these settings, overexpression of StarD4 is able to enhance the specific production capacity of the cells grown in serial cultures or in bioreactor batch or fed batch cultures.
b) CHO host cells (CHO DG44) are first transfected with vectors encoding StarD4. Cells are subjected to selection pressure and cell lines are picked that demonstrate heterologous expression of StarD4. Subsequently these cell lines and in parallel CHO DG 44 wild type cells are transfected with vectors encoding humanized anti-CD44v6 IgG antibody BIWA 4 as the gene of interest. After a second round of selection, supernatant is taken from seed-stock cultures of all stable cell pools over a period of six subsequent passages, the IgG titer is determined by ELISA and divided by the mean number of cells to calculate the specific productivity. The highest values are seen in the cell pools harbouring StarD4. IgG expression is markedly enhanced compared to cells that don't have heterologous expression of StarD4. Very similar results can be obtained if the stable transfectants are subjected to batch or fed-batch fermentations. In each of these settings, overexpression of StarD4 is able to enhance the specific production capacity of the cells grown in serial cultures or in bioreactor batch or fed batch cultures.
This indicates, that heterologous expression of StarD4, can enhance antibody secretion in transiently as well as stably transfected CHO cell lines .

### References

1. al-Rubeai,M. and Singh,R.P. (1998). Apoptosis in cell culture. Curr. Opin. Biotechnol. 9, 152-156.
2. Alpy,F. and Tomasetto,C. (2005). Give lipids a START: the StAR-related lipid transfer (START) domain in mammals. J. Cell Sci. 118, 2791-2801.
3. Altschul,S.F., Gish,W., Miller,W., Myers,E.W., and Lipman,D.J. (1990). Basic local alignment search tool. J. Mol. Biol. 215, 403-410.
4. Ausubel,F.M., Brent,R., Kingston,R.E., Moore,D.D., Seidman,J.G., Smith,J.A., and Struhl,K. (2002). Current Protocols in Molecular Biology. John Wiley & Sons, Inc.).
5. az Anel,A.M. and Malhotra,V. (2005). PKCeta is required for betalgamma2/beta3gamma2- and PKD-mediated transport to the cell surface and the organization of the Golgi apparatus. J. Cell Biol. 169, 83-91.
6. Bard,F., Casano,L., Mallabiabarrena,A., Wallace,E., Saito,K., Kitayama,H., Guizzunti,G., Hu,Y., Wendler,F., Dasgupta,R., Perrimon,N., and Malhotra,V. (2006). Functional genomics reveals genes involved in protein secretion and Golgi organization. Nature 439, 604-607.
7. Barnes,L.M., Bentley,C.M., Moy,N., and Dickson,A.J. (2007). Molecular analysis of successful cell line selection in transfected GS-NS0 myeloma cells. Biotechnol. Bioeng. 96, 337-348.
8. Barnes,L.M. and Dickson,A.J. (2006). Mammalian cell factories for efficient and stable protein expression. Curr. Opin. Biotechnol. 17, 381-386.
9. Baron,C.L. and Malhotra,V. (2002). Role of diacylglycerol in PKD recruitment to the TGN and protein transport to the plasma membrane. Science 295, 325-328.
10. Blobel,C.P. (2005). ADAMs: key components in EGFR signalling and development. Nat Rev Mol Cell Biol 6, 32-43.
11. Borth,N., Mattanovich,D., Kunert,R., and Katinger,H. (2005). Effect of increased expression of protein disulfide isomerase and heavy chain binding protein on antibody secretion in a recombinant CHO cell line. Biotechnol. Prog. 21, 106-111.
12. Brewer,J.W. and Hendershot,L.M. (2005). Building an antibody factory: a job for the unfolded protein response. Nat Immunol 6, 23-29.
13. Chen-Kiang,S. (2003). Cell-cycle control of plasma cell differentiation and tumorigenesis. Immunol. Rev. 194, 39-47.
14. Chiang,G.G. and Sisk,W.P. (2005). Bcl-x(L) mediates increased production of humanized monoclonal antibodies in Chinese hamster ovary cells. Biotechnol. Bioeng. 91, 779-792.
15. Davis,R., Schooley,K., Rasmussen,B., Thomas,J., and Reddy,P. (2000). Effect of PDI overexpression on recombinant protein secretion in CHO cells. Biotechnol. Prog. 16, 736-743.
16. Doppler,H., Storz,P., Li,J., Comb,M.J., and Toker,A. (2005). A phosphorylation state-specific antibody recognizes Hsp27, a novel substrate of protein kinase D. J. Biol. Chem. 280, 15013-15019.
17. Dorner,A.J. and Kaufman,R.J. (1994). The levels of endoplasmic reticulum proteins and ATP affect folding and secretion of selective proteins. Biologicals 22, 103-112.
18. Egeblad,M. and Werb,Z. (2002). New functions for the matrix metalloproteinases in cancer progression. Nat Rev Cancer 2, 161-174.
19. Fukunaga,T., Nagahama,M., Hatsuzawa,K., Tani,K., Yamamoto,A., and Tagaya,M. (2000). Implication of sphingolipid metabolism in the stability of the Golgi apparatus. J. Cell Sci. 113 (Pt 18), 3299-3307.
20. Hanada,K. (2006). Discovery of the molecular machinery CERT for endoplasmic reticulum-to-Golgi trafficking of ceramide. Mol. Cell Biochem. 286, 23-31.
21. Hanada,K., Kumagai,K., Yasuda,S., Miura,Y., Kawano,M., Fukasawa,M., and Nishijima,M. (2003). Molecular machinery for non-vesicular trafficking of ceramide. Nature 426, 803-809.
22. Hanahan,D. and Weinberg,R.A. (2000). The hallmarks of cancer. Cell 100, 57-70.
23. Harris and Angal (1995). Protein Purification Methods. IRL Press).
24. Hausser,A., Link,G., Bamberg,L., Burzlaff,A., Lutz,S., Pfizenmaier,K., and Johannes,F.J. (2002). Structural requirements for localization and activation of protein kinase C mu (PKC mu) at the Golgi compartment. J. Cell Biol 156, 65-74.
25. Hausser,A., Storz,P., Martens,S., Link,G., Toker,A., and Pfizenmaier,K. (2005). Protein kinase D regulates vesicular transport by phosphorylating and activating phosphatidylinositol-4 kinase IIIbeta at the Golgi complex. Nat. Cell Biol. 7, 880-886.
26. Hooker,A.D., Green,N.H., Baines,A.J., Bull,A.T., Jenkins,N., Strange,P.G., and James,D.C. (1999). Constraints on the transport and glycosylation of recombinant IFN-gamma in Chinese hamster ovary and insect cells. Biotechnol. Bioeng. 63, 559-572.
27. Hu,S., Shively,L., Raubitschek,A., Sherman,M., Williams,L.E., Wong,J.Y., Shively,J.E., and Wu,A.M. (1996). Minibody: A novel engineered anti-carcinoembryonic antigen antibody fragment (single-chain Fv-CH3) which exhibits rapid, high-level targeting of xenografts. Cancer Res. 56, 3055-3061.
28. Huston,J.S., Levinson,D., Mudgett-Hunter,M., Tai,M.S., Novotny,J., Margolies,M.N., Ridge,R.J., Bruccoleri,R.E., Haber,E., Crea,R., and. (1988). Protein engineering of antibody binding sites: recovery of specific activity in an anti-digoxin single-chain Fv analogue produced in Escherichia coli. Proc. Natl. Acad. Sci. U. S. A 85, 5879-5883.
29. Iglesias,T., Cabrera-Poch,N., Mitchell,M.P., Naven,T.J., Rozengurt,E., and Schiavo,G. (2000). Identification and cloning of Kidins220, a novel neuronal substrate of protein kinase D. J. Biol. Chem. 275, 40048-40056.
30. Iwakoshi,N.N., Lee,A.H., and Glimcher,L.H. (2003). The X-box binding protein-1 transcription factor is required for plasma cell differentiation and the unfolded protein response. Immunol Rev 194, 29-38.
31. Jaggi,M., Rao,P.S., Smith,D.J., Wheelock,M.J., Johnson,K.R., Hemstreet,G.P., and Balaji,K.C. (2005). E-cadherin phosphorylation by protein kinase D1/protein kinase C {mu} is associated with altered cellular aggregation and motility in prostate cancer. Cancer Res. 65, 483-492.
32. Kaufmann and Fussenegger (2003). Gene Transfer and Expression in Mammalian Cells.
33. Kawano,M., Kumagai,K., Nishijima,M., and Hanada,K. (2006). Efficient trafficking of ceramide from the endoplasmic reticulum to the Golgi apparatus requires a VAMP-associated protein-interacting FFAT motif of CERT. J. Biol Chem. 281, 30279-30288.
34. Kortt,A.A., Lah,M., Oddie,G.W., Gruen,C.L., Burns,J.E., Pearce,L.A., Atwell,J.L., McCoy,A.J., Howlett,G.J., Metzger,D.W., Webster,R.G., and Hudson,P.J. (1997). Single-chain Fv fragments of anti-neuraminidase antibody NC10 containing five- and ten-residue linkers form dimers and with zero-residue linker a trimer. Protein Eng 10, 423-433.
35. Levine,T. and Loewen,C. (2006). Inter-organelle membrane contact sites: through a glass, darkly. Curr. Opin. Cell Biol 18, 371-378.
36. Levine,T.P. and Munro,S. (2002). Targeting of Golgi-specific pleckstrin homology domains involves both PtdIns 4-kinase-dependent and -independent components. Curr. Biol. 12, 695-704.
37. Liljedahl,M., Maeda,Y., Colanzi,A., Ayala,I., Van,L.J., and Malhotra,V. (2001). Protein kinase D regulates the fission of cell surface destined transport carriers from the trans-Golgi network. Cell 104, 409-420.
38. Litvak,V., Dahan,N., Ramachandran,S., Sabanay,H., and Lev,S. (2005). Maintenance of the diacylglycerol level in the Golgi apparatus by the Nir2 protein is critical for Golgi secretory function. Nat. Cell Biol. 7, 225-234.
39. Loewen,C.J., Roy,A., and Levine,T.P. (2003). A conserved ER targeting motif in three families of lipid binding proteins and in Opilp binds VAP. EMBO J. 22, 2025-2035.
40. Lottspeich and Zorbas (1998). Buch ?
41. Lovejoy,B., Choe,S., Cascio,D., McRorie,D.K., DeGrado,W.F., and Eisenberg,D. (1993). Crystal structure of a synthetic triple-stranded alpha-helical bundle. Science 259, 1288-1293.
42. Madden,T.L., Tatusov,R.L., and Zhang,J. (1996). Applications of network BLAST server. Methods Enzymol. 266, 131-141.
43. Maeda,Y., Beznoussenko,G.V., Van,L.J., Mironov,A.A., and Malhotra,V. (2001). Recruitment of protein kinase D to the trans-Golgi network via the first cysteine-rich domain. EMBO J. 20, 5982-5990.
44. Olayioye,M.A., Vehring,S., Muller,P., Herrmann,A., Schiller,J., Thiele,C., Lindeman,G.J., Visvader,J.E., and Pomorski,T. (2005). StarD10, a START domain protein overexpressed in breast cancer, functions as a phospholipid transfer protein. J. Biol. Chem. 280, 27436-27442.
45. Overall,C.M. and Kleifeld,O. (2006). Tumour microenvironment - opinion: validating matrix metalloproteinases as drug targets and anti-targets for cancer therapy. Nat Rev Cancer 6, 227-239.
46. Pack,P., Kujau,M., Schroeckh,V., Knupfer,U., Wenderoth,R., Riesenberg,D., and Pluckthun,A. (1993). Improved bivalent miniantibodies, with identical avidity as whole antibodies, produced by high cell density fermentation of Escherichia coli. Biotechnology (N. Y.) 11, 1271-1277.
47. Pack,P., Muller,K., Zahn,R., and Pluckthun,A. (1995). Tetravalent miniantibodies with high avidity assembling in Escherichia coli. J. Mol. Biol. 246, 28-34.
48. Pak,C.O., Hunt,M.N., Bridges,M.W., Sleigh,M.J., and Gray,P.P. (1996). Super-CHO-A cell line capable of autocrine growth under fully defined protein-free conditions. Cytotechnology V22, 139-146.
49. Perisic,O., Webb,P.A., Holliger,P., Winter,G., and Williams,R.L. (1994). Crystal structure of a diabody, a bivalent antibody fragment. Structure. 2, 1217-1226.
50. Perry,R.J. and Ridgway,N.D. (2005). Molecular mechanisms and regulation of ceramide transport. Biochim. Biophys. Acta 1734, 220-234.
51. Raya,A., Revert-Ros,F., Martinez-Martinez,P., Navarro,S., Rosello,E., Vieites,B., Granero,F., Forteza,J., and Saus,J. (2000). Goodpasture antigen-binding protein, the kinase that phosphorylates the goodpasture antigen, is an alternatively spliced variant implicated in autoimmune pathogenesis. J. Biol. Chem. 275, 40392-40399.
52. Robert Scopes (1988). Protein Purification. Springer-Verlag).
53. Rykx,A., De,K.L., Mikhalap,S., Vantus,T., Seufferlein,T., Vandenheede,J.R., and Van,L.J. (2003). Protein kinase D: a family affair. FEBS Lett. 546, 81-86.
54. Sambrook,J., Fritsch,d.F., and Maniatis,T. (1989). Molecular Cloning: A Laboratory Manual. (Cold Spring Harbor: Cold Spring Harbor Laboratory Press).
55. Schroder,M. (2006). The unfolded protein response. Mol Biotechnol. 34, 279-290.
56. Seth,G., Hossler,P., Yee,J.C., and Hu,W.S. (2006). Engineering cells for cell culture bioprocessing--physiological fundamentals. Adv. Biochem. Eng Biotechnol. 101, 119-164.
57. Shaffer,A.L., Shapiro-Shelef,M., Iwakoshi,N.N., Lee,A.H., Qian,S.B., Zhao,H., Yu,X., Yang,L., Tan,B.K., Rosenwald,A., Hurt,E.M., Petroulakis,E., Sonenberg,N., Yewdell,J.W., Calame,K., Glimcher,L.H., and Staudt,L.M. (2004). XBP1, downstream of Blimp-1, expands the secretory apparatus and other organelles, and increases protein synthesis in plasma cell differentiation. Immunity. 21, 81-93.
58. Soccio,R.E. and Breslow,J.L. (2003). StAR-related lipid transfer (START) proteins: mediators of intracellular lipid metabolism. J. Biol Chem. 278, 22183-22186.
59. Somerharju,P. (2002). Pyrene-labeled lipids as tools in membrane biophysics and cell biology. Chem. Phys. Lipids 116, 57-74.
60. Tigges,M. and Fussenegger,M. (2006). Xbp1-based engineering of secretory capacity enhances the productivity of Chinese hamster ovary cells. Metab Eng.
61. Toth,B., Balla,A., Ma,H., Knight,Z.A., Shokat,K.M., and Balla,T. (2006). Phosphatidylinositol 4-kinase IIIbeta regulates the transport of ceramide between the endoplasmic reticulum and Golgi. J. Biol. Chem. 281, 36369-36377.
62. Tsujishita,Y. and Hurley,J.H. (2000). Structure and lipid transport mechanism of a StAR-related domain. Nat. Struct. Biol. 7, 408-414.
63. Urlaub,G., Kas,E., Carothers,A.M., and Chasin,L.A. (1983). Deletion of the diploid dihydrofolate reductase locus from cultured mammalian cells. Cell 33, 405-412.
64. Vega,R.B., Harrison,B.C., Meadows,E., Roberts,C.R., Papst,P.J., Olson,E.N., and McKinsey,T.A. (2004). Protein kinases C and D mediate agonist-dependent cardiac hypertrophy through nuclear export of histone deacetylase 5. Mol. Cell Biol. 24, 8374-8385.
65. Wang,Q.J. (2006). PKD at the crossroads of DAG and PKC signaling. Trends Pharmacol. Sci. 27, 317-323.
66. Wang,Y., Waldron,R.T., Dhaka,A., Patel,A., Riley,M.M., Rozengurt,E., and Colicelli,J. (2002). The RAS effector RIN1 directly competes with RAF and is regulated by 14-3-3 proteins. Mol. Cell Biol. 22, 916-926.
67. Weigert,R., Silletta,M.G., Spano,S., Turacchio,G., Cericola,C., Colanzi,A., Senatore,S., Mancini,R., Polishchuk,E.V., Salmona,M., Facchiano,F., Burger,K.N., Mironov,A., Luini,A., and Corda,D. (1999). CtBPBARS induces fission of Golgi membranes by acylating lysophosphatidic acid. Nature 402, 429-433.
68. Werner,R.G. (2004). Economic aspects of commercial manufacture of biopharmaceuticals. J. Biotechnol. 113, 171-182.
69. Wirtz,K.W. (2006). Phospholipid transfer proteins in perspective. FEBS Lett. 580, 5436-5441.
70. Wurm,F.M. (2004). Production of recombinant protein therapeutics in cultivated mammalian cells. Nat. Biotechnol. 22, 1393-1398.
71. Yeaman,C., Ayala,M.I., Wright,J.R., Bard,F., Bossard,C., Ang,A., Maeda,Y., Seufferlein,T., Mellman,I., Nelson,W.J., and Malhotra,V. (2004). Protein kinase D regulates basolateral membrane protein exit from trans-Golgi network. Nat. Cell Biol. 6, 106-112.
72. Zhang,J. and Madden,T.L. (1997). PowerBLAST: a new network BLAST application for interactive or automated sequence analysis and annotation. Genome Res. 7, 649-656.

## Claims

1. Method of producing a heterologous protein of interest in a cell comprising
a. Increasing the expression or activity of a protein having an amino acid sequence comprising a steroidogenic acute regulatory related lipid transfer (START) domain or aderivative or mutant thereof, and
b. Effecting the expression of said protein of interest.

2. Method according to claim 1 whereby the START domain protein is a mammalian START domain family member such as PCTP (SEQ ID NO. 27), StarD7, GPBP, StarD10, StarD8, StarD13, DLC-1, StarD4 (SEQ ID NO. 21), StarD6 (SEQ ID NO. 25), StarD5 (SEQ ID NO. 23), MLN64, StAR, THEA-2, CACH or StarD9 or a derivative or mutant thereof.

3. Method according to claims 1 or 2 whereby the START domain protein is **characterized by** being induced upon ER stress and/or is structurally **characterized by** consisting solely of a START domain such as StarD4 (SEQ ID NO. 21), StarD5 (SEQ ID NO. 23), StarD6 (SEQ ID NO. 25) or phosphatidylcholin transfer protein (PCTP) (SEQ ID NO. 27).

4. Method according to claims 1 to 3 whereby the START domain comprises at least the 219 amino acid START domain of CERT_{L} (SEQ ID NO. 19), or at least the 223 amino acid START domain of CERT and CERT S132A (SEQ ID NO. 17), or at least the START domain of StarD4 (SEQ ID NO. 21) or at least the START domain of StarD5 (SEQ ID NO. 23) or a derivative or mutant thereof.

5. Method according to claims 1 to 4 whereby the START domain protein is ceramide transfer protein CERT (SEQ ID NO. 11 or SEQ ID NO. 13) or a derivative or mutant thereof.

6. Method according to claim 5 whereby the START domain protein is mutated ceramide transfer protein CERT and said mutation disables and /or deletes a phosphorylation site at any serine, threonine or tyrosine position of CERT.

7. Method according to claim 6 whereby the START domain protein is mutated ceramide transfer protein CERT and said mutation disables and /or deletes the protein kinase D (PKD) phosphorylation site of CERT at position 132.

8. Method according to claim 7 whereby the mutated CERT is CERT _{S132A} (SEQ ID NO. 15).

9. Method according to claims 1 to 8 whereby said method results in increased specific cellular productivity of said protein of interest in said cell in comparison to a control cell expressing said protein of interest, but whereby said control cell does not have increased expression or activity of a protein having an amino acid sequence comprising a steroidogenic acute regulatory related lipid transfer (START) domain or a derivative or mutant thereof.

10. Method according to claim 9 whereby the increase in productivity is about 5% to about 10%, about 11 % to about 20%, about 21 % to about 30%, about 31 % to about 40%, about 41 % to about 50%, about 51 % to about 60%, about 61 % to about 70%, about 71 % to about 80%, about 81% to about 90%, about 91 % to about 100%, about 101% to about 149%, about 150% to about 199%, about 200% to about 299%, about 300% to about 499%, or about 500% to about 1000%.

11. Method according to any of claims 1 to 10 whereby said cell is a eukaryotic cell such as a yeast, plant, worm, insect, avian, fish, reptile or mammalian cell.

12. Method according to claim 11 whereby said eukaryotic cell is a mammalian cell.

13. Method according to claim 12 whereby said mammalian cell is a Chinese Hamster Ovary (CHO), monkey kidney CV1, monkey kidney COS, human lens epitheliaim PER.C6TM, human embryonic kidney, HEK293, baby hamster kidney, African green monkey kidney, human cervical carcinoma, canine kidney, buffalo rat liver, human lung, human liver, mouse mammary tumor or myeloma cell, a dog, pig or macaque cell, rat, rabbit, cat, goat, preferably a CHO cell.

14. Method according to claim 13 whereby said CHO cell is CHO wild type, CHO K1, CHO DG44, CHO DUKX-B11, CHO Pro-5, preferably CHO DG44.

15. Method according to claims 1 to 14 whereby the protein of interest is a membrane or secreted protein.

16. Method according to claim 15 whereby the protein of interest is an antibody or antibody fragment.

17. Method according to claim 16 whereby the antibody is monoclonal, polyclonal, mammalian, murine, chimeric, humanized, primatized, primate, human or an antibody fragment or derivative thereof such as antibody, immunoglobulin light chain, immunoglobulin heavy chain, immunoglobulin light and heavy chains, Fab, F(ab')2, Fc, Fc-Fc fusion proteins, Fv, single chain Fv, single domain Fv, tetravalent single chain Fv, disulfide-linked Fv, domain deleted, minibody, diabody, or a fusion polypeptide of one of the above fragments with another peptide or polypeptide, Fc-peptide fusion, Fc-toxine fusion, scaffold proteins.

18. Method of increasing specific cellular productivity of a membrane or secreted protein of interest in a cell comprising introducing into a cell one or more vector systems comprising nucleic acid sequences encoding for at least two polypeptides whereby
a. a first polynucleotide encodes a protein having an amino acid sequence comprising a steroidogenic acute regulatory related lipid transfer (START) domain or a derivative or mutant thereof and
b. a second polynucleotide encodes a protein of interest
c. and whereby the protein of interest and the protein having an amino acid sequence comprising a steroidogenic acute regulatory related lipid transfer (START) domain or a derivative or mutant thereof are expressed by said cell.

19. Method of increasing the transfection efficiency of a cell expressing a membrane or secreted protein of interest in a cell comprising
a. transfecting said cell with a first polynucleotide encoding a protein having an amino acid sequence comprising a steroidogenic acute regulatory related lipid transfer (START) domain or a derivative or mutant thereof,
b. subsequently transfecting said cell with a second polynucleotide encoding a protein of interest,
c. whereby said first and second polynucleotides are located on different vector systems.

20. Expression vector comprising two polynucleotides,
a. a first polynucleotide encoding for a protein having an amino acid sequence comprising a steroidogenic acute regulatory related lipid transfer (START) domain or a derivative or mutant thereof and
b. a second polynucleotide encoding for a protein of interest.

21. Expression vector according to claim 20 whereby the START domain protein is a mammalian START domain family member such as PCTP, StarD7, GPBP, StarD10, StarD8, StarD13, DLC-1, StarD4, StarD6, StarD5, MLN64, StAR, THEA-2, CACH or StarD9 or a derivative or mutant thereof.

22. Expression vector according to claim 20 or 21 whereby the START domain protein is ceramide transfer protein CERT (SEQ ID NO. 11 or SEQ ID NO. 13) or a derivative or mutant thereof.

23. Expression vector according to claims 22 whereby the mutated CERT is CERT _{S132A} (SEQ ID NO. 15).

24. Expression vector of claims 20 to 23 whereby said first polynucleotide increases the protein transport in a cell via the secretory pathway.

25. A cell comprising the expression vector of claims 20 to 24.

26. A cell according to claim 25 whereby said cell is a eukaryotic cell such as a yeast, plant, worm, insect, avian, fish, reptile or mammalian cell.

27. A cell according to claim 26 whereby said eukaryotic cell is a mammalian cell.

28. A cell according to claim 27 whereby said mammalian cell is a Chinese Hamster Ovary (CHO), monkey kidney CV1, monkey kidney COS, human lens epitheliaim PER.C6TM, human embryonic kidney, HEK 293, baby hamster kidney, African green monkey kidney, human cervical carcinoma, canine kidney, buffalo rat liver, human lung, human liver, mouse mammary tumor or myeloma cell, a dog, pig or macaque cell, rat, rabbit, cat, goat, preferably a CHO cell.

29. A cell according to claim 28 whereby said CHO cell is CHO wild type, CHO K1, CHO DG44, CHO DUKX-B11, CHO Pro-5, preferably CHO DG44.

30. A protein of interest, preferably an antibody produced by any of the methods according to claim 1 to 29.

31. Pharmaceutical composition comprising a polynucleotide sequence useful for blocking or reducing the expression of a protein having an amino acid sequence comprising a steroidogenic acute regulatory related lipid transfer (START) domain or a derivative or mutant thereof.

32. Pharmaceutical composition according to claim 31 whereby the START domain sequence is ceramide transfer protein CERT (SEQ ID NO. 11 or SEQ ID NO. 13) or a derivative or mutant thereof.

33. Pharmaceutical composition according to claim 31 or 32 whereby the polynucleotide sequence is RNAi, siRNA or antisense-RNA.

34. Pharmaceutical composition comprising an inhibitor or suppressor of a protein having an amino acid sequence comprising a steroidogenic acute regulatory related lipid transfer (START) domain, preferably CERT (SEQ ID NO. 11 or SEQ ID NO. 13) or a derivative or mutant thereof.

35. Method for identifying a modulator of START domain protein function, preferably CERT function, comprising
a. providing a protein having an amino acid sequence comprising a steroidogenic acute regulatory related lipid transfer (START) domain or a derivative or mutant thereof, preferably CERT,
b. contacting said protein of step a) with a test agent,
c. determining an effect related to increased or decreased protein secretion or expression of cell-surface proteins.

36. Method comprising application of a pharmaceutical composition according to claims 31 to 34 for the treatment of cancer.

37. Use of a START domain protein or a polynucleotide encoding for a START domain protein to increase secretion and /or production of a protein of interest.

38. Diagnostic use of any of the methods, expression vectors, cells or pharmaceutical compositions of claims 1 to 38.


SEQUENCE LISTING

<110>  Boehringer Ingelheim Pharma GmbH & CO KG

<120>  Improvement of protein production

<130>  P01-2188

<160>  27

<170>  PatentIn version 3.3

<210>  1
<211>  28
<212>  DNA
<213>  Artificial

<220>
<223>  PCR primer for human DNA CERT-S132A

<400>  1
cgtcgacatg gcgcaatggt gtccctgg                                          28


<210>  2
<211>  28
<212>  DNA
<213>  Artificial

<220>
<223>  PCR primer for human DNA CERT-S132Arev

<400>  2
ccagggacac cattgcgcca tgtcgacg                                          28


<210>  3
<211>  35
<212>  DNA
<213>  Artificial

<220>
<223>  PCR primer for human DNACERT-S272A

<400>  3
ggttaaacgt gaggacgcct ggcagaagag actgg                                  35


<210>  4
<211>  35
<212>  DNA
<213>  Artificial

<220>
<223>  PCR primer for human DNA CERT-S272Arev

<400>  4
ccagtctctt ctgccaggcg tcctcacgtt taacc                                  35


<210>  5
<211>  33
<212>  DNA
<213>  Artificial

<220>
<223>  PCR primer for human DNA CERT-138truncation

<400>  5

ggtgtccctg gtgtcttgag caagtggcta ctc 33

<210> 6
<211> 33
<212> DNA
<213> Artificial

<220>
<223> PCR primer for human DNA CERT-138truncationrev

<400> 6
gagtagccac ttgctcaaga caccagggac acc 33

<210> 7
<211> 21
<212> DNA
<213> Artificial

<220>
<223> siRNA/DNA siCERT-1

<400> 7
ccacaugacu uacucauuat t 21

<210> 8
<211> 21
<212> DNA
<213> Artificial

<220>
<223> siRNA/DNA siCERT-2

<400> 8
gaacagagga agcauauaat t 21

<210> 9
<211> 21
<212> DNA
<213> Artificial

<220>
<223> siRNA/DNA siLacZ

<400> 9
gcggcugccg gaauuuacct t 21

<210> 10
<211> 1797
<212> DNA
<213> human: CERT cDNA

<400> 10
atgtcggata atcagagctg gaactcgtcg ggctcggagg aggatccaga gacggagtct 60

gggccgcctg tggagcgctg cggggtcctc agtaagtgga caaactacat tcatgggtgg 120

caggatcgtt gggtagtttt gaaaaataat gctctgagtt actacaaatc tgaagatgaa 180

acagagtatg gctgcagagg atccatctgt cttagcaagg ctgtcatcac acctcacgat 240

tttgatgaat gtcgatttga tattagtgta aatgatagtg tttggtatct tcgtgctcag 300

gatccagatc atagacagca atggatagat gccattgaac agcacaagac tgaatctgga 360

tatggatctg aatccagctt gcgtcgacat ggctcaatgg tgtccctggt gtctggagca 420
agtggctact ctgcaacatc cacctcttca ttcaagaaag gccacagttt acgtgagaag 480
ttggctgaaa tggaaacatt tagagacatc ttatgtagac aagttgacac gctacagaag 540
tactttgatg cctgtgctga tgctgtctct aaggatgaac ttcaaaggga taaagtggta 600
gaagatgatg aagatgactt tcctacaacg cgttctgatg gtgacttctt gcatagtacc 660
aacggcaata aagaaaagtt atttccacat gtgacaccaa aaggaattaa tggtatagac 720
tttaaagggg aagcgataac ttttaaagca actactgctg gaatccttgc aacactttct 780
cattgtattg aactaatggt taaacgtgag gacagctggc agaagagact ggataaggaa 840
actgagaaga aaagaagaac agaggaagca tataaaaatg caatgacaga acttaagaaa 900
aaatcccact ttggaggacc agattatgaa gaaggcccta acagtctgat taatgaagaa 960
gagttctttg atgctgttga agctgctctt gacagacaag ataaaataga agaacagtca 1020
cagagtgaaa aggtgagatt acattggcct acatccttgc cctctggaga tgccttttct 1080
tctgtgggga cacatagatt tgtccaaaag gttgaagaga tggtgcagaa ccacatgact 1140
tactcattac aggatgtagg cggagatgcc aattggcagt tggttgtaga agaaggagaa 1200
atgaaggtat acagaagaga agtagaagaa aatgggattg ttctggatcc tttaaaagct 1260
acccatgcag ttaaaggcgt cacaggacat gaagtctgca attatttctg gaatgttgac 1320
gttcgcaatg actgggaaac aactatagaa aactttcatg tggtggaaac attagctgat 1380
aatgcaatca tcatttatca aacacacaag agggtgtggc ctgcttctca gcgagacgta 1440
ttatatcttt ctgtcattcg aaagatacca gccttgactg aaaatgaccc tgaaacttgg 1500
atagtttgta atttttctgt ggatcatgac agtgctcctc taaacaaccg atgtgtccgt 1560
gccaaaataa atgttgctat gatttgtcaa accttggtaa gcccaccaga gggaaaccag 1620
gaaattagca gggacaacat tctatgcaag attacatatg tagctaatgt gaaccctgga 1680
ggatgggcac cagcctcagt gttaagggca gtggcaaagc gagagtatcc taaatttcta 1740
aaacgtttta cttcttacgt ccaagaaaaa actgcaggaa agcctatttt gttctag 1797

<210> 11
<211> 598
<212> PRT
<213> human: CERT protein

<400> 11

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly

50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val
        355                 360                 365

Gln Lys Val Glu Glu Met Val Gln Asn His Met Thr Tyr Ser Leu Gln
    370                 375                 380

Asp Val Gly Gly Asp Ala Asn Trp Gln Leu Val Val Glu Glu Gly Glu
385                 390                 395                 400

Met Lys Val Tyr Arg Arg Glu Val Glu Glu Asn Gly Ile Val Leu Asp
                405                 410                 415

Pro Leu Lys Ala Thr His Ala Val Lys Gly Val Thr Gly His Glu Val
            420                 425                 430

Cys Asn Tyr Phe Trp Asn Val Asp Val Arg Asn Asp Trp Glu Thr Thr
        435                 440                 445

Ile Glu Asn Phe His Val Val Glu Thr Leu Ala Asp Asn Ala Ile Ile
    450                 455                 460

Ile Tyr Gln Thr His Lys Arg Val Trp Pro Ala Ser Gln Arg Asp Val
465                 470                 475                 480

Leu Tyr Leu Ser Val Ile Arg Lys Ile Pro Ala Leu Thr Glu Asn Asp
                485                 490                 495

Pro Glu Thr Trp Ile Val Cys Asn Phe Ser Val Asp His Asp Ser Ala
            500                 505                 510

Pro Leu Asn Asn Arg Cys Val Arg Ala Lys Ile Asn Val Ala Met Ile
        515                 520                 525

Cys Gln Thr Leu Val Ser Pro Pro Glu Gly Asn Gln Glu Ile Ser Arg
    530                 535                 540

Asp Asn Ile Leu Cys Lys Ile Thr Tyr Val Ala Asn Val Asn Pro Gly
545                 550                 555                 560

Gly Trp Ala Pro Ala Ser Val Leu Arg Ala Val Ala Lys Arg Glu Tyr
                565                 570                 575

Pro Lys Phe Leu Lys Arg Phe Thr Ser Tyr Val Gln Glu Lys Thr Ala
            580                 585                 590

Gly Lys Pro Ile Leu Phe
        595

<210> 12
<211> 2389
<212> DNA
<213> human: CERT L cDNA

<400> 12
gcaggaagat ggcggcggta gcggaggtgt gagtggacgc gggactcagc ggccggattt 60

tctcttccct tcttttccct tttccttccc tatttgaaat tggcatcgag ggggctaagt 120

tcgggtggca gcgccgggcg caacgcaggg gtcacggcga cggcggcggc ggctgacggc 180

tggaagggta ggcttcattc accgctcgtc ctccttcctc gctccgctcg gtgtcaggcg 240

cggcggcggc gcggcgggcg gacttcgtcc ctcctcctgc tcccccccac accggagcgg 300

gcactcttcg cttcgccatc ccccgaccct tcaccccgag gactgggcgc ctcctccggc 360

gcagctgagg gagcgggggc cggtctcctg ctcggttgtc gagcctccat gtcggataat 420

cagagctgga actcgtcggg ctcggaggag gatccagaga cggagtctgg gccgcctgtg 480

gagcgctgcg gggtcctcag taagtggaca aactacattc atgggtggca ggatcgttgg 540

gtagttttga aaaataatgc tctgagttac tacaaatctg aagatgaaac agagtatggc 600

tgcagaggat ccatctgtct tagcaaggct gtcatcacac ctcacgattt tgatgaatgt 660

cgatttgata ttagtgtaaa tgatagtgtt tggtatcttc gtgctcagga tccagatcat 720

agacagcaat ggatagatgc cattgaacag cacaagactg aatctggata tggatctgaa 780

tccagcttgc gtcgacatgg ctcaatggtg tccctggtgt ctggagcaag tggctactct 840

gcaacatcca cctcttcatt caagaaaggc cacagtttac gtgagaagtt ggctgaaatg 900

gaaacattta gagacatctt atgtagacaa gttgacacgc tacagaagta ctttgatgcc 960

tgtgctgatg ctgtctctaa ggatgaactt caaagggata aagtggtaga agatgatgaa 1020

gatgactttc ctacaacgcg ttctgatggt gacttcttgc atagtaccaa cggcaataaa 1080

gaaaagttat ttccacatgt gacaccaaaa ggaattaatg gtatagactt taaaggggaa 1140

gcgataactt ttaaagcaac tactgctgga atccttgcaa cactttctca ttgtattgaa 1200

ctaatggtta aacgtgagga cagctggcag aagagactgg ataaggaaac tgagaagaaa 1260

agaagaacag aggaagcata taaaaatgca atgacagaac ttaagaaaaa atcccacttt 1320

ggaggaccag attatgaaga aggccctaac agtctgatta atgaagaaga gttctttgat 1380

gctgttgaag ctgctcttga cagacaagat aaaatagaag aacagtcaca gagtgaaaag 1440

gtgagattac attggcctac atccttgccc tctggagatg ccttttcttc tgtggggaca 1500

catagatttg tccaaaagcc ctatagtcgc tcttcctcca tgtcttccat tgatctagtc 1560

agtgcctctg atgatgttca cagattcagc tcccaggttg aagagatggt gcagaaccac 1620

atgacttact cattacagga tgtaggcgga gatgccaatt ggcagttggt tgtagaagaa 1680

ggagaaatga aggtatacag aagagaagta gaagaaaatg ggattgttct ggatccttta 1740

aaagctaccc atgcagttaa aggcgtcaca ggacatgaag tctgcaatta tttctggaat 1800

gttgacgttc gcaatgactg ggaaacaact atagaaaact ttcatgtggt ggaaacatta 1860

gctgataatg caatcatcat ttatcaaaca cacaagaggg tgtggcctgc ttctcagcga 1920

gacgtattat atctttctgt cattcgaaag ataccagcct tgactgaaaa tgaccctgaa 1980

acttggatag tttgtaattt ttctgtggat catgacagtg ctcctctaaa caaccgatgt 2040

gtccgtgcca aaataaatgt tgctatgatt tgtcaaacct tggtaagccc accagaggga 2100

aaccaggaaa ttagcaggga caacattcta tgcaagatta catatgtagc taatgtgaac 2160

cctggaggat gggcaccagc ctcagtgtta agggcagtgg caaagcgaga gtatcctaaa 2220

tttctaaaac gttttacttc ttacgtccaa gaaaaaactg caggaaagcc tattttgttc 2280

tagtattaac aggtactaga agatatgttt tatctttttt taactttatt tgactaatat 2340

gactgtcaat actaaaattt agttgttgaa agtatttact atgtttttt 2389

<210> 13
<211> 624
<212> PRT
<213> human: CERT L protein

<400> 13

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val
        355                 360                 365

Gln Lys Pro Tyr Ser Arg Ser Ser Ser Met Ser Ser Ile Asp Leu Val
    370                 375                 380

Ser Ala Ser Asp Asp Val His Arg Phe Ser Ser Gln Val Glu Glu Met
385                 390                 395                 400

Val Gln Asn His Met Thr Tyr Ser Leu Gln Asp Val Gly Gly Asp Ala
                405                 410                 415

Asn Trp Gln Leu Val Val Glu Glu Gly Glu Met Lys Val Tyr Arg Arg
            420                 425                 430

Glu Val Glu Glu Asn Gly Ile Val Leu Asp Pro Leu Lys Ala Thr His
        435                 440                 445

Ala Val Lys Gly Val Thr Gly His Glu Val Cys Asn Tyr Phe Trp Asn
    450                 455                 460

Val Asp Val Arg Asn Asp Trp Glu Thr Thr Ile Glu Asn Phe His Val
465                 470                 475                 480

Val Glu Thr Leu Ala Asp Asn Ala Ile Ile Ile Tyr Gln Thr His Lys
                485                 490                 495

Arg Val Trp Pro Ala Ser Gln Arg Asp Val Leu Tyr Leu Ser Val Ile
            500                 505                 510

Arg Lys Ile Pro Ala Leu Thr Glu Asn Asp Pro Glu Thr Trp Ile Val
        515                 520                 525

Cys Asn Phe Ser Val Asp His Asp Ser Ala Pro Leu Asn Asn Arg Cys
    530                 535                 540

Val Arg Ala Lys Ile Asn Val Ala Met Ile Cys Gln Thr Leu Val Ser
545                 550                 555                 560

Pro Pro Glu Gly Asn Gln Glu Ile Ser Arg Asp Asn Ile Leu Cys Lys
                565                 570                 575

Ile Thr Tyr Val Ala Asn Val Asn Pro Gly Gly Trp Ala Pro Ala Ser
            580                 585                 590

Val Leu Arg Ala Val Ala Lys Arg Glu Tyr Pro Lys Phe Leu Lys Arg
        595                 600                 605

Phe Thr Ser Tyr Val Gln Glu Lys Thr Ala Gly Lys Pro Ile Leu Phe
    610                 615                 620

<210>  14
<211>  1797
<212>  DNA
<213>  human: CERT S132A cDNA

<400>  14
atgtcggata atcagagctg gaactcgtcg ggctcggagg aggatccaga gacggagtct      60

gggccgcctg tggagcgctg cggggtcctc agtaagtgga caaactacat tcatgggtgg     120

caggatcgtt gggtagtttt gaaaaataat gctctgagtt actacaaatc tgaagatgaa     180

acagagtatg gctgcagagg atccatctgt cttagcaagg ctgtcatcac acctcacgat     240

tttgatgaat gtcgatttga tattagtgta aatgatagtg tttggtatct tcgtgctcag     300

gatccagatc atagacagca atggatagat gccattgaac agcacaagac tgaatctgga     360

tatggatctg aatccagctt gcgtcgacat ggcgcaatgg tgtccctggt gtctggagca     420

agtggctact ctgcaacatc cacctcttca ttcaagaaag gccacagttt acgtgagaag     480

ttggctgaaa tggaaacatt tagagacatc ttatgtagac aagttgacac gctacagaag     540

tactttgatg cctgtgctga tgctgtctct aaggatgaac ttcaaaggga taaagtggta     600

gaagatgatg aagatgactt tcctacaacg cgttctgatg gtgacttctt gcatagtacc 660

aacggcaata aagaaaagtt atttccacat gtgacaccaa aaggaattaa tggtatagac 720

tttaaagggg aagcgataac ttttaaagca actactgctg gaatccttgc aacactttct 780

cattgtattg aactaatggt taaacgtgag gacagctggc agaagagact ggataaggaa 840

actgagaaga aaagaagaac agaggaagca tataaaaatg caatgacaga acttaagaaa 900

aaatcccact ttggaggacc agattatgaa gaaggcccta acagtctgat taatgaagaa 960

gagttctttg atgctgttga agctgctctt gacagacaag ataaaataga agaacagtca 1020

cagagtgaaa aggtgagatt acattggcct acatccttgc cctctggaga tgccttttct 1080

tctgtgggga cacatagatt tgtccaaaag gttgaagaga tggtgcagaa ccacatgact 1140

tactcattac aggatgtagg cggagatgcc aattggcagt tggttgtaga agaaggagaa 1200

atgaaggtat acagaagaga agtagaagaa aatgggattg ttctggatcc tttaaaagct 1260

acccatgcag ttaaaggcgt cacaggacat gaagtctgca attatttctg gaatgttgac 1320

gttcgcaatg actgggaaac aactatagaa aactttcatg tggtggaaac attagctgat 1380

aatgcaatca tcatttatca aacacacaag agggtgtggc ctgcttctca gcgagacgta 1440

ttatatcttt ctgtcattcg aaagatacca gccttgactg aaaatgaccc tgaaacttgg 1500

atagtttgta atttttctgt ggatcatgac agtgctcctc taaacaaccg atgtgtccgt 1560

gccaaaataa atgttgctat gatttgtcaa accttggtaa gcccaccaga gggaaaccag 1620

gaaattagca gggacaacat tctatgcaag attacatatg tagctaatgt gaaccctgga 1680

ggatgggcac cagcctcagt gttaagggca gtggcaaagc gagagtatcc taaatttcta 1740

aaacgtttta cttcttacgt ccaagaaaaa actgcaggaa agcctatttt gttctag 1797

<210> 15
<211> 598
<212> PRT
<213> human: CERT S132A protein

<400> 15

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ala Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val

355                 360                 365

Gln Lys Val Glu Glu Met Val Gln Asn His Met Thr Tyr Ser Leu Gln
    370                 375                 380

Asp Val Gly Gly Asp Ala Asn Trp Gln Leu Val Val Glu Glu Gly Glu
385                 390                 395                 400

Met Lys Val Tyr Arg Arg Glu Val Glu Glu Asn Gly Ile Val Leu Asp
                405                 410                 415

Pro Leu Lys Ala Thr His Ala Val Lys Gly Val Thr Gly His Glu Val
            420                 425                 430

Cys Asn Tyr Phe Trp Asn Val Asp Val Arg Asn Asp Trp Glu Thr Thr
        435                 440                 445

Ile Glu Asn Phe His Val Val Glu Thr Leu Ala Asp Asn Ala Ile Ile
    450                 455                 460

Ile Tyr Gln Thr His Lys Arg Val Trp Pro Ala Ser Gln Arg Asp Val
465                 470                 475                 480

Leu Tyr Leu Ser Val Ile Arg Lys Ile Pro Ala Leu Thr Glu Asn Asp
                485                 490                 495

Pro Glu Thr Trp Ile Val Cys Asn Phe Ser Val Asp His Asp Ser Ala
            500                 505                 510

Pro Leu Asn Asn Arg Cys Val Arg Ala Lys Ile Asn Val Ala Met Ile
        515                 520                 525

Cys Gln Thr Leu Val Ser Pro Pro Glu Gly Asn Gln Glu Ile Ser Arg
    530                 535                 540

Asp Asn Ile Leu Cys Lys Ile Thr Tyr Val Ala Asn Val Asn Pro Gly
545                 550                 555                 560

Gly Trp Ala Pro Ala Ser Val Leu Arg Ala Val Ala Lys Arg Glu Tyr
                565                 570                 575

Pro Lys Phe Leu Lys Arg Phe Thr Ser Tyr Val Gln Glu Lys Thr Ala
            580                 585                 590

Gly Lys Pro Ile Leu Phe
        595

<210>  16
<211>  669
<212>  DNA
<213>  human: START Domain CERT cDNA

<400>  16

agatttgtcc aaaaggttga agagatggtg cagaaccaca tgacttactc attacaggat 60

gtaggcggag atgccaattg gcagttggtt gtagaagaag gagaaatgaa ggtatacaga 120

agagaagtag aagaaaatgg gattgttctg gatcctttaa aagctaccca tgcagttaaa 180

ggcgtcacag gacatgaagt ctgcaattat ttctggaatg ttgacgttcg caatgactgg 240

gaaacaacta tagaaaactt tcatgtggtg gaaacattag ctgataatgc aatcatcatt 300

tatcaaacac acaagagggt gtggcctgct tctcagcgag acgtattata tctttctgtc 360

attcgaaaga taccagcctt gactgaaaat gaccctgaaa cttggatagt ttgtaatttt 420

tctgtggatc atgacagtgc tcctctaaac aaccgatgtg tccgtgccaa aataaatgtt 480

gctatgattt gtcaaacctt ggtaagccca ccagagggaa accaggaaat tagcagggac 540

aacattctat gcaagattac atatgtagct aatgtgaacc ctggaggatg ggcaccagcc 600

tcagtgttaa gggcagtggc aaagcgagag tatcctaaat ttctaaaacg ttttacttct 660

tacgtccaa 669

<210> 17
<211> 223
<212> PRT
<213> human: START Domain CERT protein

<400> 17

Arg Phe Val Gln Lys Val Glu Glu Met Val Gln Asn His Met Thr Tyr
1               5                   10                  15

Ser Leu Gln Asp Val Gly Gly Asp Ala Asn Trp Gln Leu Val Val Glu
            20                  25                  30

Glu Gly Glu Met Lys Val Tyr Arg Arg Glu Val Glu Glu Asn Gly Ile
        35                  40                  45

Val Leu Asp Pro Leu Lys Ala Thr His Ala Val Lys Gly Val Thr Gly
    50                  55                  60

His Glu Val Cys Asn Tyr Phe Trp Asn Val Asp Val Arg Asn Asp Trp
65                  70                  75                  80

Glu Thr Thr Ile Glu Asn Phe His Val Val Glu Thr Leu Ala Asp Asn
                85                  90                  95

Ala Ile Ile Ile Tyr Gln Thr His Lys Arg Val Trp Pro Ala Ser Gln
            100                 105                 110

Arg Asp Val Leu Tyr Leu Ser Val Ile Arg Lys Ile Pro Ala Leu Thr
        115                 120                 125

Glu Asn Asp Pro Glu Thr Trp Ile Val Cys Asn Phe Ser Val Asp His
    130                 135                 140

Asp Ser Ala Pro Leu Asn Asn Arg Cys Val Arg Ala Lys Ile Asn Val

145                 150                 155                 160

Ala Met Ile Cys Gln Thr Leu Val Ser Pro Pro Glu Gly Asn Gln Glu
                165                 170                 175

Ile Ser Arg Asp Asn Ile Leu Cys Lys Ile Thr Tyr Val Ala Asn Val
            180                 185                 190

Asn Pro Gly Gly Trp Ala Pro Ala Ser Val Leu Arg Ala Val Ala Lys
        195                 200                 205

Arg Glu Tyr Pro Lys Phe Leu Lys Arg Phe Thr Ser Tyr Val Gln
    210                 215                 220

<210>  18
<211>  658
<212>  DNA
<213>  human: START Domain CERT L cDNA

<400>  18
caggttgaag agatggtgca gaaccacatg acttactcat tacaggatgt aggcggagat      60

gccaattggc agttggttgt agaagaagga gaaatgaagg tatacagaag agaagtagaa     120

gaaaatggga ttgttctgga tcctttaaaa gctacccatg cagttaaagg cgtcacagga     180

catgaagtct gcaattattt ctggaatgtt gacgttcgca atgactggga aacaactata     240

gaaaactttc atgtggtgga aacattagct gataatgcaa tcatcattta tcaaacacac     300

aagagggtgt ggcctgcttc tcagcgagac gtattatatc tttctgtcat tcgaaagata     360

ccagccttga ctgaaaatga ccctgaaact tggatagttt gtaatttttc tgtggatcat     420

gacagtgctc ctctaaacaa ccgatgtgtc cgtgccaaaa taaatgttgc tatgatttgt     480

caaaccttgg taagcccacc agagggaaac caggaaatta gcagggacaa cattctatgc     540

aagattacat atgtagctaa tgtgaaccct ggaggatggg caccagcctc agtgttaagg     600

gcagtggcaa agcgagagta tcctaaattt ctaaaacgtt ttacttctta cgtccaag       658

<210>  19
<211>  219
<212>  PRT
<213>  human: START Domain CERT L protein

<400>  19

Gln Val Glu Glu Met Val Gln Asn His Met Thr Tyr Ser Leu Gln Asp
1               5                   10                  15

Val Gly Gly Asp Ala Asn Trp Gln Leu Val Val Glu Glu Gly Glu Met
            20                  25                  30

Lys Val Tyr Arg Arg Glu Val Glu Glu Asn Gly Ile Val Leu Asp Pro
        35                  40                  45

Leu Lys Ala Thr His Ala Val Lys Gly Val Thr Gly His Glu Val Cys
    50                  55                  60

Asn Tyr Phe Trp Asn Val Asp Val Arg Asn Asp Trp Glu Thr Thr Ile
65                  70                  75                  80

Glu Asn Phe His Val Val Glu Thr Leu Ala Asp Asn Ala Ile Ile Ile
                85                  90                  95

Tyr Gln Thr His Lys Arg Val Trp Pro Ala Ser Gln Arg Asp Val Leu
            100                 105                 110

Tyr Leu Ser Val Ile Arg Lys Ile Pro Ala Leu Thr Glu Asn Asp Pro
        115                 120                 125

Glu Thr Trp Ile Val Cys Asn Phe Ser Val Asp His Asp Ser Ala Pro
    130                 135                 140

Leu Asn Asn Arg Cys Val Arg Ala Lys Ile Asn Val Ala Met Ile Cys
145                 150                 155                 160

Gln Thr Leu Val Ser Pro Pro Glu Gly Asn Gln Glu Ile Ser Arg Asp
                165                 170                 175

Asn Ile Leu Cys Lys Ile Thr Tyr Val Ala Asn Val Asn Pro Gly Gly
            180                 185                 190

Trp Ala Pro Ala Ser Val Leu Arg Ala Val Ala Lys Arg Glu Tyr Pro
        195                 200                 205

Lys Phe Leu Lys Arg Phe Thr Ser Tyr Val Gln
    210                 215

<210>  20
<211>  2264
<212>  DNA
<213>  human: StarD4 cDNA

<400>  20
actgttgaga gcggtgtgag gtgcttggta gcgcgccgta gctgcttcca cgtccttgct      60

tcacctcagg taaagagaga agtaatggaa ggcctgtctg atgttgcttc ttttgcaact     120

aaacttaaaa acactctcat ccagtaccat agcattgaag aagataagtg gcgagttgct     180

aagaaaacga aagatgtaac tgtttggaga aaaccctcag aagaatttaa tggatatctc     240

tacaaagccc aaggtgttat agatgacctt gtctatagta taatagacca tatacgccca     300

gggccttgtc gtttggattg ggacagcttg atgacttctt tggatattct ggagaacttt     360

gaagagaatt gctgtgtgat gcgttacact actgctggtc agctttggaa tataatttcc     420

ccaagagaat ttgttgattt ctcctatact gtgggctata aagaagggct tttatcttgt     480

ggaataagtc ttgactggga tgaaaagaga ccagaatttg ttcgaggata taaccatccc     540

tgtggttggt tttgtgttcc acttaaagac aacccaaacc agagtctttt gacaggatat     600

attcagacag atctgcgtgg gatgattcct cagtctgcgg tagatacagc catggcaagc     660

actttaacca acttctatgg tgatttacga aaagctttat gagaggcaaa atacattcaa 720
acttgtagta ctacagatca actctctcag ctacatggcc tgtaaaaatc attgattcca 780
cttttctgca tagccggtag aaaaatttga aatgtttttg gttcactagt acaatgtttg 840
gttttattcc taaagtaaat agctatctaa gagagggcat tttcactttt ttttttttaa 900
attttgagac aggctctcac tctgttgccc atgctggagg gcagtggtat gatcacagct 960
cactgcagct ttgatctgac cgctcaaggg gttattctac ctcagcctcc tgaatagctg 1020
ggaatacagg tgcacgccac tatgcatggc taatttttgt ttaatttttt gtagagatgt 1080
ggtcacactg tgttgcccag gctggtcttg aactcctggc ctcaagtcat tccccacctt 1140
agcctcccaa agtgttggga ttataagcgt gagccaccat gcctggcccc aatttaaaat 1200
gtggaattca gttggtgtcc aagacttatc ttgagactct taaaagcatc agtctgtaac 1260
tagaacaaat acagtcttag atttacccaa gtgcctagat atcattttat aatgattaga 1320
attgagtatt gtgggtcccc taattctgtg ggtgccttaa gtgagaattt ctaaatgatt 1380
ttcacattct aaatgacttt gggttttgaa ctctccatct agtttacttc taaaatggga 1440
acttgaggca attcaggtat ccaggcaaat ctttgtatat atttttttgt gtacatgcac 1500
acatctcgaa atccatttcc gtgtttaatg ttagttgttt atgtgttagt attcctgtgt 1560
ctactgtttt gttgttgtta atatgggtaa agtgagccct gaaatacatg ctaaacaaga 1620
catgaaattc agaaaggtac atagtgtttc aagtgcatgg tagtttgatc tgtgttttac 1680
tttattgtgt tttcttgagt gtaaagaaag aataaatcaa agttcttcat acccattttg 1740
acaaagtgga acagtggagc tgttttttgc ttttgttttt atttattttt tgccactggt 1800
gatgatagat ttcaaaaaac aaaaggtggc agcagcacaa tgttcatggt gaattatctc 1860
atagtatcta gattgatcaa gatctgacag aaggaatgca caaaggattc tatattctta 1920
atgatttatt aattaccagg atccttttct aaattgaatg tacttttgaa ttactaggtt 1980
tcttcttttt ttttgttctg caatagtgaa agaaaactca gtagtttagt ttcagtttct 2040
catggaaatt ggtaaatgtt agttttgact tcatctattt tttatttgtt tttattagcg 2100
tagagtagga agtctcatat tctactgttc tatctaggat ggtgaaattc caaaggtgcc 2160
taacttgagt aagggatttg tgacaagata gtacacatta ctataagggc tattatttcc 2220
tgaactggat gtccctaaaa gcaaataaac tgcccactat ctct 2264

<210> 21
<211> 205
<212> PRT
<213> human: StarD4 protein

<400> 21

Met Glu Gly Leu Ser Asp Val Ala Ser Phe Ala Thr Lys Leu Lys Asn
1               5                   10                  15

Thr Leu Ile Gln Tyr His Ser Ile Glu Glu Asp Lys Trp Arg Val Ala
            20                  25                  30

Lys Lys Thr Lys Asp Val Thr Val Trp Arg Lys Pro Ser Glu Glu Phe
        35                  40                  45

Asn Gly Tyr Leu Tyr Lys Ala Gln Gly Val Ile Asp Asp Leu Val Tyr
    50                  55                  60

Ser Ile Ile Asp His Ile Arg Pro Gly Pro Cys Arg Leu Asp Trp Asp
65                  70                  75                  80

Ser Leu Met Thr Ser Leu Asp Ile Leu Glu Asn Phe Glu Glu Asn Cys
                85                  90                  95

Cys Val Met Arg Tyr Thr Thr Ala Gly Gln Leu Trp Asn Ile Ile Ser
            100                 105                 110

Pro Arg Glu Phe Val Asp Phe Ser Tyr Thr Val Gly Tyr Lys Glu Gly
        115                 120                 125

Leu Leu Ser Cys Gly Ile Ser Leu Asp Trp Asp Glu Lys Arg Pro Glu
    130                 135                 140

Phe Val Arg Gly Tyr Asn His Pro Cys Gly Trp Phe Cys Val Pro Leu
145                 150                 155                 160

Lys Asp Asn Pro Asn Gln Ser Leu Leu Thr Gly Tyr Ile Gln Thr Asp
                165                 170                 175

Leu Arg Gly Met Ile Pro Gln Ser Ala Val Asp Thr Ala Met Ala Ser
            180                 185                 190

Thr Leu Thr Asn Phe Tyr Gly Asp Leu Arg Lys Ala Leu
        195                 200                 205

<210>  22
<211>  1344
<212>  DNA
<213>  human: StarD5 cDNA

<400>  22
gagctccagc ctccaggcac ccgggatcca gcgccgccgc tcataacacc cgcgaccccg      60

cagctaagcg cagctcccga cgcaatggac ccggcgctgg cagcccagat gagcgaggct     120

gtggccgaga agatgctcca gtaccggcgg gacacagcag gctggaagat ttgccgggaa     180

ggcaatggag tttcagtttc ctggaggcca tctgtggagt ttccagggaa cctgtaccga     240

ggagaaggca ttgtatatgg gacactagag gaggtgtggg actgtgtgaa gccagctgtt     300

ggaggcctac gagtgaagtg ggatgagaat gtgaccggtt ttgaaattat ccaaagcatc     360

actgacaccc tgtgtgtaag cagaacctcc actccctccg ctgccatgaa gctcatttct     420

cccagagatt ttgtggactt ggtgctagtc aagagatatg aggatgggac catcagttcc     480

aacgccaccc atgtggagca tccgttatgt cccccgaagc caggttttgt gagaggattt     540

aaccatcctt gtggttgctt ctgtgaacct cttccagggg aacccaccaa gaccaacctg 600

gtcacattct tccataccga cctcagcggt tacctcccac agaacgtggt ggactccttc 660

ttcccccgca gcatgacccg gttttatgcc aaccttcaga aagcagtgaa gcaattccat 720

gagtaatgct atcgttactt cttggcaaag aactcccgtg actcatcgag gagctccagc 780

tgttgggaca ccaaggagcc tgggagcacg cagaggcctg tgttcactct ttggaacaag 840

ctgatggact gcgcatctct gagaatgcca accagaggcg gcagcccacc cttcctgcct 900

cctgccccac tcagggttgg cgtgtgatga gccattcatg tgttccaaac tccatctgcc 960

tgttacccaa acacgcctct cctggcaggg tagacccagg cctctaacca tctgacagag 1020

actcggcctg gacaccatgc gatgcactct ggcaccaagg ctttatgtgc ccatcactct 1080

cagagaccac gtttccctga ctgtcataga gaatcatcat cgccactgaa aaccaggccc 1140

tgttgccttt taagcatgta ccgctccctc agtcctgtgc tgcagccccc caaatatatt 1200

tttctgatat agaccttgta tatggcttta atgccgcaaa atatttattt ttccttaaaa 1260

aaggtgtcaa cttggaaata atggtttaaa aacaggataa gcattaagga aaaacaaaaa 1320

aaaaaaaaaa aaaaaaaaaa aaaa 1344

<210> 23
<211> 213
<212> PRT
<213> human: StarD5 protein

<400> 23

Met Asp Pro Ala Leu Ala Ala Gln Met Ser Glu Ala Val Ala Glu Lys
1               5                   10                  15

Met Leu Gln Tyr Arg Arg Asp Thr Ala Gly Trp Lys Ile Cys Arg Glu
            20                  25                  30

Gly Asn Gly Val Ser Val Ser Trp Arg Pro Ser Val Glu Phe Pro Gly
        35                  40                  45

Asn Leu Tyr Arg Gly Glu Gly Ile Val Tyr Gly Thr Leu Glu Glu Val
    50                  55                  60

Trp Asp Cys Val Lys Pro Ala Val Gly Gly Leu Arg Val Lys Trp Asp
65                  70                  75                  80

Glu Asn Val Thr Gly Phe Glu Ile Ile Gln Ser Ile Thr Asp Thr Leu
                85                  90                  95

Cys Val Ser Arg Thr Ser Thr Pro Ser Ala Ala Met Lys Leu Ile Ser
            100                 105                 110

Pro Arg Asp Phe Val Asp Leu Val Leu Val Lys Arg Tyr Glu Asp Gly
        115                 120                 125

Thr Ile Ser Ser Asn Ala Thr His Val Glu His Pro Leu Cys Pro Pro
    130                 135                 140

Lys Pro Gly Phe Val Arg Gly Phe Asn His Pro Cys Gly Cys Phe Cys
145                 150                 155                 160

Glu Pro Leu Pro Gly Glu Pro Thr Lys Thr Asn Leu Val Thr Phe Phe
                165                 170                 175

His Thr Asp Leu Ser Gly Tyr Leu Pro Gln Asn Val Val Asp Ser Phe
            180                 185                 190

Phe Pro Arg Ser Met Thr Arg Phe Tyr Ala Asn Leu Gln Lys Ala Val
        195                 200                 205

Lys Gln Phe His Glu
    210

<210>  24
<211>  663
<212>  DNA
<213>  human: StarD6 cDNA

<400>  24
atggacttca aggcaattgc ccaacaaact gcccaagaag ttttaggtta taatcgagat      60

acatcaggct ggaaagtggt taaaacttca aaaaagataa ctgtttccag taaggcttct     120

agaaaattcc atggaaatct atatcgtgtt gaagggataa ttccagaatc accagctaaa     180

ctatctgatt tcctctacca aactggagac agaattacat gggataaatc attgcaagtg     240

tataatatgg tacacaggat tgattcggac acattcatat gtcataccat tacacaaagt     300

tttgccgtgg gctccatttc ccctcgagac tttatcgact tagtgtacat caagcgctac     360

gaaggaaata tgaacattat cagttctaaa agtgtggatt ttccagaata tcctccatct     420

tcaaattata tccgcggtta taaccatcct tgtggctttg tatgttcacc aatggaagaa     480

aacccagcat attccaaact agtgatgttt gtccagacag aaatgagagg aaaattgtcc     540

ccatcaataa ttgaaaaaac catgccttcc aacttagtaa acttcatcct caatgcaaaa     600

gatggaataa aggcacacag aactccatca agacgtggat ttcatcataa tagtcattca     660

tga                                                                   663

<210>  25
<211>  220
<212>  PRT
<213>  human: StarD6 protein

<400>  25

Met Asp Phe Lys Ala Ile Ala Gln Gln Thr Ala Gln Glu Val Leu Gly
1               5                   10                  15

Tyr Asn Arg Asp Thr Ser Gly Trp Lys Val Val Lys Thr Ser Lys Lys
            20                  25                  30

Ile Thr Val Ser Ser Lys Ala Ser Arg Lys Phe His Gly Asn Leu Tyr
        35                  40                  45

Arg Val Glu Gly Ile Ile Pro Glu Ser Pro Ala Lys Leu Ser Asp Phe
    50                  55                  60

Leu Tyr Gln Thr Gly Asp Arg Ile Thr Trp Asp Lys Ser Leu Gln Val
65                  70                  75                  80

Tyr Asn Met Val His Arg Ile Asp Ser Asp Thr Phe Ile Cys His Thr
                85                  90                  95

Ile Thr Gln Ser Phe Ala Val Gly Ser Ile Ser Pro Arg Asp Phe Ile
            100                 105                 110

Asp Leu Val Tyr Ile Lys Arg Tyr Glu Gly Asn Met Asn Ile Ile Ser
        115                 120                 125

Ser Lys Ser Val Asp Phe Pro Glu Tyr Pro Pro Ser Ser Asn Tyr Ile
    130                 135                 140

Arg Gly Tyr Asn His Pro Cys Gly Phe Val Cys Ser Pro Met Glu Glu
145                 150                 155                 160

Asn Pro Ala Tyr Ser Lys Leu Val Met Phe Val Gln Thr Glu Met Arg
                165                 170                 175

Gly Lys Leu Ser Pro Ser Ile Ile Glu Lys Thr Met Pro Ser Asn Leu
            180                 185                 190

Val Asn Phe Ile Leu Asn Ala Lys Asp Gly Ile Lys Ala His Arg Thr
        195                 200                 205

Pro Ser Arg Arg Gly Phe His His Asn Ser His Ser
    210                 215                 220

<210>  26
<211>  1932
<212>  DNA
<213>  human: PCTP cDNA

<400>  26
ccggactgcg gaaggatgga gctggccgcc ggaagcttct cggaggagca gttctgggag      60

gcctgcgccg agctccagca gcccgctctg gccggggccg actggcagct cctagtggag     120

acctcgggca tcagcatcta ccggctgctg gacaagaaga ctggacttca tgagtataaa     180

gtctttggtg ttctggagga ctgctcacca actctactgg cagacatcta tatggactca     240

gattacagaa aacaatggga ccagtatgtt aaagaactct atgaacaaga atgcaacgga     300

gagactgtgg tctactggga agtgaagtac ccttttccca tgtccaacag agactatgtc     360

taccttcggc agcggcgaga cctggacatg gaagggagga agatccatgt gatcctggcc     420

cggagcacct ccatgcctca gcttggcgag aggtctgggg tgatccgggt gaagcaatac 480

aagcagagcc tggcgattga gagtgacggc aagaagggga gcaaagtttt catgtattac 540

ttcgataacc cgggtggcca aattccgtcc tggctcatta actgggccgc caagaatgga 600

gttcctaact tcttgaaaga catggcaaga gcctgtcaga actacctcaa gaaaacctaa 660

gaaagagaac tgggaacatt gcatccatgg gttgatgtct ctggaagtgc aaccacccaa 720

tgtctctgga agtgccacct ggaagtgcca cctggaagtg tctctggaag agcacccacc 780

actgttcagc cttcccctgc tgtttctgtc ttcagaggcc tacacactac cacatccttt 840

ctaagcatgt ttgcctgaca tccagctcac tcgtctgctt cctttctcgc tccccccatc 900

ctgggctggg ctgccttctt ctacagttca atatggggca gactagggaa acctttgctt 960

gcttactatt aggaggggaa gtcttcagta gggaacacga tcattccatt gtgcaatttt 1020

acggggatgg gtgggcggag ggacacaaca aaatttaaga atgactattt gggcgggctg 1080

gctcttttgc agcttgtgat ttcttccagc ttgggagggg ctgctggaag tggcatttcg 1140

ttcagagctg actttcagtg cacccaaact ggatgacgtg ccaatgtcca tttgccttat 1200

gctttgtgga gctgattagg ctgggatttg aggtgataat ccagtaagtc tttcctcgtt 1260

cctacttgtg gaggatcagt agctgttatg atgccagacc atttggagaa gtatcagagg 1320

cctgaccgga cacataatac gacaaccaca tttttcctca tcatccatga ggaaatggat 1380

gatttctctt ttccatatgt cactggggga aaggctgcct gtacctctca agctttgcat 1440

tttactggaa actgaggcgt caagatggct gtggcagcta gcaaaagcaa agatgctttg 1500

tgcatagcct tgtgaaaaag tatctttcta tgcaataaga tgaattttcc tcccagaata 1560

tttagaaatg tagaagggat aacagttcac agccaggtaa aatttaactg gtggcttaat 1620

gactctgcac ctttttctca ggaattctgc ctaagttgtc tgccttttct accaccaaaa 1680

agacttttag ttttctatgc tttctcctga attttggtag ggtaagtatt tctatgtcaa 1740

aggcacagcc ttgatgatct cagggaaaaa ttttaatcac tgtgtataat gatactgaac 1800

cttgattaat aacagaaatt caggatgtaa agccacagaa tgggatttat taatgtggga 1860

tacctcagac tgtttgtttt ctttctggga agaaaagtgt gttctataat gaataaatat 1920

agagtggttt tt 1932

<210> 27
<211> 214
<212> PRT
<213> human: PCTP protein

<400> 27

Met Glu Leu Ala Ala Gly Ser Phe Ser Glu Glu Gln Phe Trp Glu Ala
1               5                   10                  15

Cys Ala Glu Leu Gln Gln Pro Ala Leu Ala Gly Ala Asp Trp Gln Leu
            20                  25                  30

Leu Val Glu Thr Ser Gly Ile Ser Ile Tyr Arg Leu Leu Asp Lys Lys

        35                  40                  45

Thr Gly Leu His Glu Tyr Lys Val Phe Gly Val Leu Glu Asp Cys Ser
    50                  55                  60

Pro Thr Leu Leu Ala Asp Ile Tyr Met Asp Ser Asp Tyr Arg Lys Gln
65                  70                  75                  80

Trp Asp Gln Tyr Val Lys Glu Leu Tyr Glu Gln Glu Cys Asn Gly Glu
                85                  90                  95

Thr Val Val Tyr Trp Glu Val Lys Tyr Pro Phe Pro Met Ser Asn Arg
            100                 105                 110

Asp Tyr Val Tyr Leu Arg Gln Arg Arg Asp Leu Asp Met Glu Gly Arg
        115                 120                 125

Lys Ile His Val Ile Leu Ala Arg Ser Thr Ser Met Pro Gln Leu Gly
    130                 135                 140

Glu Arg Ser Gly Val Ile Arg Val Lys Gln Tyr Lys Gln Ser Leu Ala
145                 150                 155                 160

Ile Glu Ser Asp Gly Lys Lys Gly Ser Lys Val Phe Met Tyr Tyr Phe
                165                 170                 175

Asp Asn Pro Gly Gly Gln Ile Pro Ser Trp Leu Ile Asn Trp Ala Ala
            180                 185                 190

Lys Asn Gly Val Pro Asn Phe Leu Lys Asp Met Ala Arg Ala Cys Gln
        195                 200                 205

Asn Tyr Leu Lys Lys Thr
    210

SEQUENCE LISTING

<110>  Boehringer Ingelheim Pharma GmbH & CO KG

<120>  Improvement of protein production

<130>  P01-2188

<160>  27

<170>  PatentIn version 3.3

<210>  1
<211>  28
<212>  DNA
<213>  Artificial

<220>
<223>  PCR primer for human DNA CERT-S132A

<400>  1
cgtcgacatg gcgcaatggt gtccctgg                                          28


<210>  2
<211>  28
<212>  DNA
<213>  Artificial

<220>
<223>  PCR primer for human DNA CERT-S132Arev

<400>  2
ccagggacac cattgcgcca tgtcgacg                                          28


<210>  3
<211>  35
<212>  DNA
<213>  Artificial

<220>

<223>  PCR primer for human DNACERT-S272A

<400>  3
ggttaaacgt gaggacgcct ggcagaagag actgg                                35


<210>  4
<211>  35
<212>  DNA
<213>  Artificial

<220>
<223>  PCR primer for human DNA CERT-S272Arev

<400>  4
ccagtctctt ctgccaggcg tcctcacgtt taacc                                35


<210>  5
<211>  33
<212>  DNA
<213>  Artificial

<220>
<223>  PCR primer for human DNA CERT-138truncation

<400>  5
ggtgtccctg gtgtcttgag caagtggcta ctc                                  33


<210>  6
<211>  33
<212>  DNA
<213>  Artificial

<220>
<223>  PCR primer for human DNA CERT-138truncationrev

<400>  6
gagtagccac ttgctcaaga caccagggac acc                                  33


<210>  7

<211>  21
<212>  DNA
<213>  Artificial

<220>
<223>  siRNA/DNA siCERT-1

<400>  7
ccacaugacu uacucauuat t                                                21


<210>  8
<211>  21
<212>  DNA
<213>  Artificial

<220>
<223>  siRNA/DNA siCERT-2

<400>  8
gaacagagga agcauauaat t                                                21


<210>  9
<211>  21
<212>  DNA
<213>  Artificial

<220>
<223>  siRNA/DNA siLacZ

<400>  9
gcggcugccg gaauuuacct t                                                21


<210>  10
<211>  1797
<212>  DNA
<213>  human: CERT cDNA

<400>  10
atgtcggata atcagagctg gaactcgtcg ggctcggagg aggatccaga gacggagtct    60

gggccgcctg tggagcgctg cggggtcctc agtaagtgga caaactacat tcatgggtgg 120
caggatcgtt gggtagtttt gaaaaataat gctctgagtt actacaaatc tgaagatgaa 180
acagagtatg gctgcagagg atccatctgt cttagcaagg ctgtcatcac acctcacgat 240
tttgatgaat gtcgatttga tattagtgta aatgatagtg tttggtatct tcgtgctcag 300
gatccagatc atagacagca atggatagat gccattgaac agcacaagac tgaatctgga 360
tatggatctg aatccagctt gcgtcgacat ggctcaatgg tgtccctggt gtctggagca 420
agtggctact ctgcaacatc cacctcttca ttcaagaaag gccacagttt acgtgagaag 480
ttggctgaaa tggaaacatt tagagacatc ttatgtagac aagttgacac gctacagaag 540
tactttgatg cctgtgctga tgctgtctct aaggatgaac ttcaaaggga taaagtggta 600
gaagatgatg aagatgactt tcctacaacg cgttctgatg gtgacttctt gcatagtacc 660
aacggcaata aagaaaagtt atttccacat gtgacaccaa aaggaattaa tggtatagac 720
tttaaagggg aagcgataac ttttaaagca actactgctg gaatccttgc aacactttct 780
cattgtattg aactaatggt taaacgtgag gacagctggc agaagagact ggataaggaa 840
actgagaaga aaagaagaac agaggaagca tataaaaatg caatgacaga acttaagaaa 900
aaatcccact ttggaggacc agattatgaa gaaggcccta acagtctgat taatgaagaa 960
gagttctttg atgctgttga agctgctctt gacagacaag ataaaataga agaacagtca 1020
cagagtgaaa aggtgagatt acattggcct acatccttgc cctctggaga tgccttttct 1080
tctgtgggga cacatagatt tgtccaaaag gttgaagaga tggtgcagaa ccacatgact 1140
tactcattac aggatgtagg cggagatgcc aattggcagt tggttgtaga agaaggagaa 1200
atgaaggtat acagaagaga agtagaagaa aatgggattg ttctggatcc tttaaaagct 1260
acccatgcag ttaaaggcgt cacaggacat gaagtctgca attatttctg gaatgttgac 1320
gttcgcaatg actgggaaac aactatagaa aactttcatg tggtggaaac attagctgat 1380

aatgcaatca tcatttatca aacacacaag agggtgtggc ctgcttctca gcgagacgta 1440

ttatatcttt ctgtcattcg aaagatacca gccttgactg aaaatgaccc tgaaacttgg 1500

atagtttgta atttttctgt ggatcatgac agtgctcctc taaacaaccg atgtgtccgt 1560

gccaaaataa atgttgctat gatttgtcaa accttggtaa gcccaccaga gggaaaccag 1620

gaaattagca gggacaacat tctatgcaag attacatatg tagctaatgt gaaccctgga 1680

ggatgggcac cagcctcagt gttaagggca gtggcaaagc gagagtatcc taaatttcta 1740

aaacgtttta cttcttacgt ccaagaaaaa actgcaggaa agcctatttt gttctag 1797

<210> 11
<211> 598
<212> PRT
<213> human: CERT protein

<400> 11

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
                340                 345                 350

Leu Pro Ser Gly Asp Ala Phe Ser Ser Val Gly Thr His Arg Phe Val
            355                 360                 365

Gln Lys Val Glu Glu Met Val Gln Asn His Met Thr Tyr Ser Leu Gln
        370                 375                 380

Asp Val Gly Gly Asp Ala Asn Trp Gln Leu Val Val Glu Glu Gly Glu
385                 390                 395                 400

Met Lys Val Tyr Arg Arg Glu Val Glu Glu Asn Gly Ile Val Leu Asp
                405                 410                 415

Pro Leu Lys Ala Thr His Ala Val Lys Gly Val Thr Gly His Glu Val
                420                 425                 430

Cys Asn Tyr Phe Trp Asn Val Asp Val Arg Asn Asp Trp Glu Thr Thr
            435                 440                 445

Ile Glu Asn Phe His Val Val Glu Thr Leu Ala Asp Asn Ala Ile Ile
        450                 455                 460

Ile Tyr Gln Thr His Lys Arg Val Trp Pro Ala Ser Gln Arg Asp Val
465                 470                 475                 480

Leu Tyr Leu Ser Val Ile Arg Lys Ile Pro Ala Leu Thr Glu Asn Asp
                485                 490                 495

Pro Glu Thr Trp Ile Val Cys Asn Phe Ser Val Asp His Asp Ser Ala
                500                 505                 510

Pro Leu Asn Asn Arg Cys Val Arg Ala Lys Ile Asn Val Ala Met Ile
            515                 520                 525

Cys Gln Thr Leu Val Ser Pro Pro Glu Gly Asn Gln Glu Ile Ser Arg
        530                 535                 540

Asp Asn Ile Leu Cys Lys Ile Thr Tyr Val Ala Asn Val Asn Pro Gly
545                 550                 555                 560

Gly Trp Ala Pro Ala Ser Val Leu Arg Ala Val Ala Lys Arg Glu Tyr
                565                 570                 575

Pro Lys Phe Leu Lys Arg Phe Thr Ser Tyr Val Gln Glu Lys Thr Ala
            580                 585                 590

Gly Lys Pro Ile Leu Phe
        595

<210>  12
<211>  2389
<212>  DNA
<213>  human: CERT L cDNA

<400>  12
gcaggaagat ggcggcggta gcggaggtgt gagtggacgc gggactcagc ggccggattt    60

tctcttccct tcttttccct tttccttccc tatttgaaat tggcatcgag ggggctaagt    120

tcgggtggca gcgccgggcg caacgcaggg gtcacggcga cggcggcggc ggctgacggc   180

tggaagggta ggcttcattc accgctcgtc ctccttcctc gctccgctcg gtgtcaggcg   240

cggcggcggc gcggcgggcg gacttcgtcc ctcctcctgc tcccccccac accggagcgg   300

gcactcttcg cttcgccatc ccccgaccct tcaccccgag gactgggcgc ctcctccggc   360

gcagctgagg gagcgggggc cggtctcctg ctcggttgtc gagcctccat gtcggataat   420

cagagctgga actcgtcggg ctcggaggag gatccagaga cggagtctgg gccgcctgtg   480

gagcgctgcg gggtcctcag taagtggaca aactacattc atgggtggca ggatcgttgg   540

gtagttttga aaaataatgc tctgagttac tacaaatctg aagatgaaac agagtatggc   600

tgcagaggat ccatctgtct tagcaaggct gtcatcacac ctcacgattt tgatgaatgt   660

cgatttgata ttagtgtaaa tgatagtgtt tggtatcttc gtgctcagga tccagatcat   720

agacagcaat ggatagatgc cattgaacag cacaagactg aatctggata tggatctgaa   780

tccagcttgc gtcgacatgg ctcaatggtg tccctggtgt ctggagcaag tggctactct   840

gcaacatcca cctcttcatt caagaaaggc cacagtttac gtgagaagtt ggctgaaatg 900

gaaacattta gagacatctt atgtagacaa gttgacacgc tacagaagta ctttgatgcc 960

tgtgctgatg ctgtctctaa ggatgaactt caaagggata aagtggtaga agatgatgaa 1020

gatgactttc ctacaacgcg ttctgatggt gacttcttgc atagtaccaa cggcaataaa 1080

gaaaagttat ttccacatgt gacaccaaaa ggaattaatg gtatagactt taaaggggaa 1140

gcgataactt ttaaagcaac tactgctgga atccttgcaa cactttctca ttgtattgaa 1200

ctaatggtta aacgtgagga cagctggcag aagagactgg ataaggaaac tgagaagaaa 1260

agaagaacag aggaagcata taaaaatgca atgacagaac ttaagaaaaa atcccacttt 1320

ggaggaccag attatgaaga aggccctaac agtctgatta atgaagaaga gttctttgat 1380

gctgttgaag ctgctcttga cagacaagat aaaatagaag aacagtcaca gagtgaaaag 1440

gtgagattac attggcctac atccttgccc tctggagatg ccttttcttc tgtggggaca 1500

catagatttg tccaaaagcc ctatagtcgc tcttcctcca tgtcttccat tgatctagtc 1560

agtgcctctg atgatgttca cagattcagc tcccaggttg aagagatggt gcagaaccac 1620

atgacttact cattacagga tgtaggcgga gatgccaatt ggcagttggt tgtagaagaa 1680

ggagaaatga aggtatacag aagagaagta gaagaaaatg ggattgttct ggatccttta 1740

aaagctaccc atgcagttaa aggcgtcaca ggacatgaag tctgcaatta tttctggaat 1800

gttgacgttc gcaatgactg ggaaacaact atagaaaact ttcatgtggt ggaaacatta 1860

gctgataatg caatcatcat ttatcaaaca cacaagaggg tgtggcctgc ttctcagcga 1920

gacgtattat atctttctgt cattcgaaag ataccagcct tgactgaaaa tgaccctgaa 1980

acttggatag tttgtaattt ttctgtggat catgacagtg ctcctctaaa caaccgatgt 2040

gtccgtgcca aaataaatgt tgctatgatt tgtcaaacct tggtaagccc accagaggga 2100

aaccaggaaa ttagcaggga caacattcta tgcaagatta catatgtagc taatgtgaac 2160

cctggaggat gggcaccagc ctcagtgtta agggcagtgg caaagcgaga gtatcctaaa 2220

tttctaaaac gttttacttc ttacgtccaa gaaaaaactg caggaaagcc tattttgttc 2280

tagtattaac aggtactaga agatatgttt tatctttttt taactttatt tgactaatat 2340

gactgtcaat actaaaattt agttgttgaa agtatttact atgttttttt 2389

<210> 13
<211> 624
<212> PRT
<213> human: CERT L protein

<400> 13

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320
Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val
        355                 360                 365

Gln Lys Pro Tyr Ser Arg Ser Ser Ser Met Ser Ser Ile Asp Leu Val
    370                 375                 380

Ser Ala Ser Asp Asp Val His Arg Phe Ser Ser Gln Val Glu Glu Met
385                 390                 395                 400

Val Gln Asn His Met Thr Tyr Ser Leu Gln Asp Val Gly Gly Asp Ala
                405                 410                 415

Asn Trp Gln Leu Val Val Glu Glu Gly Glu Met Lys Val Tyr Arg Arg
            420                 425                 430

Glu Val Glu Glu Asn Gly Ile Val Leu Asp Pro Leu Lys Ala Thr His
        435                 440                 445

Ala Val Lys Gly Val Thr Gly His Glu Val Cys Asn Tyr Phe Trp Asn
    450                 455                 460

Val Asp Val Arg Asn Asp Trp Glu Thr Thr Ile Glu Asn Phe His Val
465                 470                 475                 480

Val Glu Thr Leu Ala Asp Asn Ala Ile Ile Ile Tyr Gln Thr His Lys
                485                 490                 495

Arg Val Trp Pro Ala Ser Gln Arg Asp Val Leu Tyr Leu Ser Val Ile
            500                 505                 510

Arg Lys Ile Pro Ala Leu Thr Glu Asn Asp Pro Glu Thr Trp Ile Val
        515                 520                 525

Cys Asn Phe Ser Val Asp His Asp Ser Ala Pro Leu Asn Asn Arg Cys
    530                 535                 540

Val Arg Ala Lys Ile Asn Val Ala Met Ile Cys Gln Thr Leu Val Ser
545                 550                 555                 560

Pro Pro Glu Gly Asn Gln Glu Ile Ser Arg Asp Asn Ile Leu Cys Lys
                565                 570                 575

Ile Thr Tyr Val Ala Asn Val Asn Pro Gly Gly Trp Ala Pro Ala Ser
            580                 585                 590

Val Leu Arg Ala Val Ala Lys Arg Glu Tyr Pro Lys Phe Leu Lys Arg
        595                 600                 605

Phe Thr Ser Tyr Val Gln Glu Lys Thr Ala Gly Lys Pro Ile Leu Phe
    610                 615                 620

<210>  14
<211>  1797
<212>  DNA
<213>  human: CERT S132A cDNA

<400>  14
atgtcggata atcagagctg gaactcgtcg ggctcggagg aggatccaga gacggagtct    60

gggccgcctg tggagcgctg cggggtcctc agtaagtgga caaactacat tcatgggtgg   120

caggatcgtt gggtagtttt gaaaaataat gctctgagtt actacaaatc tgaagatgaa   180

acagagtatg gctgcagagg atccatctgt cttagcaagg ctgtcatcac acctcacgat   240

tttgatgaat gtcgatttga tattagtgta aatgatagtg tttggtatct tcgtgctcag   300

gatccagatc atagacagca atggatagat gccattgaac agcacaagac tgaatctgga   360

tatggatctg aatccagctt gcgtcgacat ggcgcaatgg tgtccctggt gtctggagca   420

agtggctact ctgcaacatc cacctcttca ttcaagaaag gccacagttt acgtgagaag   480

ttggctgaaa tggaaacatt tagagacatc ttatgtagac aagttgacac gctacagaag   540

tactttgatg cctgtgctga tgctgtctct aaggatgaac ttcaaaggga taaagtggta   600

gaagatgatg aagatgactt tcctacaacg cgttctgatg gtgacttctt gcatagtacc   660

aacggcaata aagaaaagtt atttccacat gtgacaccaa aaggaattaa tggtatagac   720

tttaaagggg aagcgataac ttttaaagca actactgctg gaatccttgc aacactttct   780

cattgtattg aactaatggt taaacgtgag gacagctggc agaagagact ggataaggaa   840

actgagaaga aaagaagaac agaggaagca tataaaaatg caatgacaga acttaagaaa   900

aaatcccact ttggaggacc agattatgaa gaaggcccta acagtctgat taatgaagaa 960

gagttctttg atgctgttga agctgctctt gacagacaag ataaaataga agaacagtca 1020

cagagtgaaa aggtgagatt acattggcct acatccttgc cctctggaga tgccttttct 1080

tctgtgggga cacatagatt tgtccaaaag gttgaagaga tggtgcagaa ccacatgact 1140

tactcattac aggatgtagg cggagatgcc aattggcagt tggttgtaga agaaggagaa 1200

atgaaggtat acagaagaga agtagaagaa aatgggattg ttctggatcc tttaaaagct 1260

acccatgcag ttaaaggcgt cacaggacat gaagtctgca attatttctg gaatgttgac 1320

gttcgcaatg actgggaaac aactatagaa aactttcatg tggtggaaac attagctgat 1380

aatgcaatca tcatttatca aacacacaag agggtgtggc ctgcttctca gcgagacgta 1440

ttatatcttt ctgtcattcg aaagatacca gccttgactg aaaatgaccc tgaaacttgg 1500

atagtttgta atttttctgt ggatcatgac agtgctcctc taaacaaccg atgtgtccgt 1560

gccaaaataa atgttgctat gatttgtcaa accttggtaa gcccaccaga gggaaaccag 1620

gaaattagca gggacaacat tctatgcaag attacatatg tagctaatgt gaaccctgga 1680

ggatgggcac cagcctcagt gttaagggca gtggcaaagc gagagtatcc taaatttcta 1740

aaacgtttta cttcttacgt ccaagaaaaa actgcaggaa agcctatttt gttctag 1797

<210> 15
<211> 598
<212> PRT
<213> human: CERT S132A protein

<400> 15

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ala Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val
        355                 360                 365

Gln Lys Val Glu Glu Met Val Gln Asn His Met Thr Tyr Ser Leu Gln
    370                 375                 380

Asp Val Gly Gly Asp Ala Asn Trp Gln Leu Val Val Glu Glu Gly Glu
385                 390                 395                 400

Met Lys Val Tyr Arg Arg Glu Val Glu Glu Asn Gly Ile Val Leu Asp
                405                 410                 415

Pro Leu Lys Ala Thr His Ala Val Lys Gly Val Thr Gly His Glu Val
            420                 425                 430

Cys Asn Tyr Phe Trp Asn Val Asp Val Arg Asn Asp Trp Glu Thr Thr
        435                 440                 445

Ile Glu Asn Phe His Val Val Glu Thr Leu Ala Asp Asn Ala Ile Ile
    450                 455                 460

Ile Tyr Gln Thr His Lys Arg Val Trp Pro Ala Ser Gln Arg Asp Val
465                 470                 475                 480

Leu Tyr Leu Ser Val Ile Arg Lys Ile Pro Ala Leu Thr Glu Asn Asp
                485                 490                 495

Pro Glu Thr Trp Ile Val Cys Asn Phe Ser Val Asp His Asp Ser Ala
            500                 505                 510

Pro Leu Asn Asn Arg Cys Val Arg Ala Lys Ile Asn Val Ala Met Ile
        515                 520                 525

Cys Gln Thr Leu Val Ser Pro Pro Glu Gly Asn Gln Glu Ile Ser Arg
    530                 535                 540

Asp Asn Ile Leu Cys Lys Ile Thr Tyr Val Ala Asn Val Asn Pro Gly
545                 550                 555                 560

Gly Trp Ala Pro Ala Ser Val Leu Arg Ala Val Ala Lys Arg Glu Tyr
                565                 570                 575

Pro Lys Phe Leu Lys Arg Phe Thr Ser Tyr Val Gln Glu Lys Thr Ala
            580                 585                 590

Gly Lys Pro Ile Leu Phe
        595


<210>  16
<211>  669
<212>  DNA
<213>  human: START Domain CERT cDNA

<400>  16
agatttgtcc aaaaggttga agagatggtg cagaaccaca tgacttactc attacaggat    60

gtaggcggag atgccaattg gcagttggtt gtagaagaag gagaaatgaa ggtatacaga   120

agagaagtag aagaaaatgg gattgttctg gatcctttaa aagctaccca tgcagttaaa   180

ggcgtcacag gacatgaagt ctgcaattat ttctggaatg ttgacgttcg caatgactgg   240

gaaacaacta tagaaaactt tcatgtggtg gaaacattag ctgataatgc aatcatcatt   300

tatcaaacac acaagagggt gtggcctgct tctcagcgag acgtattata tctttctgtc   360

attcgaaaga taccagcctt gactgaaaat gaccctgaaa cttggatagt ttgtaatttt 420

tctgtggatc atgacagtgc tcctctaaac aaccgatgtg tccgtgccaa aataaatgtt 480

gctatgattt gtcaaacctt ggtaagccca ccagagggaa accaggaaat tagcagggac 540

aacattctat gcaagattac atatgtagct aatgtgaacc ctggaggatg ggcaccagcc 600

tcagtgttaa gggcagtggc aaagcgagag tatcctaaat ttctaaaacg ttttacttct 660

tacgtccaa 669

<210> 17
<211> 223
<212> PRT
<213> human: START Domain CERT protein

<400> 17

Arg Phe Val Gln Lys Val Glu Glu Met Val Gln Asn His Met Thr Tyr
1               5                   10                  15

Ser Leu Gln Asp Val Gly Gly Asp Ala Asn Trp Gln Leu Val Val Glu
            20                  25                  30

Glu Gly Glu Met Lys Val Tyr Arg Arg Glu Val Glu Glu Asn Gly Ile
        35                  40                  45

Val Leu Asp Pro Leu Lys Ala Thr His Ala Val Lys Gly Val Thr Gly
    50                  55                  60

His Glu Val Cys Asn Tyr Phe Trp Asn Val Asp Val Arg Asn Asp Trp
65                  70                  75                  80

Glu Thr Thr Ile Glu Asn Phe His Val Val Glu Thr Leu Ala Asp Asn
                85                  90                  95

Ala Ile Ile Ile Tyr Gln Thr His Lys Arg Val Trp Pro Ala Ser Gln
            100                 105                 110

Arg Asp Val Leu Tyr Leu Ser Val Ile Arg Lys Ile Pro Ala Leu Thr
        115                 120                 125

Glu Asn Asp Pro Glu Thr Trp Ile Val Cys Asn Phe Ser Val Asp His
    130                 135                 140

Asp Ser Ala Pro Leu Asn Asn Arg Cys Val Arg Ala Lys Ile Asn Val
145                 150                 155                 160

Ala Met Ile Cys Gln Thr Leu Val Ser Pro Pro Glu Gly Asn Gln Glu
                165                 170                 175

Ile Ser Arg Asp Asn Ile Leu Cys Lys Ile Thr Tyr Val Ala Asn Val
            180                 185                 190

Asn Pro Gly Gly Trp Ala Pro Ala Ser Val Leu Arg Ala Val Ala Lys
        195                 200                 205

Arg Glu Tyr Pro Lys Phe Leu Lys Arg Phe Thr Ser Tyr Val Gln
    210                 215                 220

<210>  18
<211>  658
<212>  DNA
<213>  human: START Domain CERT L cDNA

<400>  18
caggttgaag agatggtgca gaaccacatg acttactcat tacaggatgt aggcggagat   60

gccaattggc agttggttgt agaagaagga gaaatgaagg tatacagaag agaagtagaa  120

gaaaatggga ttgttctgga tcctttaaaa gctacccatg cagttaaagg cgtcacagga  180

catgaagtct gcaattattt ctggaatgtt gacgttcgca atgactggga aacaactata  240

gaaaactttc atgtggtgga aacattagct gataatgcaa tcatcattta tcaaacacac  300

aagagggtgt ggcctgcttc tcagcgagac gtattatatc tttctgtcat tcgaaagata  360

ccagccttga ctgaaaatga ccctgaaact tggatagttt gtaatttttc tgtggatcat  420

gacagtgctc ctctaaacaa ccgatgtgtc cgtgccaaaa taaatgttgc tatgatttgt  480

caaaccttgg taagcccacc agagggaaac caggaaatta gcagggacaa cattctatgc  540

aagattacat atgtagctaa tgtgaaccct ggaggatggg caccagcctc agtgttaagg 600

gcagtggcaa agcgagagta tcctaaattt ctaaaacgtt ttacttctta cgtccaag 658

<210> 19
<211> 219
<212> PRT
<213> human: START Domain CERT L protein

<400> 19

Gln Val Glu Glu Met Val Gln Asn His Met Thr Tyr Ser Leu Gln Asp
1               5                   10                  15

Val Gly Gly Asp Ala Asn Trp Gln Leu Val Val Glu Glu Gly Glu Met
            20                  25                  30

Lys Val Tyr Arg Arg Glu Val Glu Glu Asn Gly Ile Val Leu Asp Pro
        35                  40                  45

Leu Lys Ala Thr His Ala Val Lys Gly Val Thr Gly His Glu Val Cys
    50                  55                  60

Asn Tyr Phe Trp Asn Val Asp Val Arg Asn Asp Trp Glu Thr Thr Ile
65                  70                  75                  80

Glu Asn Phe His Val Val Glu Thr Leu Ala Asp Asn Ala Ile Ile Ile
                85                  90                  95

Tyr Gln Thr His Lys Arg Val Trp Pro Ala Ser Gln Arg Asp Val Leu
            100                 105                 110

Tyr Leu Ser Val Ile Arg Lys Ile Pro Ala Leu Thr Glu Asn Asp Pro
        115                 120                 125

Glu Thr Trp Ile Val Cys Asn Phe Ser Val Asp His Asp Ser Ala Pro
    130                 135                 140

Leu Asn Asn Arg Cys Val Arg Ala Lys Ile Asn Val Ala Met Ile Cys
145                 150                 155                 160

Gln Thr Leu Val Ser Pro Pro Glu Gly Asn Gln Glu Ile Ser Arg Asp
                165                 170                 175

Asn Ile Leu Cys Lys Ile Thr Tyr Val Ala Asn Val Asn Pro Gly Gly
            180                 185                 190

Trp Ala Pro Ala Ser Val Leu Arg Ala Val Ala Lys Arg Glu Tyr Pro
        195                 200                 205

Lys Phe Leu Lys Arg Phe Thr Ser Tyr Val Gln
    210                 215

<210>  20
<211>  2264
<212>  DNA
<213>  human: StarD4 cDNA

<400>  20
actgttgaga gcggtgtgag gtgcttggta gcgcgccgta gctgcttcca cgtccttgct    60

tcacctcagg taaagagaga agtaatggaa ggcctgtctg atgttgcttc ttttgcaact   120

aaacttaaaa acactctcat ccagtaccat agcattgaag aagataagtg gcgagttgct   180

aagaaaacga aagatgtaac tgtttggaga aaaccctcag aagaatttaa tggatatctc   240

tacaaagccc aaggtgttat agatgacctt gtctatagta taatagacca tatacgccca   300

gggccttgtc gtttggattg ggacagcttg atgacttctt tggatattct ggagaacttt   360

gaagagaatt gctgtgtgat gcgttacact actgctggtc agctttggaa tataatttcc   420

ccaagagaat ttgttgattt ctcctatact gtgggctata aagaagggct tttatcttgt   480

ggaataagtc ttgactggga tgaaaagaga ccagaatttg ttcgaggata taaccatccc   540

tgtggttggt tttgtgttcc acttaaagac aacccaaacc agagtctttt gacaggatat   600

attcagacag atctgcgtgg gatgattcct cagtctgcgg tagatacagc catggcaagc   660

actttaacca acttctatgg tgatttacga aaagctttat gagaggcaaa atacattcaa   720

acttgtagta ctacagatca actctctcag ctacatggcc tgtaaaaatc attgattcca 780

cttttctgca tagccggtag aaaaatttga aatgtttttg gttcactagt acaatgtttg 840

gttttattcc taaagtaaat agctatctaa gagagggcat tttcactttt ttttttttaa 900

attttgagac aggctctcac tctgttgccc atgctggagg gcagtggtat gatcacagct 960

cactgcagct ttgatctgac cgctcaaggg gttattctac ctcagcctcc tgaatagctg 1020

ggaatacagg tgcacgccac tatgcatggc taatttttgt ttaatttttt gtagagatgt 1080

ggtcacactg tgttgcccag gctggtcttg aactcctggc ctcaagtcat tccccacctt 1140

agcctcccaa agtgttggga ttataagcgt gagccaccat gcctggcccc aatttaaaat 1200

gtggaattca gttggtgtcc aagacttatc ttgagactct taaaagcatc agtctgtaac 1260

tagaacaaat acagtcttag atttacccaa gtgcctagat atcattttat aatgattaga 1320

attgagtatt gtgggtcccc taattctgtg ggtgccttaa gtgagaattt ctaaatgatt 1380

ttcacattct aaatgacttt gggttttgaa ctctccatct agtttacttc taaaatggga 1440

acttgaggca attcaggtat ccaggcaaat ctttgtatat atttttttgt gtacatgcac 1500

acatctcgaa atccatttcc gtgtttaatg ttagttgttt atgtgttagt attcctgtgt 1560

ctactgtttt gttgttgtta atatgggtaa agtgagccct gaaatacatg ctaaacaaga 1620

catgaaattc agaaaggtac atagtgtttc aagtgcatgg tagtttgatc tgtgttttac 1680

tttattgtgt tttcttgagt gtaaagaaag aataaatcaa agttcttcat acccattttg 1740

acaaagtgga acagtggagc tgttttttgc ttttgttttt atttattttt tgccactggt 1800

gatgatagat ttcaaaaaac aaaaggtggc agcagcacaa tgttcatggt gaattatctc 1860

atagtatcta gattgatcaa gatctgacag aaggaatgca caaaggattc tatattctta 1920

atgatttatt aattaccagg atccttttct aaattgaatg tacttttgaa ttactaggtt 1980

tcttcttttt ttttgttctg caatagtgaa agaaaactca gtagtttagt ttcagtttct 2040

catggaaatt ggtaaatgtt agttttgact tcatctattt tttatttgtt tttattagcg 2100

tagagtagga agtctcatat tctactgttc tatctaggat ggtgaaattc caaaggtgcc 2160

taacttgagt aagggatttg tgacaagata gtacacatta ctataagggc tattatttcc 2220

tgaactggat gtccctaaaa gcaaataaac tgcccactat ctct 2264

<210> 21
<211> 205
<212> PRT
<213> human: StarD4 protein

<400> 21

Met Glu Gly Leu Ser Asp Val Ala Ser Phe Ala Thr Lys Leu Lys Asn
1               5                   10                  15

Thr Leu Ile Gln Tyr His Ser Ile Glu Glu Asp Lys Trp Arg Val Ala
            20                  25                  30

Lys Lys Thr Lys Asp Val Thr Val Trp Arg Lys Pro Ser Glu Glu Phe
        35                  40                  45

Asn Gly Tyr Leu Tyr Lys Ala Gln Gly Val Ile Asp Asp Leu Val Tyr
    50                  55                  60

Ser Ile Ile Asp His Ile Arg Pro Gly Pro Cys Arg Leu Asp Trp Asp
65                  70                  75                  80

Ser Leu Met Thr Ser Leu Asp Ile Leu Glu Asn Phe Glu Glu Asn Cys
                85                  90                  95

Cys Val Met Arg Tyr Thr Thr Ala Gly Gln Leu Trp Asn Ile Ile Ser
            100                 105                 110

Pro Arg Glu Phe Val Asp Phe Ser Tyr Thr Val Gly Tyr Lys Glu Gly
        115                 120                 125

Leu Leu Ser Cys Gly Ile Ser Leu Asp Trp Asp Glu Lys Arg Pro Glu
    130                 135                 140

Phe Val Arg Gly Tyr Asn His Pro Cys Gly Trp Phe Cys Val Pro Leu
145                 150                 155                 160

Lys Asp Asn Pro Asn Gln Ser Leu Leu Thr Gly Tyr Ile Gln Thr Asp
                165                 170                 175

Leu Arg Gly Met Ile Pro Gln Ser Ala Val Asp Thr Ala Met Ala Ser
            180                 185                 190

Thr Leu Thr Asn Phe Tyr Gly Asp Leu Arg Lys Ala Leu
        195                 200                 205


<210>  22
<211>  1344
<212>  DNA
<213>  human: StarD5 cDNA

<400>  22
gagctccagc ctccaggcac ccgggatcca gcgccgccgc tcataacacc cgcgaccccg    60

cagctaagcg cagctcccga cgcaatggac ccggcgctgg cagcccagat gagcgaggct   120

gtggccgaga agatgctcca gtaccggcgg gacacagcag gctggaagat ttgccgggaa   180

ggcaatggag tttcagtttc ctggaggcca tctgtggagt ttccagggaa cctgtaccga   240

ggagaaggca ttgtatatgg gacactagag gaggtgtggg actgtgtgaa gccagctgtt   300

ggaggcctac gagtgaagtg ggatgagaat gtgaccggtt ttgaaattat ccaaagcatc   360

actgacaccc tgtgtgtaag cagaacctcc actccctccg ctgccatgaa gctcatttct   420

cccagagatt ttgtggactt ggtgctagtc aagagatatg aggatgggac catcagttcc   480

aacgccaccc atgtggagca tccgttatgt cccccgaagc caggttttgt gagaggattt   540

aaccatcctt gtggttgctt ctgtgaacct cttccagggg aacccaccaa gaccaacctg   600

gtcacattct tccataccga cctcagcggt tacctcccac agaacgtggt ggactccttc   660

ttcccccgca gcatgacccg gttttatgcc aaccttcaga aagcagtgaa gcaattccat   720

gagtaatgct atcgttactt cttggcaaag aactcccgtg actcatcgag gagctccagc 780

tgttgggaca ccaaggagcc tgggagcacg cagaggcctg tgttcactct ttggaacaag 840

ctgatggact gcgcatctct gagaatgcca accagaggcg gcagcccacc cttcctgcct 900

cctgccccac tcagggttgg cgtgtgatga gccattcatg tgttccaaac tccatctgcc 960

tgttacccaa acacgcctct cctggcaggg tagacccagg cctctaacca tctgacagag 1020

actcggcctg gacaccatgc gatgcactct ggcaccaagg ctttatgtgc ccatcactct 1080

cagagaccac gtttccctga ctgtcataga gaatcatcat cgccactgaa aaccaggccc 1140

tgttgccttt taagcatgta ccgctccctc agtcctgtgc tgcagccccc caaatatatt 1200

tttctgatat agaccttgta tatggcttta atgccgcaaa atatttattt ttccttaaaa 1260

aaggtgtcaa cttggaaata atggtttaaa aacaggataa gcattaagga aaaacaaaaa 1320

aaaaaaaaaa aaaaaaaaaa aaaa 1344

<210> 23
<211> 213
<212> PRT
<213> human: StarD5 protein

<400> 23

Met Asp Pro Ala Leu Ala Ala Gln Met Ser Glu Ala Val Ala Glu Lys
1               5                   10                  15

Met Leu Gln Tyr Arg Arg Asp Thr Ala Gly Trp Lys Ile Cys Arg Glu
            20                  25                  30

Gly Asn Gly Val Ser Val Ser Trp Arg Pro Ser Val Glu Phe Pro Gly
        35                  40                  45

Asn Leu Tyr Arg Gly Glu Gly Ile Val Tyr Gly Thr Leu Glu Glu Val
    50                  55                  60

Trp Asp Cys Val Lys Pro Ala Val Gly Gly Leu Arg Val Lys Trp Asp
65                  70                  75                  80

Glu Asn Val Thr Gly Phe Glu Ile Ile Gln Ser Ile Thr Asp Thr Leu
                85                  90                  95

Cys Val Ser Arg Thr Ser Thr Pro Ser Ala Ala Met Lys Leu Ile Ser
            100                 105                 110

Pro Arg Asp Phe Val Asp Leu Val Leu Val Lys Arg Tyr Glu Asp Gly
        115                 120                 125

Thr Ile Ser Ser Asn Ala Thr His Val Glu His Pro Leu Cys Pro Pro
    130                 135                 140

Lys Pro Gly Phe Val Arg Gly Phe Asn His Pro Cys Gly Cys Phe Cys
145                 150                 155                 160

Glu Pro Leu Pro Gly Glu Pro Thr Lys Thr Asn Leu Val Thr Phe Phe
                165                 170                 175

His Thr Asp Leu Ser Gly Tyr Leu Pro Gln Asn Val Val Asp Ser Phe
            180                 185                 190

Phe Pro Arg Ser Met Thr Arg Phe Tyr Ala Asn Leu Gln Lys Ala Val
        195                 200                 205

Lys Gln Phe His Glu
    210

<210>  24
<211>  663
<212>  DNA
<213>  human: StarD6 cDNA

<400>  24
atggacttca aggcaattgc ccaacaaact gcccaagaag ttttaggtta taatcgagat    60

acatcaggct ggaaagtggt taaaacttca aaaaagataa ctgtttccag taaggcttct   120

agaaaattcc atggaaatct atatcgtgtt gaagggataa ttccagaatc accagctaaa   180

ctatctgatt tcctctacca aactggagac agaattacat gggataaatc attgcaagtg 240

tataatatgg tacacaggat tgattcggac acattcatat gtcataccat tacacaaagt 300

tttgccgtgg gctccatttc ccctcgagac tttatcgact tagtgtacat caagcgctac 360

gaaggaaata tgaacattat cagttctaaa agtgtggatt ttccagaata tcctccatct 420

tcaaattata tccgcggtta taaccatcct tgtggctttg tatgttcacc aatggaagaa 480

aacccagcat attccaaact agtgatgttt gtccagacag aaatgagagg aaaattgtcc 540

ccatcaataa ttgaaaaaac catgccttcc aacttagtaa acttcatcct caatgcaaaa 600

gatggaataa aggcacacag aactccatca agacgtggat ttcatcataa tagtcattca 660

tga 663

<210> 25
<211> 220
<212> PRT
<213> human: StarD6 protein

<400> 25

Met Asp Phe Lys Ala Ile Ala Gln Gln Thr Ala Gln Glu Val Leu Gly
1               5                   10                  15

Tyr Asn Arg Asp Thr Ser Gly Trp Lys Val Val Lys Thr Ser Lys Lys
            20                  25                  30

Ile Thr Val Ser Ser Lys Ala Ser Arg Lys Phe His Gly Asn Leu Tyr
        35                  40                  45

Arg Val Glu Gly Ile Ile Pro Glu Ser Pro Ala Lys Leu Ser Asp Phe
    50                  55                  60

Leu Tyr Gln Thr Gly Asp Arg Ile Thr Trp Asp Lys Ser Leu Gln Val
65                  70                  75                  80

Tyr Asn Met Val His Arg Ile Asp Ser Asp Thr Phe Ile Cys His Thr
                85                  90                  95

Ile Thr Gln Ser Phe Ala Val Gly Ser Ile Ser Pro Arg Asp Phe Ile
            100                 105                 110

Asp Leu Val Tyr Ile Lys Arg Tyr Glu Gly Asn Met Asn Ile Ile Ser
        115                 120                 125

Ser Lys Ser Val Asp Phe Pro Glu Tyr Pro Pro Ser Ser Asn Tyr Ile
    130                 135                 140

Arg Gly Tyr Asn His Pro Cys Gly Phe Val Cys Ser Pro Met Glu Glu
145                 150                 155                 160

Asn Pro Ala Tyr Ser Lys Leu Val Met Phe Val Gln Thr Glu Met Arg
                165                 170                 175

Gly Lys Leu Ser Pro Ser Ile Ile Glu Lys Thr Met Pro Ser Asn Leu
            180                 185                 190

Val Asn Phe Ile Leu Asn Ala Lys Asp Gly Ile Lys Ala His Arg Thr
        195                 200                 205

Pro Ser Arg Arg Gly Phe His His Asn Ser His Ser
    210                 215                 220


<210>  26
<211>  1932
<212>  DNA
<213>  human: PCTP cDNA

<400>  26
ccggactgcg gaaggatgga gctggccgcc ggaagcttct cggaggagca gttctgggag    60

gcctgcgccg agctccagca gcccgctctg gccggggccg actggcagct cctagtggag   120

acctcgggca tcagcatcta ccggctgctg gacaagaaga ctggacttca tgagtataaa   180

gtctttggtg ttctggagga ctgctcacca actctactgg cagacatcta tatggactca   240

gattacagaa aacaatggga ccagtatgtt aaagaactct atgaacaaga atgcaacgga   300

gagactgtgg tctactggga agtgaagtac ccttttccca tgtccaacag agactatgtc   360

taccttcggc agcggcgaga cctggacatg gaagggagga agatccatgt gatcctggcc 420
cggagcacct ccatgcctca gcttggcgag aggtctgggg tgatccgggt gaagcaatac 480
aagcagagcc tggcgattga gagtgacggc aagaagggga gcaaagtttt catgtattac 540
ttcgataacc cgggtggcca aattccgtcc tggctcatta actgggccgc caagaatgga 600
gttcctaact tcttgaaaga catggcaaga gcctgtcaga actacctcaa gaaaacctaa 660
gaaagagaac tgggaacatt gcatccatgg gttgatgtct ctggaagtgc aaccacccaa 720
tgtctctgga agtgccacct ggaagtgcca cctggaagtg tctctggaag agcacccacc 780
actgttcagc cttcccctgc tgtttctgtc ttcagaggcc tacacactac cacatccttt 840
ctaagcatgt ttgcctgaca tccagctcac tcgtctgctt cctttctcgc tccccccatc 900
ctgggctggg ctgccttctt ctacagttca atatggggca gactagggaa acctttgctt 960
gcttactatt aggaggggaa gtcttcagta gggaacacga tcattccatt gtgcaatttt 1020
acggggatgg gtgggcggag ggacacaaca aaatttaaga atgactattt gggcgggctg 1080
gctcttttgc agcttgtgat ttcttccagc ttgggagggg ctgctggaag tggcatttcg 1140
ttcagagctg actttcagtg cacccaaact ggatgacgtg ccaatgtcca tttgccttat 1200
gctttgtgga gctgattagg ctgggatttg aggtgataat ccagtaagtc tttcctcgtt 1260
cctacttgtg gaggatcagt agctgttatg atgccagacc atttggagaa gtatcagagg 1320
cctgaccgga cacataatac gacaaccaca tttttcctca tcatccatga ggaaatggat 1380
gatttctctt ttccatatgt cactggggga aaggctgcct gtacctctca agctttgcat 1440
tttactggaa actgaggcgt caagatggct gtggcagcta gcaaaagcaa agatgctttg 1500
tgcatagcct tgtgaaaaag tatctttcta tgcaataaga tgaattttcc tcccagaata 1560
tttagaaatg tagaagggat aacagttcac agccaggtaa aatttaactg gtggcttaat 1620

gactctgcac ctttttctca ggaattctgc ctaagttgtc tgccttttct accaccaaaa 1680

agacttttag ttttctatgc tttctcctga attttggtag ggtaagtatt tctatgtcaa 1740

aggcacagcc ttgatgatct cagggaaaaa ttttaatcac tgtgtataat gatactgaac 1800

cttgattaat aacagaaatt caggatgtaa agccacagaa tgggatttat taatgtggga 1860

tacctcagac tgtttgtttt ctttctggga agaaaagtgt gttctataat gaataaatat 1920

agagtggttt tt 1932

<210> 27
<211> 214
<212> PRT
<213> human: PCTP protein

<400> 27

Met Glu Leu Ala Ala Gly Ser Phe Ser Glu Glu Gln Phe Trp Glu Ala
1               5                   10                  15

Cys Ala Glu Leu Gln Gln Pro Ala Leu Ala Gly Ala Asp Trp Gln Leu
            20                  25                  30

Leu Val Glu Thr Ser Gly Ile Ser Ile Tyr Arg Leu Leu Asp Lys Lys
        35                  40                  45

Thr Gly Leu His Glu Tyr Lys Val Phe Gly Val Leu Glu Asp Cys Ser
    50                  55                  60

Pro Thr Leu Leu Ala Asp Ile Tyr Met Asp Ser Asp Tyr Arg Lys Gln
65                  70                  75                  80

Trp Asp Gln Tyr Val Lys Glu Leu Tyr Glu Gln Glu Cys Asn Gly Glu
                85                  90                  95

Thr Val Val Tyr Trp Glu Val Lys Tyr Pro Phe Pro Met Ser Asn Arg
            100                 105                 110

Asp Tyr Val Tyr Leu Arg Gln Arg Arg Asp Leu Asp Met Glu Gly Arg
        115                 120                 125

Lys Ile His Val Ile Leu Ala Arg Ser Thr Ser Met Pro Gln Leu Gly
    130                 135                 140

Glu Arg Ser Gly Val Ile Arg Val Lys Gln Tyr Lys Gln Ser Leu Ala
145                 150                 155                 160

Ile Glu Ser Asp Gly Lys Lys Gly Ser Lys Val Phe Met Tyr Tyr Phe
                165                 170                 175

Asp Asn Pro Gly Gly Gln Ile Pro Ser Trp Leu Ile Asn Trp Ala Ala
            180                 185                 190

Lys Asn Gly Val Pro Asn Phe Leu Lys Asp Met Ala Arg Ala Cys Gln
        195                 200                 205

Asn Tyr Leu Lys Lys Thr
    210